# FASCICULE DE BREVET EUROPEEN

(11) **EP 3 463 292 B1**
(45) Date de publication et mention de la délivrance du brevet: **05.08.2020**
(21) Numéro de dépôt: 17727906.4
(22) Date de dépôt: 07.06.2017
(51) Int. Cl.: A61K 9/08, A61K 47/34, A61K 38/28

(54) **SOLUTION INJECTABLE A PH 7 COMPRENANT AU MOINS UNE INSULINE BASALE DONT LE PI EST COMPRIS ENTRE 5,8 ET 8,5 ET UN CO-POLYAMINOACIDE PORTEUR DE CHARGES CARBOXYLATES ET DE RADICAUX HYDROPHOBES**
INJEKTIONSLÖSUNG MIT PH-WERT 7 MIT MINDESTENS EINEM BASALINSULIN MIT EINEMPI ZWISCHEN 5,8 UND 8,5 UND EINER COPOLYAMINOSÄURE MIT CARBOXYLATLADUNGEN UND HYDROPHOBEN RADIKALEN
INJECTABLE SOLUTION AT PH 7 COMPRISING AT LEAST ONE BASAL INSULIN HAVING A PI OF BETWEEN 5.8 AND 8.5 AND A COPOLYAMINO ACID BEARING CARBOXYLATE CHARGES AND HYDROPHOBIC RADICALS

(30) Priorité: 07.06.2016 FR 1655222
(43) Date de publication de la demande: 10.04.2019
(73) Titulaire: Adocia, 69003 Lyon (FR)
(72) Inventeur: GEISSLER, Alexandre, 69007 LYON (FR); LAAGE, Ségolène, 69003 Lyon (FR); CHARVET, Richard, 69140 Rillieux La Pape (FR); SOULA, Olivier, 69330 Meyzieu (FR)
(74) Mandataire: Tripoz, Inès
(86) Numéro de dépôt international: PCT/EP2017/063865
(87) Numéro de publication internationale: WO 2017/211903

(56) Documents cités:
- US-A1- 2013 178 415

## Description

L'invention concerne les thérapies par injection d'insuline(s) pour traiter le diabète.

L'invention concerne des compositions stables physiquement sous forme d'une solution aqueuse injectable, dont le pH est compris entre 6,0 et 8,0, comprenant au moins une insuline basale dont le point isoélectrique (pI) est compris entre 5,8 et 8,5 et un co-polyaminoacide porteur de charges carboxylates et de radicaux hydrophobes.

L'insulinothérapie, ou thérapie du diabète par injection d'insuline, a connu ces dernières années des progrès remarquables grâce notamment à la mise au point de nouvelles insulines offrant une meilleure correction de la glycémie des patients en comparaison de l'insuline humaine et qui permettent de mieux simuler l'activité physiologique du pancréas.

Lorsqu'un diabète de type II est diagnostiqué chez un patient, un traitement graduel est mis en place. Le patient prend en premier lieu des antidiabétiques oraux (OAD) comme la Metformine. Lorsque les OAD seuls ne suffisent plus à réguler le niveau de glucose dans le sang, un changement dans le traitement doit être fait et, en fonction des spécificités des patients, différentes associations de traitements peuvent être mises en place. Le patient peut par exemple avoir un traitement à base d'une insuline basale de type insuline glargine ou insuline detemir en complément des OAD, puis ensuite en fonction de l'évolution de la pathologie un traitement à base d'insuline basale et d'insuline prandiale.

Par ailleurs, aujourd'hui, pour assurer la transition des traitements par les OAD, lorsque ceux-ci ne sont plus en mesure de contrôler le niveau de glucose dans le sang, vers un traitement insuline basale/insuline prandiale, l'injection d'analogues de GLP-1 RA est préconisée.

Les GLP-1 RA pour agonistes du récepteur Glucagon-Like Peptide-1, sont des peptides insulinotropiques ou incrétines, et appartiennent à la famille des hormones gastro-intestinales (ou Gut Hormones) qui stimulent la sécrétion d'insuline lorsque la glycémie est trop élevée, par exemple après un repas.

Les hormones gastro-intestinales (Gut hormones) sont aussi appelées hormones de satiété. Elles comprennent notamment le GLP-1 RA (Glucagon like peptide-1 receptor agonist) et le GIP (Glucose-dependent insulinotropic peptide), l'oxyntomoduline (un dérivé du proglucagon), le peptide YY, l'amyline, la cholecystokinine, le polypeptide pancréatique (PP), la ghreline et l'entérostatine qui ont des structures peptidiques ou protéiques. Elles stimulent également la sécrétion d'insuline, en réponse au glucose et aux acides gras et sont donc à ce titre des candidats potentiels pour le traitement du diabète.

Parmi celles-ci, les GLP-1 RA sont celles qui ont apporté à ce jour les meilleurs résultats dans le développement de médicaments. Elles ont permis à des patients atteints de diabète de type II de perdre du poids tout en ayant un meilleur contrôle de leur glycémie.

Des analogues ou des dérivés de GLP-1 RA ont ainsi été développés notamment pour améliorer leur stabilité.

D'autre part, pour couvrir ses besoins journaliers en insuline, un patient diabétique dispose, actuellement, de façon schématisée, de deux types d'insulines ayant des actions complémentaires : les insulines prandiales (ou insulines dites à action rapide) et les insulines basales (ou insulines dites à action lente).

Les insulines prandiales permettent une prise en charge rapide (métabolisation et/ou stockage) du glucose apporté lors des repas et collations. Le patient doit s'injecter une insuline prandiale avant chaque prise alimentaire, soit environ 2 à 3 injections par jour. Les insulines prandiales les plus utilisées sont : l'insuline humaine recombinante, NovoLog® (insuline aspart de NOVO NORDISK), Humalog® (insuline lispro de ELI LILLY) et Apidra® (insuline glulisine de SANOFI).

Les insulines basales assurent le maintien de l'homéostasie glycémique du patient, en dehors des périodes de prise alimentaire. Elles agissent essentiellement pour bloquer la production endogène de glucose (glucose hépatique). La dose journalière d'insuline basale correspond généralement à 40-50 % des besoins totaux journaliers en insuline. Selon l'insuline basale utilisée, cette dose est dispensée en 1 ou 2 injections, régulièrement réparties au cours de la journée. Les insulines basales les plus utilisées sont Levemir® (insuline detemir de NOVO NORDISK) et Lantus® (insuline glargine de SANOFI).

On notera pour être exhaustif que la NPH (insuline NPH pour Neutral Protamine Hagedorn ; Humuline NPH®, Insulatard®) est la plus ancienne insuline basale. Cette formulation est le résultat d'une précipitation de l'insuline humaine (anionique à pH neutre) par une protéine cationique, la protamine. Les microcristaux ainsi formés sont dispersés dans une suspension aqueuse et se dissolvent lentement après injection sous-cutanée. Cette dissolution lente assure une libération prolongée de l'insuline. Cependant cette libération n'assure pas une concentration constante d'insuline au cours du temps. Le profil de libération est en forme de cloche et dure seulement entre 12 et 16 heures. Elle est donc injectée deux fois par jour. Cette insuline basale NPH est bien moins performante que les insulines basales modernes, Levemir® et Lantus®. La NPH est une insuline basale à action intermédiaire.

Le principe de la NPH a évolué avec l'apparition des insulines analogues rapides pour donner des produits appelés « Premix » offrant à la fois une action rapide et une action intermédiaire. NovoLog Mix® (NOVO NORDISK) et Humalog Mix® (ELI LILLY) sont des formulations comprenant une insuline analogue rapide, Novolog® et Humalog®, complexée partiellement par la protamine. Ces formulations contiennent ainsi des microcristaux d'insuline analogue dont l'action est dite intermédiaire et une partie d'insuline restée soluble dont l'action est rapide. Ces formulations offrent bien l'avantage d'une insuline rapide mais elles ont aussi le défaut de la NPH, c.-à-d. une durée d'action limitée entre 12 et 16 heures et une insuline libérée en « cloche ». Cependant, ces produits permettent au patient de s'injecter en une seule fois une insuline basale à action intermédiaire avec une insuline prandiale à action rapide. Or nombreux sont les patients soucieux de réduire leur nombre d'injections.

Les insulines basales actuellement commercialisées peuvent être classées en fonction de la solution technique qui permet d'obtenir l'action prolongée et à ce jour deux approches sont utilisées.

La première, celle de l'insuline detemir est la liaison à l'albumine *in vivo.* Il s'agit d'un analogue, soluble à pH 7, qui comprend une chaine latérale d'acide gras (tetradecanoyl) fixée à la position B29 qui, *in vivo,* permet à cette insuline de s'associer à l'albumine. Son action prolongée est principalement due à cette affinité pour l'albumine après injection sous-cutanée.

Cependant son profil pharmacocinétique ne permet pas de couvrir une journée, ce qui fait qu'elle est le plus souvent utilisée en deux injections par jour.

Une autre insuline soluble à pH 7, est l'insuline degludec commercialisée sous le nom de Tresiba®^{d}. Elle comprend également une chaîne latérale d'acide gras fixée sur l'insuline (hexadecandioyl-γ-L-Glu).

La seconde, celle de l'insuline glargine, est la précipitation à pH physiologique. L'insuline glargine est un analogue de l'insuline humaine obtenu par élongation de la partie C-terminale de la chaine B de l'insuline humaine par deux résidus arginine, et par substitution du résidu d'asparagine A21, par un résidu de glycine (US 5,656,722). L'addition de deux résidus d'arginine a été pensée pour ajuster le pI (point isoélectrique) d'insuline glargine au pH physiologique, et ainsi rendre cet analogue de l'insuline humaine insoluble en milieu physiologique.

Aussi, la substitution de l'A21 a été pensée afin de rendre l'insuline glargine stable à pH acide et pouvoir ainsi la formuler sous forme de solution injectable à pH acide. Lors de l'injection sous-cutanée, le passage de l'insuline glargine d'un pH acide (pH 4-4,5) à un pH physiologique (pH neutre) provoque sa précipitation sous la peau. La redissolution lente des micro-particules d'insuline glargine assure une action lente et prolongée.

L'effet hypoglycémiant de l'insuline glargine est quasi-constant sur une durée de 24 heures ce qui permet à la plupart des patients de se limiter à une seule injection par jour.

L'insuline glargine est considérée aujourd'hui comme l'insuline basale la plus utilisée.

Cependant le pH nécessairement acide des formulations d'insulines basales, dont le point isoélectrique est compris entre 5,8 et 8,5, de type insuline glargine, peut être un réel inconvénient, car ce pH acide de la formulation d'insuline glargine entraîne parfois chez les patients des douleurs à l'injection et surtout empêche toute formulation avec d'autres protéines et en particulier avec les insulines prandiales car ces dernières ne sont pas stables à pH acide. L'impossibilité de formuler une insuline prandiale, à pH acide, tient au fait qu'une insuline prandiale subit, dans ces conditions, une réaction secondaire de déamidation en position A21, ce qui ne permet pas de répondre aux exigences de stabilité applicables aux médicaments injectables.

A ce jour, dans les demandes WO 2013/021143 A1, WO 2013/104861 A1, WO 2014/124994 A1 et WO 2014/124993 A1 il a été démontré qu'il était possible de solubiliser ces insulines basales, de type insuline glargine dont le point isoélectrique est compris entre 5,8 et 8,5, à pH neutre, tout en maintenant une différence de solubilité entre le milieu *in-vitro* (le contenant) et le milieu *in-vivo* (sous la peau), indépendamment du pH.

La demande WO 2013/104861 A1, en particulier, décrit des compositions sous forme d'une solution aqueuse injectable, dont le pH est compris entre 6,0 et 8,0, comprenant au moins (a) une insuline basale dont le point isoélectrique pI est compris entre 5,8 et 8,5 et (b) un co-polyaminoacide porteur de charges carboxylates substitué par des radicaux hydrophobes.

Ces compositions de l'art antérieur ont l'inconvénient majeur de ne pas être suffisamment stables pour répondre aux cahiers des charges applicables aux formulations pharmaceutiques.

Dans les exemples de la partie expérimentale de la présente demande de brevet il est démontré que les compositions décrites en particulier dans WO 2013/104861 A1 présentent une stabilité insatisfaisante dans le temps.

Il existe donc un besoin de trouver une solution qui permet de solubiliser une insuline basale dont le point isoélectrique (pI) est compris entre 5,8 et 8,5 tout en conservant son profil basal après injection mais qui permettent également de satisfaire à des conditions de stabilité physique standard pour les produits pharmaceutiques à base d'insuline.

De manière surprenante, la demanderesse a trouvé que les co-polyaminoacides porteurs de charges carboxylates et de radicaux hydrophobes selon l'invention permettent d'obtenir des compositions sous forme de solutions qui non seulement répondent aux exigences décrites dans WO 2013/104861 A1 mais qui de plus sont en mesure de conférer une stabilité physique améliorée auxdites compositions sans avoir à augmenter la quantité d'excipients utilisée.

Ces performances a priori jamais atteintes sont de plus conservées lorsque l'insuline basale dont le point isoélectrique est compris entre 5,8 et 8,5 est associée dans la composition avec une insuline prandiale et/ou une hormone gastro-intestinale.

Ainsi, de façon surprenante, l'affinité des co-polyaminoacides selon l'invention pour l'insuline glargine a été augmentée en ce qu'elle permet d'obtenir une solubilisation et une stabilisation des solutions d'insuline glargine à un ratio [Hy]/[insuline basale] inférieur à celui de l'art antérieur ; ces résultats sont de plus obtenus sans altérer, voire en améliorant, la propension de l'insuline glargine à précipiter comme cela est démontré dans la partie expérimentale.

Cette amélioration de l'affinité permet en outre dans le cadre de traitements chroniques de limiter le niveau d'exposition auxdits excipients.

Les co-polyaminoacides porteurs de charges carboxylates et de radicaux hydrophobes Hy selon l'invention présentent une excellente résistance à l'hydrolyse. Ceci peut notamment être vérifié en conditions accélérées, par exemple par des tests d'hydrolyse à pH basique (pH 12).

En outre des tests d'oxydation forcée, par exemple du type oxydation de Fenton, montrent que les co-polyaminoacides porteurs de charges carboxylates et de radicaux hydrophobes Hy présentent une bonne résistance à l'oxydation.

L'invention concerne ainsi une composition sous forme d'une solution aqueuse injectable, dont le pH est compris entre 6,0 et 8,0, comprenant au moins :
a) une insuline basale dont le point isoélectrique pI est compris entre 5,8 et 8,5 ;
b) un co-polyaminoacide porteur de charges carboxylates et de radicaux hydrophobes Hy, ledit co-polyaminoacide étant constitué d'unités glutamiques ou aspartiques et lesdits radicaux hydrophobes Hy étant de formule I suivante : 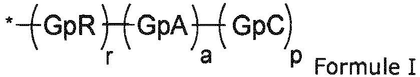 dans laquelle
   - GpR est un radical de formules II ou II' : 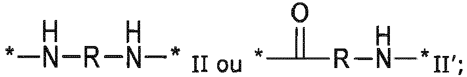
   - GpA est un radical de formules III ou III' : 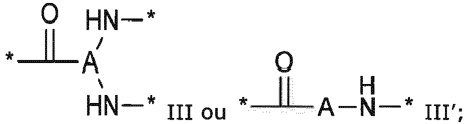
   - GpC est un radical de formule IV : 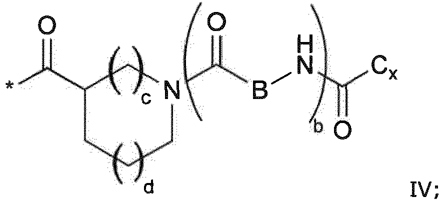
   - Hy comprend plus de 30 atomes de carbone,
   - les * indiquent les sites de rattachement des différents groupes liés par des fonctions amides ;
   - a est un entier égal à 0 ou à 1 ;
   - b est un entier égal à 0 ou à 1 ;
   - p est un entier égal à 1 ou à 2 et
      ∘ si p est égal à 1 alors a est égal à 0 ou à 1 et GpA est un radical de formule III' et,
      ∘ si p est égal à 2 alors a est égal à 1, et GpA est un radical de formule III ;
   - c est un entier égal à 0 ou à 1, et si c est égal à 0 alors d est égal à 1 ou à 2;
   - d est un entier égal à 0, à 1 ou à 2 ;
   - r est un entier égal à 0 ou à 1, et
      ∘ si r est égal à 0 alors le radical hydrophobe de formule I est lié au co-polyaminoacide via une liaison covalente entre un carbonyl du radical hydrophobe et un atome d'azote en position N terminale du co-polyaminoacide, formant ainsi une fonction amide issue de la réaction d'une fonction amine en position N terminale du précurseur du co-polyaminoacide et une fonction acide portée par le précurseur du radical hydrophobe , et ∘ si r est égal à 1 alors le radical hydrophobe de formule I est lié au co-polyaminoacide :
      ▪ via une liaison covalente entre un atome d'azote du radical hydrophobe et un carbonyl du co-polyaminoacide, formant ainsi une fonction amide issue de la réaction d'une fonction amine du précurseur du radical hydrophobe et une fonction acide portée par le précurseur du co-polyaminoacide ou
      ▪ via une liaison covalente entre un carbonyl du radical hydrophobe et un atome d'azote en position N-terminale du co-polyaminoacide, formant ainsi une fonction amide issue de la réaction d'une fonction acide du précurseur du radical hydrophobe et une fonction amine en position N-terminale portée par le précurseur du co-polyaminoacide ;
   - R est un radical choisi dans le groupe constitué par :
      ∘ un radical alkyle divalent, linéaire ou ramifié, comprenant si GpR est un radical de formule II de 2 à 12 atomes de carbone ou si GpR est un radical de formule II' de 1 à 11 atomes de carbone ;
      ∘ un radical alkyle divalent, linéaire ou ramifié, comprenant si GpR est un radical de formule II de 2 à 11 atomes de carbone ou si GpR est un radical de formule II' de 1 à 11 atomes de carbone, ledit radical alkyle portant une ou plusieurs fonctions -CONH₂, et
      ∘ un radical éther ou polyéther non substitué comprenant de 4 à 14 atomes de carbone et de 1 à 5 atomes d'oxygène ;
   - A est un radical alkyle linéaire ou ramifié comprenant de 1 à 6 atomes de carbone;
   - B est un radical alkyle linéaire ou ramifié, éventuellement comprenant un noyau aromatique, comprenant de 1 à 9 atomes de carbone ;
   - Cₓ est un radical alkyl monovalent linéaire ou ramifié, dans lequel x indique le nombre d'atomes de carbone et :
      ∘ si p est égal à 1, x est compris entre 11 à 25 (11 ≤ x ≤ 25) ;
      ∘ si p est égal à 2, x est compris entre 9 et 15 (9 ≤ x ≤ 15),
   - le ratio i entre le nombre de radicaux hydrophobes et le nombre d'unités glutamiques ou aspartiques étant compris entre entre 0 < i ≤ 0,5 ;
   - lorsque plusieurs radicaux hydrophobes sont portés par un co-polyaminoacide alors ils sont identiques ou différents,
   - le degré de polymérisation DP en unités glutamiques ou aspartiques est compris entre 5 et 250 ;
   - les fonctions acides libres étant sous forme de sel de cation alkalin choisi dans le groupe constitué par Na⁺ et K⁺.

La présente invention concerne également le copolyaminoacide tel que revendiqué à la revendication 15.

Le pH des compositions selon l'invention est compris entre 6,0 et 8,0, de préférence compris entre 6,6 et 7,8 ou encore plus préférentiellement entre 6,8 et 7,6.

Ledit co-polyaminoacide porteur de charges carboxylates et de radicaux hydrophobes Hy est soluble en solution aqueuse à pH compris entre 6 et 8, à une température de 25°C et à une concentration inférieure à 60 mg/ml.

On entend par « composition stable physiquement » des compositions qui satisfont aux critères de l'inspection visuelle décrite dans la pharmacopée européenne, américaine et internationale, c'est-à-dire des compositions qui sont claires et qui ne contiennent pas de particules visibles, mais également incolores.

On entend par « solution aqueuse injectable » des solutions dont le solvant est l'eau et qui satisfont aux conditions des pharmacopées EP et US.

On entend par « co-polyaminoacide étant constitué d'unités glutamiques ou aspartiques » des enchainements linéaires non cycliques d'unités acide glutamique ou acide aspartique liées entre elles par des liaisons peptidiques, lesdits enchainements présentant une partie C-terminale, correspondant à l'acide carboxylique d'une extrémité, et une partie N-terminale, correspondant à l'amine de l'autre extrémité de l'enchainement.

On entend par « soluble », susceptible de permettre de préparer une solution limpide et dépourvue de particules à une concentration inférieure à 60 mg/ml dans de l'eau distillée à 25°C.

On entend par « radical alkyl » une chaine carbonée, linéaire ou ramifiée, qui ne comprend pas d'hétéroatome.

Le co-polyaminoacide est un co-polyaminoacide statistique dans l'enchaînement des unités glutamiques et/ou aspartiques.

Dans les formules les * indiquent les sites de rattachements des différents éléments représentés.

Dans un mode de réalisation, la composition selon l'invention est caractérisée en ce que Hy comprend plus de 30 atomes de carbone.

Dans un mode de réalisation, la composition selon l'invention est caractérisée en ce que Hy comprend entre 30 et 70 atomes de carbone.

Dans un mode de réalisation, lorsque p = 1, x est compris entre 11 et 25 (11 ≤ x ≤ 25). En particulier, lorsque x est compris entre 15 et 16 (x = 15 ou 16) alors r = 1 et R est un radical éther ou polyéther et lorsque x est supérieur à 17 (x ≥ 17) alors r = 1 et R est un radical éther ou polyéther.

Dans un mode de réalisation, lorsque p = 2, x est compris entre 9 et 15 (9 ≤ x ≤ 15).

Dans un mode de réalisation, la composition selon l'invention est caractérisée en ce que lesdits radicaux hydrophobes sont choisis parmi les radicaux hydrophobes de formule I dans laquelle p = 1, représentée par la formule V suivante : 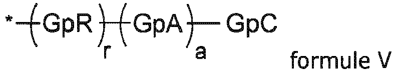 GpR, GpA, GpC, r et a ont les définitions données précédemment.

Dans un mode de réalisation, la composition selon l'invention est caractérisée en ce que le radical hydrophobe est un radical de formule V dans laquelle r est égal à 1 (r=1) et a est égal à 0 (a = 0).

Dans un mode de réalisation, la composition selon l'invention est caractérisée en ce que le radical hydrophobe est un radical de formule V dans laquelle r est égal à 1 (r=1) et a est égal à 1 (a=1).

Dans un mode de réalisation, la composition selon l'invention est caractérisée en ce que le radical hydrophobe est un radical de formule V dans laquelle GpR est un radical de formule II.

Dans un mode de réalisation, la composition selon l'invention est caractérisée en ce que le radical hydrophobe est un radical de formule V dans laquelle GpR est un radical de formule II dans laquelle R est un radical alkyle linéaire divalent comprenant de 2 à 12 atomes de carbone.

Dans un mode de réalisation, la composition selon l'invention est caractérisée en ce que le radical hydrophobe est un radical de formule V dans laquelle GpR est un radical de formule II dans laquelle R est un radical alkyle divalent comprenant de 2 à 6 atomes de carbone.

Dans un mode de réalisation, la composition selon l'invention est caractérisée en ce que le radical hydrophobe est un radical de formule V dans laquelle GpR est un radical de formule II dans laquelle R est un radical alkyle linéaire divalent comprenant de 2 à 6 atomes de carbone.

Dans un mode de réalisation, la composition selon l'invention est caractérisée en ce que le radical hydrophobe est un radical de formule V dans laquelle GpR est un radical de formule II dans laquelle R est un radical alkyle divalent comprenant de 2 à 4 atomes de carbone.

Dans un mode de réalisation, la composition selon l'invention est caractérisée en ce que le radical hydrophobe est un radical de formule V dans laquelle GpR est un radical de formule II dans laquelle R est un radical alkyle linéaire divalent comprenant de 2 à 4 atomes de carbone.

Dans un mode de réalisation, la composition selon l'invention est caractérisée en ce que le radical hydrophobe est un radical de formule V dans laquelle GpR est un radical de formule II dans laquelle R est un radical alkyle divalent comprenant 2 atomes de carbone.

Dans un mode de réalisation, la composition selon l'invention est caractérisée en ce que le radical hydrophobe est un radical de formule V dans laquelle GpR est un radical de formule II dans laquelle R est un radical alkyle divalent comprenant 6 atomes de carbone.

Dans un mode de réalisation, la composition selon l'invention est caractérisée en ce que le radical hydrophobe est un radical de formule V dans laquelle GpR est un radical de formule II'.

Dans un mode de réalisation, la composition selon l'invention est caractérisée en ce que le radical hydrophobe est un radical de formule V dans laquelle GpR est un radical de formule II' dans laquelle R est un radical alkyle linéaire divalent comprenant de 1 à 11 atomes de carbone.

Dans un mode de réalisation, la composition selon l'invention est caractérisée en ce que le radical hydrophobe est un radical de formule V dans laquelle GpR est un radical de formule II' dans laquelle R est un radical alkyle divalent comprenant de 1 à 6 atomes de carbone.

Dans un mode de réalisation, la composition selon l'invention est caractérisée en ce que le radical hydrophobe est un radical de formule V dans laquelle GpR est un radical de formule II ou II', dans laquelle R est un radical alkyle divalent, comprenant de 2 à 5 atomes de carbone et portant une ou plusieurs fonctions amide (-CONHz).

Dans un mode de réalisation, la composition selon l'invention est caractérisée en ce que le radical hydrophobe est un radical de formule V dans laquelle GpR est un radical de formule II' ou II, dans laquelle R est un radical alkyle linéaire divalent, comprenant de 2 à 5 atomes de carbone et portant une ou plusieurs fonctions amide (-CONH2).

Dans un mode de réalisation, la composition selon l'invention est, caractérisée en ce que le radical hydrophobe est un radical de formule V dans laquelle GpR est un radical de formule II ou II' dans laquelle R est un radical choisi dans le groupe constitué par les radicaux représentés par les formules ci-dessous :

| | |
|---|---|
| 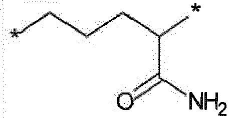 | Formule X1 |
| 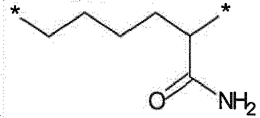 | Formule X2 |

Dans un mode de réalisation, la composition est caractérisée en ce que le radical hydrophobe de formule V dans laquelle GpR est un radical de formule II ou II', dans laquelle R est un radical de formule X1.

Dans un mode de réalisation, la composition est caractérisée en ce que le radical hydrophobe de formule V dans laquelle GpR est un radical de formule II ou II', dans laquelle R est un radical de formule X2.

Dans un mode de réalisation, la composition selon l'invention est caractérisée en ce que le radical R est lié au co-polyaminoacide via une fonction amide portée par le carbone en position delta ou epsilon (ou en position 4 ou 5 par rapport à la fonction amide (-CONH₂).

Dans un mode de réalisation, la composition selon l'invention est caractérisée en ce que le radical hydrophobe est un radical de formule V dans laquelle GpR est un radical de formule II ou II', dans laquelle R est un radical linéaire éther ou polyéther non substitué comprenant de 4 à 14 atomes de carbone et de 1 à 5 atomes d'oxygène.

Dans un mode de réalisation, la composition selon l'invention est caractérisée en ce que le radical hydrophobe est un radical de formule V dans laquelle GpR est un radical de formule II ou II', dans laquelle R est un radical éther.

Dans un mode de réalisation, la composition selon l'invention est caractérisée en ce que le radical hydrophobe est un radical de formule V laquelle GpR est un radical de formule II ou II', dans laquelle R est un radical éther comprenant de 4 à 6 atomes de carbone.

Dans un mode de réalisation, la composition selon l'invention est caractérisée en ce que le radical hydrophobe est un radical de formule V dans laquelle GpR est un radical de formule II ou II' dans laquelle R est un radical éther représenté par la formule 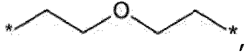 de formule X7.

Dans un mode de réalisation, la composition selon l'invention est caractérisée en ce que le radical hydrophobe est un radical de formule V dans laquelle GpR est un radical de formule II ou II', dans laquelle R est un radical polyéther.

Dans un mode de réalisation, la composition selon l'invention est caractérisée en ce que le radical hydrophobe est un radical de formule V dans laquelle GpR est un radical de formule II ou II', dans laquelle R est un radical linéaire polyéther comprenant de 6 à 10 atomes de carbone et de 2 à 3 atomes d'oxygène.

Dans un mode de réalisation, la composition selon l'invention est caractérisée en ce que le radical hydrophobe de formule V dans laquelle GpR est un radical de formule II ou II', dans laquelle R est un radical polyéther choisi dans le groupe constitué par les radicaux représentés par les formules ci-dessous :

| | |
|---|---|
| 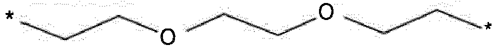 | Formule X3 |
| 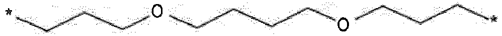 | Formule X4 |
| 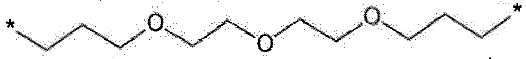 | Formule X5 |
| 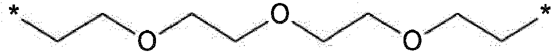 | Formule X6 |

Dans un mode de réalisation, la composition est caractérisée en ce que le radical hydrophobe de formule V dans laquelle GpR est un radical de formule II ou II', dans laquelle R est un radical de formule X3.

Dans un mode de réalisation, la composition est caractérisée en ce que le radical hydrophobe de formule V dans laquelle GpR est un radical de formule II ou II', dans laquelle R est un radical de formule X4.

Dans un mode de réalisation, la composition est caractérisée en ce que le radical hydrophobe de formule V dans laquelle GpR est un radical de formule II ou II', dans laquelle R est un radical de formule X5.

Dans un mode de réalisation, la composition est caractérisée en ce que le radical hydrophobe de formule V dans laquelle GpR est un radical de formule II ou II', dans laquelle R est un radical de formule X6.

Dans un mode de réalisation, la composition selon l'invention est caractérisée en ce que le radical hydrophobe de formule V dans laquelle GpR est un radical dans laquelle R est un radical polyéther choisi dans le groupe constitué par les radicaux représentés par les formules ci-dessous :

| | |
|---|---|
| 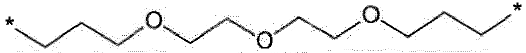 | Formule X5 |
| 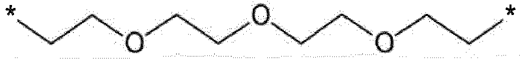 | Formule X6 |

Dans un mode de réalisation, la composition est caractérisée en ce que le radical hydrophobe de formule V dans laquelle GpR est un radical de formule II dans laquelle R est un radical polyéther de formule X5.

Dans un mode de réalisation, la composition est caractérisée en ce que le radical hydrophobe de formule V dans laquelle GpR est un radical de formule II dans laquelle R est un radical polyéther de formule X6.

Dans un mode de réalisation, la composition selon l'invention est caractérisée en ce que le radical hydrophobe est un radical de formule V dans laquelle a est égal à 0 (a=0) et r est égal à 0 (r=0).

Dans un mode de réalisation, la composition selon l'invention est caractérisée en ce que le radical hydrophobe est un radical de formule V dans laquelle a est égal à 1 (a = 1) et le radical GpA de formule III' est choisi dans le groupe constitué des radicaux représentés par les formules ci-dessous :

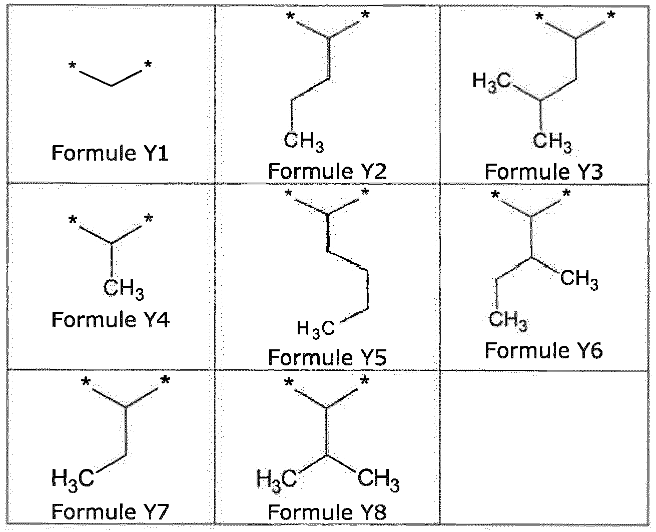

Dans un mode de réalisation, la composition est caractérisée en ce que le radical hydrophobe est un radical de formule V dans laquelle a est égal à 1 (a = 1) et le radical GpA de formule III' est un radical de formule Y1.

Dans un mode de réalisation, la composition est caractérisée en ce que le radical hydrophobe est un radical de formule V dans laquelle a est égal à 1 (a = 1) et le radical GpA de formule III' est un radical de formule Y2.

Dans un mode de réalisation, la composition est caractérisée en ce que le radical hydrophobe est un radical de formule V dans laquelle a est égal à 1 (a = 1) et le radical GpA de formule III' est un radical de formule Y3.

Dans un mode de réalisation, la composition est caractérisée en ce que le radical hydrophobe est un radical de formule V dans laquelle a est égal à 1 (a = 1) et le radical GpA de formule III' est un radical de formule Y4.

Dans un mode de réalisation, la composition est caractérisée en ce que le radical hydrophobe est un radical de formule V dans laquelle a est égal à 1 (a = 1) et le radical GpA de formule III' est un radical de formule Y5.

Dans un mode de réalisation, la composition est caractérisée en ce que le radical hydrophobe est un radical de formule V dans laquelle a est égal à 1 (a = 1) et le radical GpA de formule III' est un radical de formule Y6.

Dans un mode de réalisation, la composition est caractérisée en ce que le radical hydrophobe est un radical de formule V dans laquelle a est égal à 1 (a = 1) et le radical GpA de formule III' est un radical de formule Y7.

Dans un mode de réalisation, la composition est caractérisée en ce que le radical hydrophobe est un radical de formule V dans laquelle a est égal à 1 (a = 1) et le radical GpA de formule III' est un radical de formule Y8.

Dans un mode de réalisation, la composition selon l'invention est caractérisée en ce que le radical hydrophobe est un radical de formule V dans laquelle le radical GpC de formule IV est choisi dans le groupe constitué des radicaux de formules IVa, IVb ou IVc ci-après représentées :

| | |
|---|---|
| 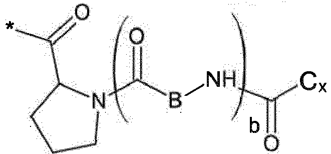 | Formule IVa |
| 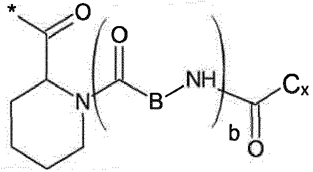 | Formule IVb |
| 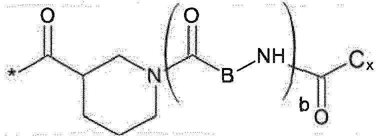 | Formule IVc |

Dans un mode de réalisation, la composition selon l'invention est caractérisée en ce que le radical hydrophobe est un radical de formule V dans laquelle le radical GpC est de formule IVa.

Dans un mode de réalisation, la composition selon l'invention est caractérisée en ce que le radical hydrophobe est un radical de formule V dans laquelle le radical GpC de formule IV est choisi dans le groupe constitué des radicaux de formules IVa, IVb ou IVc dans lesquels b est égal à 0, répondant respectivement aux formules IVd, IVe, et IVf ci-après représentées :

| | |
|---|---|
| 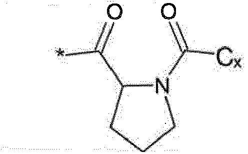 | Formule IVd |
| 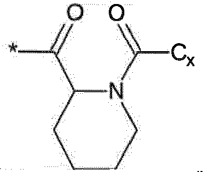 | Formule IVe |
| 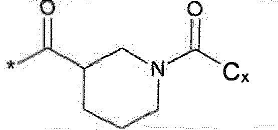 | Formule IVf |

Dans un mode de réalisation, la composition selon l'invention est caractérisée en ce que le radical hydrophobe est un radical de formule V dans laquelle le radical GpC répond à la formule IV ou IVa dans lesquelles b = 0, et répond à la formule IVd.

Dans un mode de réalisation, la composition selon l'invention est caractérisée en ce que le radical hydrophobe est un radical de formule V dans laquelle le radical GpC de formule IV dans laquelle b = 1 est choisi dans le groupe constitué des radicaux dans lesquels B est un résidu d'acide aminé choisi dans le groupe constitué par les radicaux représentés par les formules ci-dessous :

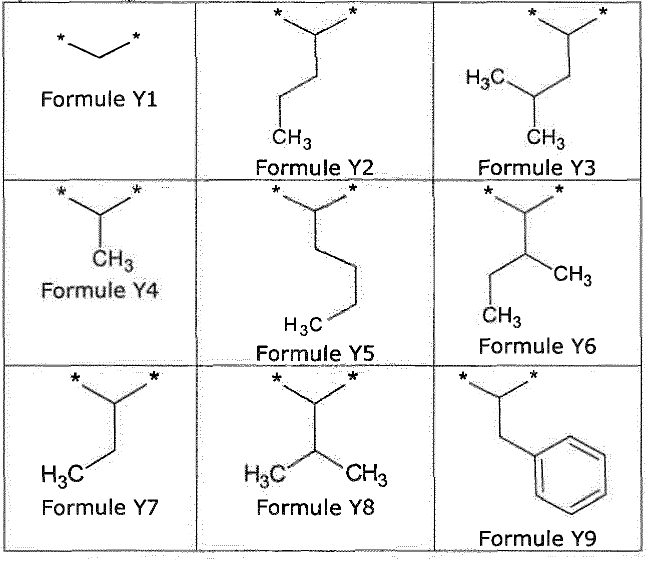

Dans un mode de réalisation, la composition selon l'invention est caractérisée en ce que le radical hydrophobe est un radical de formule V dans laquelle le radical GpC de formule répond à la formule IV ou IVa dans lesquelles b = 1, est choisi dans le groupe constitué des radicaux dans lesquels B est un résidu d'acide aminé choisi dans le groupe constitué par les radicaux représentés par les formules ci-dessous :

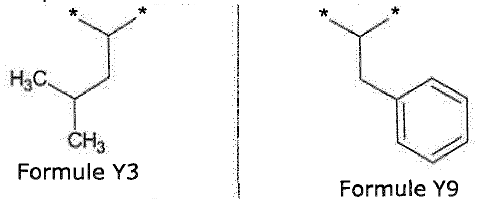

Dans un mode de réalisation, la composition selon l'invention est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles linéaires.

Dans un mode de réalisation, la composition selon l'invention est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles ramifiés.

Dans un mode de réalisation, la composition selon l'invention est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles comprenant entre 11 et 14 atomes de carbone.

Dans un mode de réalisation, la composition selon l'invention est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux représentés par les formules ci-dessous :

| | |
|---|---|
| 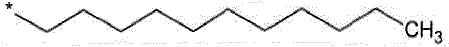 | x=11 |
| 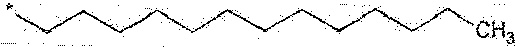 | x=13 |

Dans un mode de réalisation, la composition selon l'invention est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles comprenant entre 15 et 16 atomes de carbone.

Dans un mode de réalisation, la composition selon l'invention est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux représentés par les formules ci-dessous :

| | |
|---|---|
| 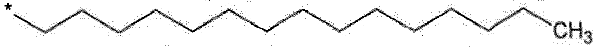 | x=15 |

Dans un mode de réalisation, la composition selon l'invention est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux représentés par les formules ci-dessous :

| | |
|---|---|
| 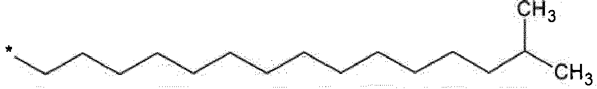 | x=16 |

Dans un mode de réalisation, la composition selon l'invention est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles comprenant entre 17 et 25 atomes de carbone.

Dans un mode de réalisation, la composition selon l'invention est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles comprenant entre 17 et 18 atomes de carbone.

Dans un mode de réalisation, la composition selon l'invention est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles représentés par les formules ci-dessous :

| | |
|---|---|
| 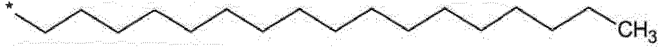 | x=17 |

Dans un mode de réalisation, la composition selon l'invention est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles comprenant entre 19 et 25 atomes de carbone.

Dans un mode de réalisation, la composition selon l'invention est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles représentés par les formules ci-dessous :

| | |
|---|---|
| 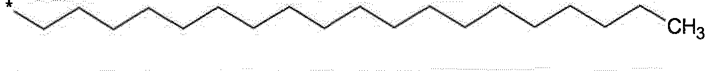 | x=19 |
| 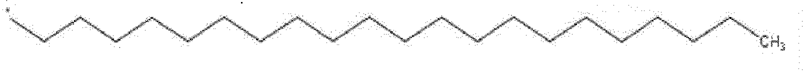 | x = 21 |

Dans un mode de réalisation, la composition selon l'invention est caractérisée en ce que le radical hydrophobe est un radical de formule V dans laquelle le radical GpC de formule IV est choisi dans le groupe constitué des radicaux dans lesquels Cx est choisi dans le groupe constitué par les radicaux alkyles comprenant entre 18 et 19 atomes de carbone.

Dans un mode de réalisation, la composition selon l'invention est caractérisée en ce que lesdits radicaux hydrophobes de formule I sont choisis parmi les radicaux hydrophobes de formule I dans laquelle a = 1 et p = 2, représentée par la formule VI suivante : 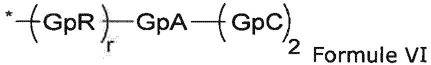 dans laquelle
GpR, GpA, GpC, r et a ont les définitions données précédemment.

Dans un mode de réalisation, la composition selon l'invention est caractérisée en ce que le radical hydrophobe est un radical de formule VI dans laquelle GpR est un radical de formule II.

Dans un mode de réalisation, la composition selon l'invention est caractérisée en ce que le radical hydrophobe est un radical de formule VI dans laquelle GpR est un radical de formule II dans lequel R est un radical alkyle linéaire divalent comprenant de 2 à 12 atomes de carbone.

Dans un mode de réalisation, la composition selon l'invention est caractérisée en ce que le radical hydrophobe est un radical de formule VI dans laquelle GpR est un radical de formule II dans laquelle R est un radical alkyle divalent comprenant de 2 à 6 atomes de carbone.

Dans un mode de réalisation, la composition selon l'invention est caractérisée en ce que le radical hydrophobe est un radical de formule VI dans laquelle GpR est un radical de formule II dans laquelle R est un radical alkyle linéaire divalent comprenant de 2 à 6 atomes de carbone.

Dans un mode de réalisation, la composition selon l'invention est caractérisée en ce que le radical hydrophobe est un radical de formule VI dans laquelle GpR est un radical de formule II dans laquelle R est un radical alkyle au moins comprenant de 2 à 4 atomes de carbone.

Dans un mode de réalisation, la composition selon l'invention est caractérisée en ce que le radical hydrophobe est un radical de formule VI dans laquelle GpR est un radical de formule II dans laquelle R est un radical alkyle linéaire divalent comprenant de 2 à 4 atomes de carbone.

Dans un mode de réalisation, la composition selon l'invention est caractérisée en ce que le radical hydrophobe est un radical de formule VI dans laquelle GpR est un radical de formule II dans laquelle R est un radical alkyle linéaire divalent comprenant 2 atomes de carbone.

Dans un mode de réalisation, la composition selon l'invention est caractérisée en ce que le radical hydrophobe est un radical de formule VI dans laquelle GpR est un radical de formule II'.

Dans un mode de réalisation, la composition selon l'invention est caractérisée en ce que le radical hydrophobe est un radical de formule VI dans laquelle GpR est un radical de formule II' dans laquelle R est un radical alkyle linéaire divalent comprenant de 1 à 11 atomes de carbone.

Dans un mode de réalisation, la composition selon l'invention est caractérisée en ce que le radical hydrophobe est un radical de formule VI dans laquelle GpR est un radical de formule II' dans laquelle R est un radical alkyle divalent comprenant de 1 à 6 atomes de carbone.

Dans un mode de réalisation, la composition selon l'invention est caractérisée en ce que le radical hydrophobe est un radical de formule VI dans laquelle GpR est un radical de formule II ou II', dans laquelle R est un radical alkyle divalent, comprenant de 2 à 5 atomes de carbone, et portant une ou plusieurs fonctions amide (-CONH2).

Dans un mode de réalisation, la composition selon l'invention est caractérisée en ce que le radical hydrophobe est un radical de formule VI dans laquelle GpR est un radical de formule II ou II', dans laquelle R est un radical alkyle linéaire divalent, comprenant de 2 à 5 atomes de carbone et portant une ou plusieurs fonctions amide (-CONH2).

Dans un mode de réalisation, la composition selon l'invention est caractérisée en ce que le radical hydrophobe est un radical de formule VI dans laquelle GpR est un radical de formule II ou II' dans laquelle R est un radical choisi dans le groupe constitué par les radicaux représentés par les formules ci-dessous :

| | |
|---|---|
| 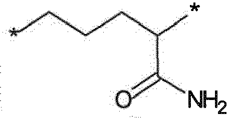 | Formule X1 |
| 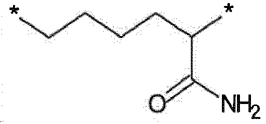 | Formule X2 |

Dans un mode de réalisation, la composition selon l'invention est caractérisée en ce que le radical hydrophobe est un radical de formule VI dans laquelle la fonction amine du radical GpR engagée dans la formation de la fonction amide qui lie ledit radical GpR au co-polyaminoacide est portée par un carbone en position delta ou epsilon (ou en position 4 ou 5) par rapport à la fonction amide (-CONH2).

Dans un mode de réalisation, la composition selon l'invention est caractérisée en ce que le radical hydrophobe est un radical de formule VI dans laquelle GpR est un radical de formule II ou II', dans laquelle R est un radical linéaire éther ou polyéther non substitué comprenant de 4 à 14 atomes de carbone et de 1 à 5 atomes d'oxygène.

Dans un mode de réalisation, la composition selon l'invention est caractérisée en ce que le radical hydrophobe est un radical de formule VI dans laquelle GpR est un radical de formule II ou II' dans laquelle R est un radical éther.

Dans un mode de réalisation, la composition selon l'invention est caractérisée en ce que le radical éther R est un radical comprenant de 4 à 6 atomes de carbone.

Dans un mode de réalisation, la composition selon l'invention est caractérisée en ce que le radical éther est 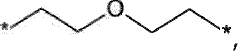 de formule X7.

Dans un mode de réalisation, la composition selon l'invention est caractérisée en ce que le radical hydrophobe est un radical de formule VI dans laquelle GpR est un radical de formule II ou II', dans laquelle R est un radical polyéther.

Dans un mode de réalisation, la composition selon l'invention est caractérisée en ce que le radical hydrophobe est un radical de formule VI dans laquelle GpR est un radical de formule II ou II', dans laquelle R est un radical linéaire polyéther comprenant de 6 à 10 atomes de carbone et de 2 à 3 atomes d'oxygène.

Dans un mode de réalisation, la composition selon l'invention est caractérisée en ce que le radical hydrophobe est un radical de formule VI dans laquelle GpR est un radical de formule II ou II' dans laquelle R est un radical linéaire polyéther choisi dans le groupe constitué par les radicaux représentés par les formules ci-dessous :

| | |
|---|---|
| 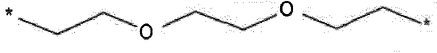 | Formule X3 |
| 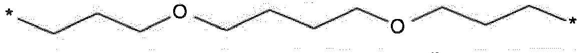 | Formule X4 |
| 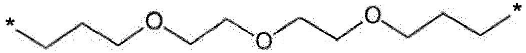 | Formule X5 |
| 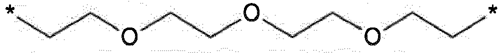 | Formule X6 |

Dans un mode de réalisation, la composition est caractérisée en ce que le radical hydrophobe est un radical de formule VI dans laquelle GpR est un radical de formule II ou II' dans laquelle R est un radical linéaire polyéther de formule X3.

Dans un mode de réalisation, la composition est caractérisée en ce que le radical hydrophobe est un radical de formule VI dans laquelle GpR est un radical de formule II ou II' dans laquelle R est un radical linéaire polyéther de formule X4.

Dans un mode de réalisation, la composition est caractérisée en ce que le radical hydrophobe est un radical de formule VI dans laquelle GpR est un radical de formule II ou II' dans laquelle R est un radical linéaire polyéther de formule X5.

Dans un mode de réalisation, la composition est caractérisée en ce que le radical hydrophobe est un radical de formule VI dans laquelle GpR est un radical de formule II ou II' dans laquelle R est un radical linéaire polyéther de formule X6.

Dans un mode de réalisation, la composition selon l'invention est caractérisée en ce que le radical hydrophobe est un radical de formule VI dans laquelle le radical GpA de formule III est choisi dans le groupe constitué des radicaux de formules IIIa, IIIb et IIIc ci-après représentées :

| | |
|---|---|
| 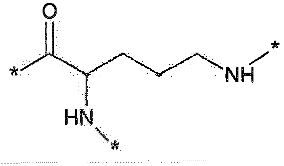 | Formule IIIa |
| 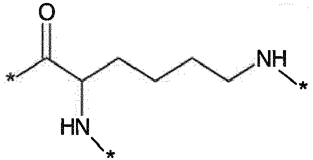 | Formule IIIb |
| 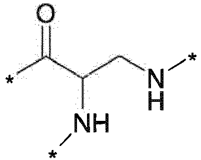 | Formule IIIc |

Dans un mode de réalisation, la composition selon l'invention est caractérisée en ce que le radical hydrophobe est un radical de formule VI dans laquelle le radical GpA de formule III est un radical de formule IIIb ci-après représentée :

| | |
|---|---|
| 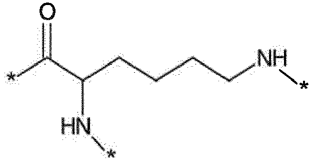 | Formule IIIb |

Dans un mode de réalisation, la composition selon l'invention est caractérisée en ce que le radical hydrophobe est un radical de formule VI dans laquelle le radical GpA de formule III est un radical de formule IIIc.

Dans un mode de réalisation, la composition selon l'invention est caractérisée en ce que le radical hydrophobe est un radical de formule VI dans laquelle le radical GpC de formule IV est choisi dans le groupe constitué des radicaux de formules IVa, IVb et IVc ci-après représentées :

| | |
|---|---|
| 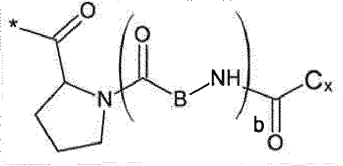 | Formule IVₐ |
| 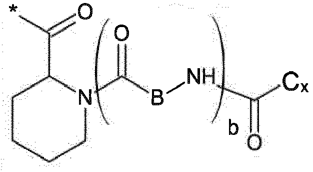 | Formule IV_{b} |
| 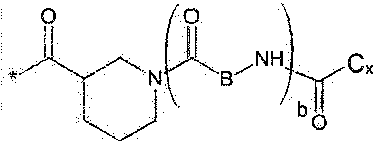 | Formule IV_{c} |

Dans un mode de réalisation, la composition selon l'invention est caractérisée en ce que le radical hydrophobe est un radical de formule VI dans laquelle le radical GpC est de formule IVa.

Dans un mode de réalisation, la composition selon l'invention est caractérisée en ce que le radical hydrophobe est un radical de formule VI dans laquelle le radical GpC de formule IV est choisi dans le groupe constitué des radicaux de formules IVa, IVb ou IVc dans lesquels b est égal à 0, répondant respectivement aux formules IVd, IVe, et IVf ci-après représentées :

| | |
|---|---|
| 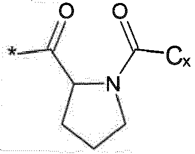 | Formule IV_{d} |
| 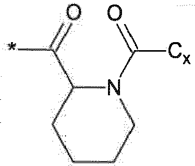 | Formule IVₑ |
| 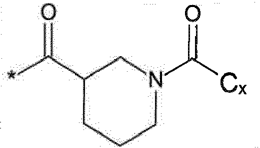 | Formule IVf |

Dans un mode de réalisation, la composition selon l'invention est caractérisée en ce que le radical hydrophobe est un radical de formule VI dans laquelle le radical GpC répond à la formule IV ou IVa dans lesquelles b = 0, et répond à la formule IVd.

Dans un mode de réalisation, la composition selon l'invention est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux alkyles linéaires comprenant entre 9 et 15 atomes de carbone.

Dans un mode de réalisation, la composition selon l'invention est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux alkyles ramifiés comprenant entre 9 et 15 atomes de carbone.

Dans un mode de réalisation, la composition selon l'invention est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux alkyles comprenant 9 ou 10 atomes de carbone.

Dans un mode de réalisation, la composition selon l'invention est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux représentés par les formules ci-dessous :

| | |
|---|---|
| 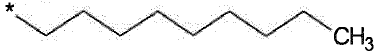 | x = 9 |

Dans un mode de réalisation, la composition selon l'invention est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux alkyles comprenant entre 11 et 15 atomes de carbone.

Dans un mode de réalisation, la composition selon l'invention est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux alkyles comprenant entre 11 et 13 atomes de carbone.

Dans un mode de réalisation, la composition selon l'invention est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux représentés par les formules ci-dessous :

| | |
|---|---|
| 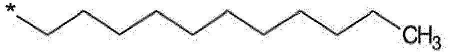 | x = 11 |
| 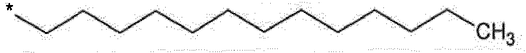 | x = 13 |

Dans un mode de réalisation, la composition selon l'invention est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux alkyles comprenant 14 ou 15 atomes de carbone.

Dans un mode de réalisation, la composition selon l'invention est caractérisée en ce que le radical hydrophobe est un radical de formule VI dans laquelle le radical GpC de formule IV est choisi dans le groupe constitué des radicaux dans lesquels Cx est choisi dans le groupe constitué par les radicaux représentés par les formules ci-dessous :

| | |
|---|---|
| 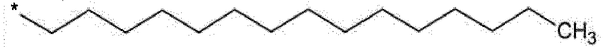 | x=15 |

Dans les formules I, V et VI, les * indiquent les sites de rattachement des radicaux hydrophobes au co-polyaminoacide. Les radicaux Hy sont rattachés au co-polyaminoacide via des fonctions amides.

Dans les formules II et II', les * indiquent, de gauche à droite respectivement, les sites de rattachement de GpR :
- au co-polyaminoacide et
- à GpA si a = 1 ou à GpC si a = 0.

Dans les formules III et III', les * indiquent, de gauche à droite respectivement, les sites de rattachement de GpA :
- à GpR si r = 1 ou au co-polyaminoacide si r = 0 et
- à GpC.

Dans la formule IV, le * indique le site de rattachement de GpC :
- à GpA si a = 1, GpR si r = 1 et a = 0 ou au co-polyaminoacide si r = 0 et a = 0.

Tous les rattachements entre les différents groupes GpR, GpA et GpC sont des fonctions amides.

Les radicaux Hy, GpR, GpA, GpC, et D sont chacun indépendamment identiques ou différents d'un résidu à l'autre.

Lorsque le co-polyaminoacide comprend une ou plusieurs d'unité(s) aspartique(s), celle(s)-ci peu(ven)t subir des réarrangements structuraux.

Dans un mode de réalisation, la composition selon l'invention est caractérisée en ce que le co-polyaminoacide porteur de charges carboxylates et de radicaux hydrophobes est choisi parmi les co-polyaminoacides de formule VII suivante : 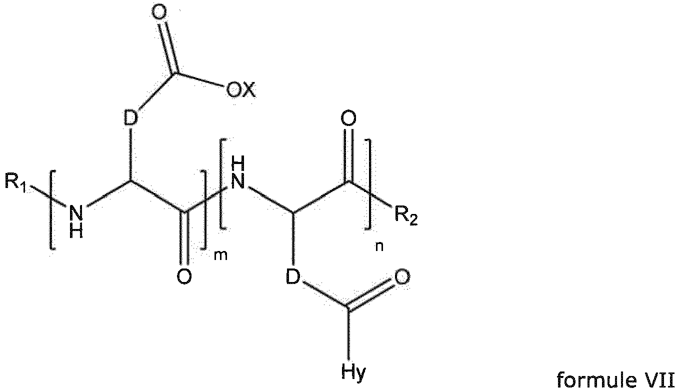 dans laquelle,
- D représente, indépendamment, soit un groupe -CH₂- (unité aspartique) soit un groupe -CH₂-CH₂- (unité glutamique),
- Hy est un radical hydrophobe choisi parmi les radicaux hydrophobes de formules I, V ou VI, dans lesquelles r = 1 et GpR est un radical de Formule II,
- R₁ est un radical hydrophobe choisi parmi les radicaux hydrophobes de formules I, V ou VI dans lesquelles r = 0 ou r = 1 et GpR est un radical de Formule II', ou un radical choisi dans le groupe constitué par un H, un groupe acyle linéaire en C2 à C10, un groupe acyle ramifié en C4 à C10, un benzyle, une unité « acide aminé » terminale et un pyroglutamate,
- R₂ est un radical hydrophobe choisi parmi les radicaux hydrophobes de formules I, V ou VI dans lesquelles r = 1 et GpR est un radical de Formule II, ou un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S ;
- X représente une entité cationique choisie dans le groupe comprenant les cations alcalins ;
- n + m représente le degré de polymérisation DP du co-polyaminoacide, c'est-à-dire le nombre moyen d'unités monomériques par chaîne de co-polyaminoacide et 5 ≤ n + m ≤ 250 ;

Dans un mode de réalisation, la composition selon l'invention est caractérisée en ce que lorsque le co-polyaminoacide comprend des unités aspartate, alors le co-polyaminoacide peut en outre comprendre des unités monomériques de formule VIII et/ou VIII' :

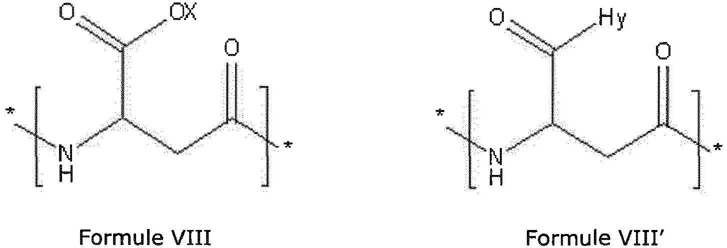

On appelle « co-polyaminoacide à greffage statistique » un co-polyaminoacide porteur de charges carboxylates et d'au moins un radical hydrophobe, un co-polyaminoacide de formule VIIa.

Dans un mode de réalisation, la composition selon l'invention est caractérisée en ce que le co-polyaminoacide porteur de charges carboxylates et de radicaux hydrophobes est choisi parmi les co-polyaminoacides de formules VII, dans laquelle R₁ = R'₁ et R₂ = R'₂, de formule VIIa suivante : 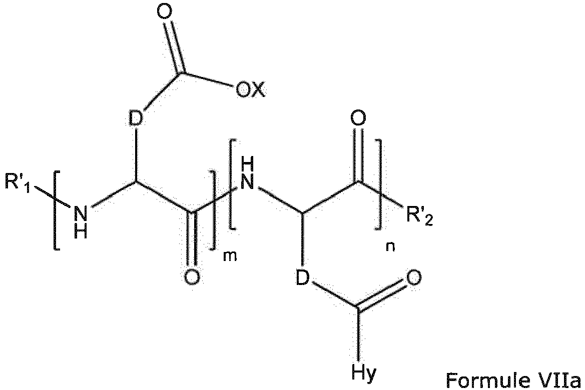 dans laquelle,
- m, n, X, D et Hy ont les définitions données précédemment,
- R'₁ est un radical choisi dans le groupe constitué par un H, un groupe acyle linéaire en C2 à C10, un groupe acyle ramifié en C4 à C10, un benzyle, une unité « acide aminé » terminale et un pyroglutamate,
- R'₂ est un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S.

Dans un mode de réalisation, la composition selon l'invention est caractérisée en ce que le co-polyaminoacide porteur de charges carboxylates et de radicaux hydrophobes est choisi parmi les co-polyaminoacides de formules VIIa, dans laquelle Hy est un radical de formule VI.

Dans un mode de réalisation, la composition selon l'invention est caractérisée en ce que le co-polyaminoacide porteur de charges carboxylates et de radicaux hydrophobes est choisi parmi les co-polyaminoacides de formule VIIa, dans laquelle Hy est un radical de formule VI, dans laquelle r=1.

Dans un mode de réalisation, la composition selon l'invention est caractérisée en ce que le co-polyaminoacide porteur de charges carboxylates et de radicaux hydrophobes est choisi parmi les co-polyaminoacides de formules VIIa, dans laquelle Hy est un radical de formule VI, dans laquelle r=1, et pour GpC, b=0.

Dans un mode de réalisation, la composition selon l'invention est caractérisée en ce que le co-polyaminoacide porteur de charges carboxylates et de radicaux hydrophobes est choisi parmi les co-polyaminoacides de formule VIIa, dans laquelle Hy est un radical de formule VI et dans laquelle GpC est un radical de formule IVd.

Dans un mode de réalisation, la composition selon l'invention est caractérisée en ce que le co-polyaminoacide porteur de charges carboxylates et de radicaux hydrophobes est choisi parmi les co-polyaminoacides de formule VIIa, dans laquelle Hy est un radical de formule VI et dans laquelle GpC est un radical de formule IVd et r=1.

On appelle « co-polyaminoacide à greffage défini » un co-polyaminoacide porteur de charges carboxylates et d'au moins un radical hydrophobe, un co-polyaminoacide de formule VIIb.

Dans un mode de réalisation, la composition selon l'invention est caractérisée en ce que le co-polyaminoacide porteur de charges carboxylates et de radicaux hydrophobes est choisi parmi les co-polyaminoacides de formule VII dans laquelle n = 0 de formule VIIb suivante : 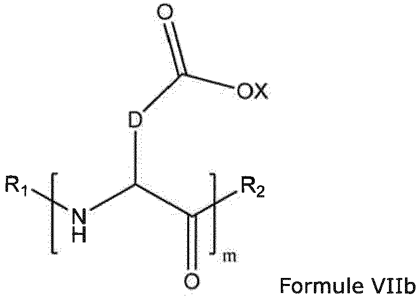 dans laquelle m, X, D, R₁ et R₂ ont les définitions données précédemment et au moins R₁ ou R₂ est un radical hydrophobe de formule I, V ou VI.

Dans un mode de réalisation, la composition selon l'invention est caractérisée en ce que le co-polyaminoacide porteur de charges carboxylates et de radicaux hydrophobes est choisi parmi les co-polyaminoacides de formule VII dans laquelle n = 0 de formule VIIb et R₁ ou R₂ est un radical hydrophobe de formule I, V ou VI.

Dans un mode de réalisation, la composition selon l'invention est caractérisée en ce que le co-polyaminoacide porteur de charges carboxylates et de radicaux hydrophobes est choisi parmi les co-polyaminoacides de formules VIIb, dans laquelle R1 = R'1, de formule VIIb' : 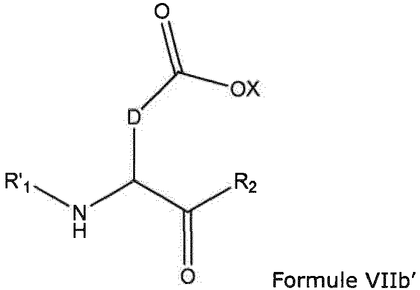 dans laquelle m, X, D, R'₁ et R₂ ont les définitions données précédemment et R₂ est un radical hydrophobe de formule I, V ou VI.

Dans un mode de réalisation, la composition selon l'invention est caractérisée en ce que le co-polyaminoacide porteur de charges carboxylates et de radicaux hydrophobes est choisi parmi les co-polyaminoacides de formules VIIb, dans laquelle R2 = R'2, de formule VIIb" : 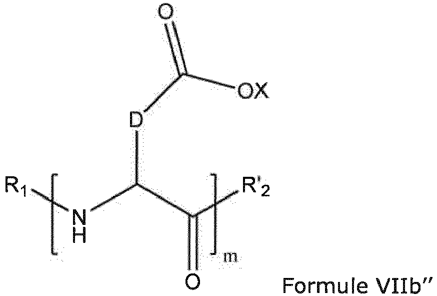 dans laquelle m, X, D, R₁ et R'₂ ont les définitions données précédemment et R₁ est un radical hydrophobe de formule I, V ou VI.

Dans un mode de réalisation, la composition selon l'invention est caractérisée en ce que le co-polyaminoacide porteur de charges carboxylates et de radicaux hydrophobes est choisi parmi les co-polyaminoacides de formule VIIb ou VIIb" dans laquelle R₁ est un radical hydrophobe de formule I, V ou VI dans lesquelles r = 0 ou r = 1 et GpR est de Formule II'.

Dans un mode de réalisation, la composition selon l'invention est caractérisée en ce que le co-polyaminoacide porteur de charges carboxylates et de radicaux hydrophobes est choisi parmi les co-polyaminoacides de formule VIIb ou VIIb" dans laquelle R₁ est un radical hydrophobe de formule VI et GpR est de formule II'.

Dans un mode de réalisation, la composition selon l'invention est caractérisée en ce que le co-polyaminoacide porteur de charges carboxylates et de radicaux hydrophobes est choisi parmi les co-polyaminoacides de formule VIIb ou VIIb" dans laquelle R₁ est un radical hydrophobe de formule VI et GpR est de formule II' et GpC est de formule IVa.

Dans un mode de réalisation, la composition selon l'invention est caractérisée en ce que le co-polyaminoacide porteur de charges carboxylates et de radicaux hydrophobes est choisi parmi les co-polyaminoacides de formule VIIb ou VIIb" dans laquelle R₁ est un radical hydrophobe de formule VI et GpR est de formule II' et GpC est de formule IVd.

Dans un mode de réalisation, la composition selon l'invention est caractérisée en ce que le co-polyaminoacide porteur de charges carboxylates et de radicaux hydrophobes est choisi parmi les co-polyaminoacides de formules VIIb ou VIIb' dans laquelle R₂ est un radical hydrophobe de formule I, V ou VI dans lesquelles r = 1 et GpR est de Formule II.

Dans un mode de réalisation, la composition selon l'invention est caractérisée en ce que le co-polyaminoacide porteur de charges carboxylates et de radicaux hydrophobes est choisi parmi les co-polyaminoacides de formules VIIb' dans laquelle R₂ est un radical hydrophobe de formule V dans laquelle r = 1 et GpR est de Formule II et a=0.

Dans un mode de réalisation, la composition selon l'invention est caractérisée en ce que le co-polyaminoacide porteur de charges carboxylates et de radicaux hydrophobes est choisi parmi les co-polyaminoacides de formules VIIb' dans laquelle R₂ est un radical hydrophobe de formule V dans laquelle r = 1, GpR est de Formule II et a=0 et GpC est de formule IVa ou IVc.

Dans un mode de réalisation, la composition selon l'invention est caractérisée en ce que le co-polyaminoacide porteur de charges carboxylates et de radicaux hydrophobes est choisi parmi les co-polyaminoacides de formules VIIb' dans laquelle R₂ est un radical hydrophobe de formule V dans laquelle r = 1, GpR est de Formule II et a=0 et GpC est de formule IVa.

Dans un mode de réalisation, la composition selon l'invention est caractérisée en ce que le co-polyaminoacide porteur de charges carboxylates et de radicaux hydrophobes est choisi parmi les co-polyaminoacides de formules VIIb' dans laquelle R₂ est un radical hydrophobe de formule V dans laquelle r = 1, GpR est de Formule II et a=0 et GpC est de formule IVc.

Dans un mode de réalisation, la composition selon l'invention est caractérisée en ce que le co-polyaminoacide porteur de charges carboxylates et de radicaux hydrophobes est choisi parmi les co-polyaminoacides de formules VIIb' dans laquelle R₂ est un radical hydrophobe de formule V dans laquelle r = 1, GpR est de Formule II et a=0 et GpC est de formule IVd ou IVf.Dans un mode de réalisation, la composition selon l'invention est caractérisée en ce que le co-polyaminoacide porteur de charges carboxylates et de radicaux hydrophobes est choisi parmi les co-polyaminoacides de formules VIIb' dans laquelle R₂ est un radical hydrophobe de formule V dans laquelle r = 1, GpR est de Formule II et a=0 et GpC est de formule IVd.

Dans un mode de réalisation, la composition selon l'invention est caractérisée en ce que le co-polyaminoacide porteur de charges carboxylates et de radicaux hydrophobes est choisi parmi les co-polyaminoacides de formules VIIb' dans laquelle R₂ est un radical hydrophobe de formule V dans laquelle r = 1, GpR est de Formule II et a=0 et GpC est de formule IVf.

Dans un mode de réalisation, la composition selon l'invention est caractérisée en ce que le co-polyaminoacide porteur de charges carboxylates et de radicaux hydrophobes est choisi parmi les co-polyaminoacides de formules VIIb' dans laquelle R₂ est un radical hydrophobe de formule VI dans laquelle r = 1 et GpR est de Formule II et a=1.

Dans un mode de réalisation, la composition selon l'invention est caractérisée en ce que le co-polyaminoacide porteur de charges carboxylates et de radicaux hydrophobes est choisi parmi les co-polyaminoacides de formules VIIb' dans laquelle R₂ est un radical hydrophobe de formule VI dans laquelle r = 1, GpR est de Formule II, a=1 et GpC est de formule IVa ou IVd.

Dans un mode de réalisation, la composition selon l'invention est caractérisée en ce que le co-polyaminoacide porteur de charges carboxylates et de radicaux hydrophobes est choisi parmi les co-polyaminoacides de formules VIIb' dans laquelle R₂ est un radical hydrophobe de formule VI dans laquelle r = 1, GpR est de Formule II, a=1 et GpC est de formule IVd.

Dans un mode de réalisation, la composition selon l'invention est caractérisée en ce que le co-polyaminoacide porteur de charges carboxylates et de radicaux hydrophobes est choisi parmi les co-polyaminoacides de formules VIIb' dans laquelle R₂ est un radical hydrophobe de formule VI dans laquelle r = 1, GpR est de Formule II , a=1 et GpC est de formule IVd, avec x=11.

Dans un mode de réalisation, la composition selon l'invention est caractérisée en ce que le co-polyaminoacide porteur de charges carboxylates et de radicaux hydrophobes est choisi parmi les co-polyaminoacides de formules VIIb' dans laquelle R₂ est un radical hydrophobe de formule VI dans laquelle r = 1, GpR est de Formule II, a=1 et GpC est de formule IVd, avec x=13.

Dans un mode de réalisation, la composition selon l'invention est caractérisée en ce que le co-polyaminoacide porteur de charges carboxylates et de radicaux hydrophobes est choisi parmi les co-polyaminoacides de formules VIIb' dans laquelle R₂ est un radical hydrophobe de formule VI dans laquelle r = 1, GpR est de Formule II , a=1 et GpC est de formule IVd avec x=15.

Dans un mode de réalisation, la composition est caractérisée en ce que le co-polyaminoacide porteur de charges carboxylates et de radicaux hydrophobes est choisi parmi les co-polyaminoacides de formule XX suivante : 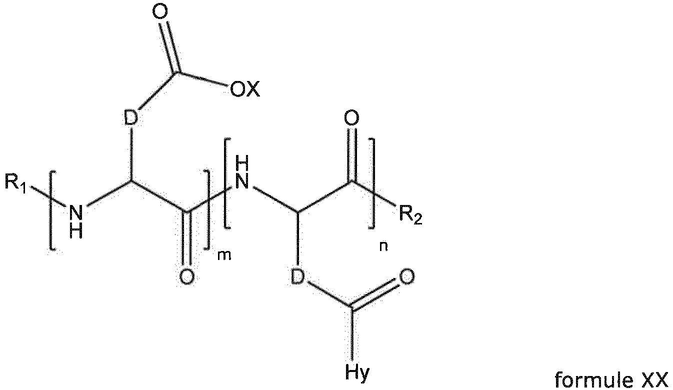 dans laquelle,
- D représente, indépendamment, soit un groupe -CH₂- (unité aspartique) soit un groupe -CH₂-CH₂- (unité glutamique),
- Hy est un radical hydrophobe choisi parmi les radicaux hydrophobes de formules I, V ou VI, dans lesquelles r = 1 et GpR est un radical de Formule II,
- R₁ est un radical hydrophobe choisi parmi les radicaux hydrophobes de formules I, V ou VI dans lesquelles r=0 ou r=1 et GpR est un radical de Formule II', ou un radical choisi dans le groupe constitué par un H, un groupe acyle linéaire en C2 à C10, un groupe acyle ramifié en C4 à C10, un benzyle, une unité « acide aminé » terminale et un pyroglutamate,
- R₂ est un radical hydrophobe choisi parmi les radicaux hydrophobes de formules I, V ou VI dans lesquelles r = 1 et GpR est un radical de Formule II, ou un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S,
- au moins un des R₁ ou R₂ est un radical hydrophobe tel que ci-dessus défini,
- X représente un H ou une entité cationique choisie dans le groupe comprenant les cations métalliques ;
- n + m représente le degré de polymérisation DP du co-polyaminoacide, c'est-à-dire le nombre moyen d'unités monomériques par chaîne de co-polyaminoacide et 5 ≤ n + m ≤ 250.

Dans un mode de réalisation, la composition selon l'invention est caractérisée en ce que R₁ est un radical choisi dans le groupe constitué par un groupe acyle linéaire en C₂ à C₁₀, un groupe acyle ramifié en C₄ à C₁₀, un benzyle, une unité « acide aminé » terminale et un pyroglutamate.

Dans un mode de réalisation, la composition selon l'invention est caractérisée en ce que R₁ est un radical choisi dans le groupe constitué par un groupe acyle linéaire en C₂ à C₁₀ ou un groupe acyle ramifié en C₄ à C₁₀.

Dans un mode de réalisation, la composition selon l'invention est caractérisée en ce que le co-polyaminoacide porteur de charges carboxylates et de radicaux hydrophobes est choisi parmi les co-polyaminoacides de formules VII, VIIa, VIIb, VIIb' ou VIIb" dans lesquels le co-polyaminoacide est choisi parmi les co-polyaminoacides dans lesquels le groupe D est un groupe -CH₂- (unité aspartique).

Dans un mode de réalisation, la composition selon l'invention est caractérisée en ce que le co-polyaminoacide porteur de charges carboxylates et de radicaux hydrophobes est choisi parmi les co-polyaminoacides de formules VII, VIIa VIIb, VIIb' ou VIIb"dans lesquels le co-polyaminoacide est choisi parmi les co-polyaminoacides dans lesquels le groupe D est un groupe -CH₂-CH₂- (unité glutamique).

On définit par le ratio radical hydrophobe par insuline basale comme étant le ratio de leurs concentrations molaires respectives : [Hy]/[insuline basale] (mol/mol) pour obtenir les performances attendues, à savoir la solubilisation de l'insuline basale à pH compris entre 6,0 et 8,0, la précipitation de l'insuline basale et la stabilité des compositions selon l'invention.

La valeur minimale du ratio radical hydrophobe par insuline basale [Hy]/[insuline basale], mesurée est la valeur à laquelle l'insuline basale est solubilisée, car la solubilisation est l'effet minimum à obtenir ; cette solubilisation conditionne tous les autres effets techniques qui ne peuvent être observés que si l'insuline basale est solubilisée à pH compris entre 6,0 et 8,0.

Dans les compositions selon l'invention, le ratio radical hydrophobe par insuline basale [Hy]/[insuline basale] peut être supérieur à la valeur minimale déterminée par la limite de solubilisation.

Dans un mode de réalisation, le ratio radical hydrophobe par insuline basale [Hy]/[insuline basale] ≤2.

Dans un mode de réalisation, le ratio radical hydrophobe par insuline basale [Hy]/[insuline basale] ≤ 1,75.

Dans un mode de réalisation, le ratio radical hydrophobe par insuline basale [Hy]/[insuline basale] ≤ 1,5.

Dans un mode de réalisation, le ratio radical hydrophobe par insuline basale [Hy]/[insuline basale] ≤ 1,25.

Dans un mode de réalisation, le ratio radical hydrophobe par insuline basale [Hy]/[insuline basale] ≤ 1,00.

Dans un mode de réalisation, le ratio radical hydrophobe par insuline basale [Hy]/[insuline basale] ≤ 0,75.

Dans un mode de réalisation, le ratio radical hydrophobe par insuline basale [Hy]/[insuline basale] ≤ 0,5.

Dans un mode de réalisation, le ratio radical hydrophobe par insuline basale [Hy]/[insuline basale] ≤ 0,25.

Dans un mode de réalisation, la composition selon l'invention est caractérisée en ce que le ratio i entre le nombre de radicaux hydrophobes et le nombre d'unités glutamiques ou aspartiques est compris entre 0,007 et 0,3.

Dans un mode de réalisation, la composition selon l'invention est caractérisée en ce que le ratio i entre le nombre de radicaux hydrophobes et le nombre d'unités glutamiques ou aspartiques est compris entre 0,01 et 0,3.

Dans un mode de réalisation, la composition selon l'invention est caractérisée en ce que le ratio i entre le nombre de radicaux hydrophobes et le nombre d'unités glutamiques ou aspartiques est compris entre 0,02 et 0,2.

Dans un mode de réalisation, la composition selon l'invention est caractérisée en ce que le radical hydrophobe répond à la formule VI et le ratio i entre le nombre de radicaux hydrophobes et le nombre d'unités glutamiques ou aspartiques est compris entre 0,007 et 0,15.

Dans un mode de réalisation, la composition selon l'invention est caractérisée en ce que le radical hydrophobe répond à la formule VI et le ratio i entre le nombre de radicaux hydrophobes et le nombre d'unités glutamiques ou aspartiques est compris entre 0,01 et 0,1.

Dans un mode de réalisation, la composition selon l'invention est caractérisée en ce que le radical hydrophobe répond à la formule VI et le ratio i entre le nombre de radicaux hydrophobes et le nombre d'unités glutamiques ou aspartiques est compris entre 0,02 et 0,08.

Dans un mode de réalisation, la composition selon l'invention est caractérisée en ce que le radical hydrophobe répond à la formule VI dans laquelle le radical Cx comprend entre 9 et 10 atomes de carbone et le ratio i entre le nombre de radicaux hydrophobes et le nombre d'unités glutamiques ou aspartiques est compris entre 0,03 et 0,15.

Dans un mode de réalisation, la composition selon l'invention est caractérisée en ce que le radical hydrophobe répond à la formule VI dans laquelle le radical Cx comprend entre 11 et 12 atomes de carbone et le ratio i entre le nombre de radicaux hydrophobes et le nombre d'unités glutamiques ou aspartiques est compris entre 0,015 et 0,1.

Dans un mode de réalisation, la composition selon l'invention est caractérisée en ce que le radical hydrophobe répond à la formule VI dans laquelle le radical Cx comprend entre 11 et 12 atomes de carbone et le ratio i entre le nombre de radicaux hydrophobes et le nombre d'unités glutamiques ou aspartiques est compris entre 0,02 et 0,08.

Dans un mode de réalisation, la composition selon l'invention est caractérisée en ce que le radical hydrophobe répond à la formule VI dans laquelle le radical Cx comprend entre 13 et 15 atomes de carbone et le ratio i entre le nombre de radicaux hydrophobes et le nombre d'unités glutamiques ou aspartiques est compris entre 0,01 et 0,1.

Dans un mode de réalisation, la composition selon l'invention est caractérisée en ce que le radical hydrophobe répond à la formule VI dans laquelle le radical Cx comprend entre 13 et 15 atomes de carbone et le ratio i entre le nombre de radicaux hydrophobes et le nombre d'unités glutamiques ou aspartiques est compris entre 0,01 et 0,06.

Dans un mode de réalisation, la composition selon l'invention est caractérisée en ce que le radical hydrophobe répond à la formule V et le ratio i entre le nombre de radicaux hydrophobes et le nombre d'unités glutamiques ou aspartiques est compris entre 0,007 et 0,3.

Dans un mode de réalisation, la composition selon l'invention est caractérisée en ce que le radical hydrophobe répond à la formule V et le ratio i entre le nombre de radicaux hydrophobes et le nombre d'unités glutamiques ou aspartiques est compris entre 0,01 et 0,3.

Dans un mode de réalisation, la composition selon l'invention est caractérisée en ce que le radical hydrophobe répond à la formule V et le ratio i entre le nombre de radicaux hydrophobes et le nombre d'unités glutamiques ou aspartiques est compris entre 0,015 et 0,2.

Dans un mode de réalisation, la composition selon l'invention est caractérisée en ce que le radical hydrophobe répond à la formule V dans laquelle le radical Cx comprend entre 11 et 14 atomes de carbone et le ratio i entre le nombre de radicaux hydrophobes et le nombre d'unités glutamiques ou aspartiques est compris entre 0,1 et 0,2.

Dans un mode de réalisation, la composition selon l'invention est caractérisée en ce que le radical hydrophobe répond à la formule V dans laquelle le radical Cx comprend entre 15 et 16 atomes de carbone et le ratio i entre le nombre de radicaux hydrophobes et le nombre d'unités glutamiques ou aspartiques est compris entre 0,04 et 0,15.

Dans un mode de réalisation, la composition selon l'invention est caractérisée en ce que le radical hydrophobe répond à la formule V dans laquelle le radical Cx comprend entre 17 et 18 atomes de carbone et le ratio i entre le nombre de radicaux hydrophobes et le nombre d'unités glutamiques ou aspartiques est compris entre 0,02 et 0,06.

Dans un mode de réalisation, la composition selon l'invention est caractérisée en ce que le radical hydrophobe répond à la formule V dans laquelle le radical Cx comprend entre 19 et 25 atomes de carbone et le ratio i entre le nombre de radicaux hydrophobes et le nombre d'unités glutamiques ou aspartiques est compris entre 0,01 et 0,06.

Dans un mode de réalisation, la composition selon l'invention est caractérisée en ce que le radical hydrophobe répond à la formule V dans laquelle le radical Cx comprend entre 19 et 25 atomes de carbone et le ratio i entre le nombre de radicaux hydrophobes et le nombre d'unités glutamiques ou aspartiques est compris entre 0,01 et 0,05.

Dans un mode de réalisation, la composition selon l'invention est caractérisée en ce que n + m est compris entre 10 et 200.

Dans un mode de réalisation, la composition selon l'invention est caractérisée en ce que n + m est compris entre 15 et 150.

Dans un mode de réalisation, la composition selon l'invention est caractérisée en ce que n + m est compris entre 15 et 100.

Dans un mode de réalisation, la composition selon l'invention est caractérisée en ce que n + m est compris entre 15 et 80.

Dans un mode de réalisation, la composition selon l'invention est caractérisée en ce que n + m est compris entre 15 et 65.

Dans un mode de réalisation, la composition selon l'invention est caractérisée en ce que n + m est compris entre 20 et 60.

Dans un mode de réalisation, la composition selon l'invention est caractérisée en ce que n + m est compris entre 20 et 50.

Dans un mode de réalisation, la composition selon l'invention est caractérisée en ce que n + m est compris entre 20 et 40.

Dans un mode de réalisation, le au moins un radical hydrophobe de formule I est choisi parmi les radicaux de formule I dans laquelle, r = 1, a = 0, p = 1, GpR répond à la formule II dans laquelle R est 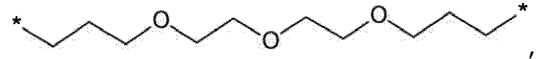 GpC répond à la formule IVd dans laquelle x = 15 et Cx est

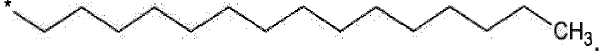

Dans un mode de réalisation, le au moins un radical hydrophobe de formule I est choisi parmi les radicaux de formule I dans laquelle, r = 1, a = 0, p = 1, GpR répond à la formule II dans laquelle R est 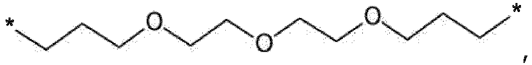 GpC répond à la formule IVd dans laquelle x = 19 et Cx est

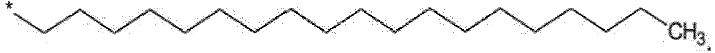

Dans un mode de réalisation, le au moins un radical hydrophobe de formule I est choisi parmi les radicaux de formule I dans laquelle, r = 1, a = 1, p = 2, GpR répond à la formule II dans laquelle R est -CH₂-CH₂-, GpA répond à la formule IIIb, GpC répond à la formule IVd dans laquelle x = 9 et Cx est

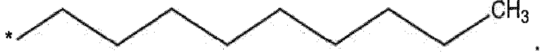

Dans un mode de réalisation, le au moins un radical hydrophobe de formule I est choisi parmi les radicaux de formule I dans laquelle, r = 1, a = 1, p = 2, GpR répond à la formule II dans laquelle R est -CH₂-CH₂-, GpA répond à la formule IIIb, GpC répond à la formule IVd dans laquelle x = 11 et Cx est

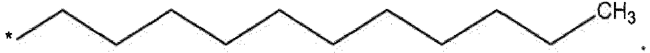

Dans un mode de réalisation, le au moins un radical hydrophobe de formule I est choisi parmi les radicaux de formule I dans laquelle, r = 1, a = 1, p = 2, GpR répond à la formule II dans laquelle R est -CH₂-CH₂-, GpA répond à la formule IIIb, GpC répond à la formule IVd dans laquelle x = 13 et Cx est

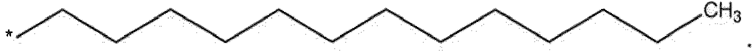

Dans un mode de réalisation, le au moins un radical hydrophobe de formule I est choisi parmi les radicaux de formule I dans laquelle, r = 1, a = 1, p = 2, GpR répond à la formule II dans laquelle R est 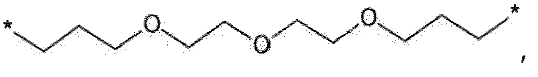 GpA répond à la formule IIIb, GpC répond à la formule IVd dans laquelle x = 13 et Cx est

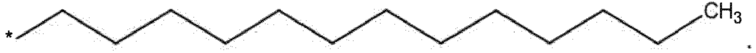

Dans un mode de réalisation, le co-polyaminoacide porteur de charges carboxylates et de radicaux hydrophobes est un co-polyaminoacide de formule VII ou VIIa, dans laquelle DP = 22 +/- 5, i = 0,05 +/- 0,02 et le au moins un radical hydrophobe de formule I est choisi parmi les radicaux de formule I dans laquelle, r = 1, a = 0, p = 1, GpR répond à la formule II dans laquelle R est 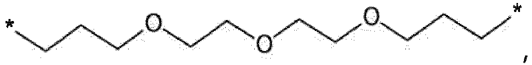 GpC répond à la formule IVd dans laquelle x = 19 et Cx est

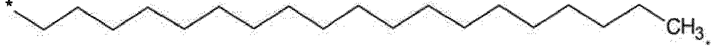

Les valeurs de degré de polymérisation DP et de ratio i sont estimées par RMN ¹H dans D₂O en comparant l'intégration des signaux provenant des groupes hydrophobes à celle des signaux provenant de la chaine principale du co-polyaminoacide.

Dans un mode de réalisation, le co-polyaminoacide porteur de charges carboxylates et de radicaux hydrophobes est un co-polyaminoacide de formule VII ou VIIa, dans laquelle DP = 25 +/- 5, i = 0,07 +/- 0,02 et le au moins un radical hydrophobe de formule I est choisi parmi les radicaux de formule I dans laquelle, r = 1, a = 0, p = 1, GpR répond à la formule II dans laquelle R est 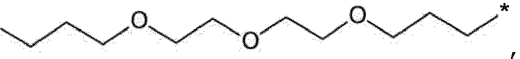 GpC répond à la formule IVd dans laquelle x = 15 et Cx est 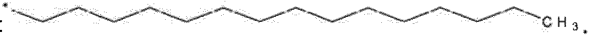 Dans un mode de réalisation, le co-polyaminoacide porteur de charges carboxylates et de radicaux hydrophobes est un co-polyaminoacide de formule VII ou VIIa, dans laquelle DP = 23 +/- 5, i = 0,05 +/- 0,02 et le au moins un radical hydrophobe de formule I est choisi parmi les radicaux de formule I dans laquelle, r = 1, a = 0, p = 1, GpR répond à la formule II dans laquelle R est 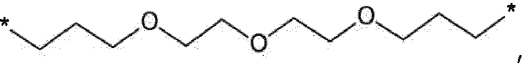 GpC répond à la formule IVd dans laquelle x = 19 et Cx est 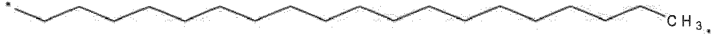 Dans un mode de réalisation, le co-polyaminoacide porteur de charges carboxylates et de radicaux hydrophobes est un co-polyaminoacide de formule VII ou VIIb, dans laquelle DP = 25 +/- 5, 0,033 ≤ i ≤ 0,05 et le radical hydrophobe de formule I est choisi parmi les radicaux de formule I dans laquelle, r = 1, a = 0, p = 1, GpR répond à la formule II dans laquelle R est 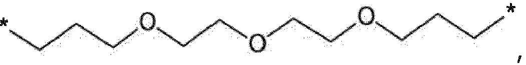 GpC répond à la formule IVd dans laquelle x = 19 et Cx est

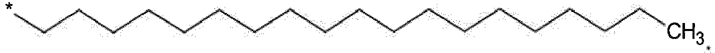

Dans un mode de réalisation, le co-polyaminoacide porteur de charges carboxylates et de radicaux hydrophobes est un co-polyaminoacide de formule VII ou VIIa, dans laquelle DP = 22 +/- 5, i = 0,05 +/- 0,02 et le au moins un radical hydrophobe de formule I est choisi parmi les radicaux de formule I dans laquelle, r = 1, a = 1, p = 2, GpR répond à la formule II dans laquelle R est -CH₂-CH₂-, GpA répond à la formule IIIb , GpC répond à la formule IVd dans laquelle x = 11 et Cx est

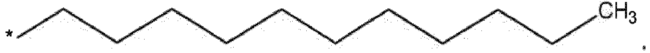

Dans un mode de réalisation, le co-polyaminoacide porteur de charges carboxylates et de radicaux hydrophobes est un co-polyaminoacide de formule VII ou VIIa, dans laquelle DP = 35 +/- 5, i = 0,05 +/- 0,02 et le au moins un radical hydrophobe de formule I est choisi parmi les radicaux de formule I dans laquelle, r = 1, a = 1, p = 2, GpR répond à la formule II et dans laquelle R est -CH₂-CH₂-, GpA répond à la formule IIIb, GpC répond à la formule IVd dans laquelle x = 11 et Cx est

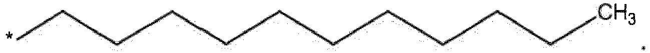

Dans un mode de réalisation, le co-polyaminoacide porteur de charges carboxylates et de radicaux hydrophobes est un co-polyaminoacide de formule VII ou VIIa, dans laquelle DP = 65 +/- 5, i = 0,05 +/- 0,02 et le au moins un radical hydrophobe de formule I est choisi parmi les radicaux de formule I dans laquelle, r = 1, a = 1, p = 2, GpR répond à la formule II dans laquelle R est -CH₂-CH₂-, GpA répond à la formule IIIb, GpC répond à la formule IVd dans laquelle x = 11 et Cx est

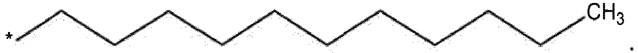

Dans un mode de réalisation, le co-polyaminoacide porteur de charges carboxylates et de radicaux hydrophobes est un co-polyaminoacide de formule VII ou VIIa, dans laquelle DP = 22 +/- 5, i = 0,04 +/- 0,02 et le au moins un radical hydrophobe de formule I est choisi parmi les radicaux de formule I dans laquelle, r = 1, a = 1, p = 2, GpR répond à la formule II dans laquelle R est -CH₂-CH₂-, GpA répond à la formule IIIb, GpC répond à la formule IVd dans laquelle x = 13 et Cx est

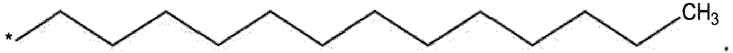

Dans un mode de réalisation, le co-polyaminoacide porteur de charges carboxylates et de radicaux hydrophobes est un co-polyaminoacide de formule VII ou VIIa, dans laquelle DP = 22 +/- 5, i = 0,03 +/- 0,01 et le au moins un radical hydrophobe de formule I est choisi parmi les radicaux de formule I dans laquelle, r = 1, a = 1, p = 2, GpR répond à la formule II dans laquelle R est 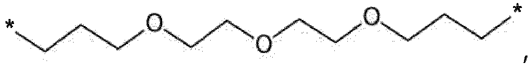 GpA répond à la formule IIIb, GpC répond à la formule IVd dans laquelle x = 13 et Cx est

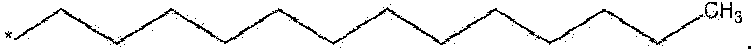

Dans un mode de réalisation, le co-polyaminoacide porteur de charges carboxylates et de radicaux hydrophobes est un co-polyaminoacide de formule VII ou VIIa, dans laquelle DP = 22 +/- 5, i = 0,07 +/- 0,02 et le au moins un radical hydrophobe de formule I est choisi parmi les radicaux de formule I dans laquelle, r = 1, a = 1, p = 2, GpR répond à la formule II dans laquelle R est 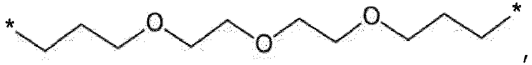 GpA répond à la formule IIIb, GpC répond à la formule IVd dans laquelle x = 9 et Cx est

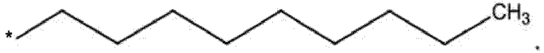

Dans un mode de réalisation, le co-polyaminoacide porteur de charges carboxylates et de radicaux hydrophobes est un co-polyaminoacide de formule VII ou VIIb, dans laquelle DP = 27 +/- 5, 0,031 ≤ i ≤ 0,045 et le au moins un radical hydrophobe de formule I est choisi parmi les radicaux de formule I dans laquelle, r = 1, a = 1, p = 2, GpR répond à la formule II dans laquelle R est -CH₂-CH₂-, GpA répond à la formule IIIb, GpC répond à la formule IVd dans laquelle x = 11 et Cx est

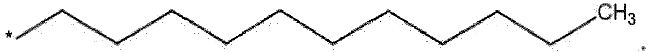

Dans un mode de réalisation, le co-polyaminoacide porteur de charges carboxylates et de radicaux hydrophobes est un co-polyaminoacide de formule VII, VIIb, VIIb' ou IIb", dans laquelle DP = 22 +/- 5, 0,037 ≤ i ≤ 0,055 et le au moins un radical hydrophobe de formule I est choisi parmi les radicaux de formule I dans laquelle, r = 1, a = 1, p = 2, GpR répond à la formule II dans laquelle R est -CH₂-CH₂-, GpA répond à la formule IIIb, GpC répond à la formule IVd dans laquelle x = 13 et Cx est

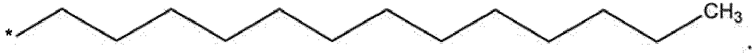

Dans un mode de réalisation, le co-polyaminoacide porteur de charges carboxylates et de radicaux hydrophobes est un co-polyaminoacide de formule VII, VIIb, VIIb' ou IIb", dans laquelle DP = 22 +/- 5, 0,037 ≤ i ≤ 0,055 et le au moins un radical hydrophobe de formule I est choisi parmi les radicaux de formule I dans laquelle, r = 1, a = 1, p = 2, GpR répond à la formule II dans laquelle R est 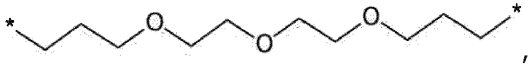 GpA répond à la formule IIIb, GpC répond à la formule IVd dans laquelle x = 13 et Cx est

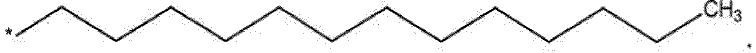

Dans un mode de réalisation, le co-polyaminoacide porteur de charges carboxylates et de radicaux hydrophobes est un co-polyaminoacide de formule VII ou VIIb, dans laquelle DP = 60 +/- 10, 0,014 ≤ i ≤ 0,02 et le au moins un radical hydrophobe de formule I est choisi parmi les radicaux de formule I dans laquelle, r = 1, a = 1, p = 2, GpR répond à la formule II dans laquelle R est -CH₂-CH₂-, GpA répond à la formule IIIb, GpC répond à la formule IVd dans laquelle x = 13 et Cx est 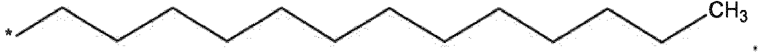 Dans un mode de réalisation, le co-polyaminoacide porteur de charges carboxylates et de radicaux hydrophobes est un co-polyaminoacide de formule VII, VIIb, VIIb' ou IIb", dans laquelle DP = 40 +/- 5, 0,022 ≤ i ≤ 0,029 et le au moins un radical hydrophobe de formule I est choisi parmi les radicaux de formule I dans laquelle, r = 1, a = 1, p = 2, GpR répond à la formule II dans laquelle R est -CH₂-CH₂-, GpA répond à la formule IIIb, GpC répond à la formule IVd dans laquelle x = 13 et Cx est

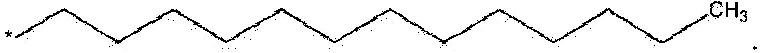

L'invention concerne également lesdits co-polyaminoacides porteurs de charges carboxylates et de radicaux hydrophobes de formule I, V ou VI.

Dans un mode de réalisation, l'invention concerne aussi les précurseurs desdits radicaux hydrophobes de formule I', V' et VI' : 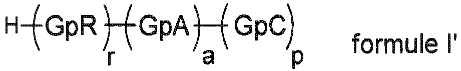 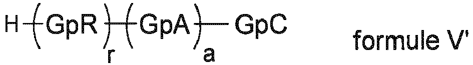 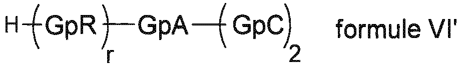 GpR, GpA, GpC, r, a, p ont les définitions données précédemment.

L'invention concerne également une méthode de préparation de compositions injectables stables.

Dans un mode de réalisation, la composition selon l'invention est caractérisée en ce que le co-polyaminoacide est issu d'un polyaminoacide obtenu par polymérisation.

Dans un mode de réalisation, la composition selon l'invention est caractérisée en ce que le co-polyaminoacide est issu d'un polyaminoacide obtenu par polymérisation par ouverture de cycle d'un dérivé de N-carboxyanhydride d'acide glutamique ou d'un dérivé de N-carboxyanhydride d'acide aspartique.

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique comme décrit dans l'article de revue Adv. Polym. Sci. 2006, 202, 1-18 (Deming, T.J.).

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique.

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique choisi dans le groupe constitué par le N-carboxyanhydride poly-glutamate de méthyle (GluOMe-NCA), le N-carboxyanhydride poly-glutamate de benzyle (GluOBzl-NCA) et le N-carboxyanhydride poly glutamate de t-butyle (GluOtBu-NCA).

Dans un mode de réalisation, le dérivé de N-carboxyanhydride d'acide glutamique est le N-carboxyanhydride poly-L-glutamate de méthyle (L-GluOMe-NCA).

Dans un mode de réalisation, le dérivé de N-carboxyanhydride d'acide glutamique est le N-carboxyanhydride poly-L-glutamate de benzyle (L-GluOBzl-NCA).

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique en utilisant comme initiateur un complexe organométallique d'un métal de transition comme décrit dans la publication Nature 1997, 390, 386-389 (Deming, T.J.).

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique en utilisant comme initiateur l'ammoniaque ou une amine primaire comme décrit dans le brevet FR 2,801,226 (Touraud, F. ; et al.) et les références citées par ce brevet.

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique en utilisant comme initiateur l'hexaméthyldisilazane comme décrit dans la publication J. Am. Chem. Soc. 2007, 129, 14114-14115 (Lu H. ; et al.) ou une amine silylée comme décrit dans la publication J. Am. Chem. Soc. 2008, 130, 12562-12563 (Lu H. ; et al.).

Dans un mode de réalisation, la composition selon l'invention est caractérisée en ce que le procédé de synthèse du polyaminoacide obtenu par polymérisation d'un dérivé de N-carboxyanhydride d'acide glutamique ou d'un dérivé de N-carboxyanhydride d'acide aspartique dont est issu le co-polyaminoacide comprend une étape d'hydrolyse de fonctions ester.

Dans un mode de réalisation, cette étape d'hydrolyse de fonctions ester peut consister en une hydrolyse en milieu acide ou une hydrolyse en milieu basique ou être effectuée par hydrogénation.

Dans un mode de réalisation, cette étape d'hydrolyse de groupements ester est une hydrolyse en milieu acide.

Dans un mode de réalisation, cette étape d'hydrolyse de groupements ester est effectuée par hydrogénation.

Dans un mode de réalisation, la composition selon l'invention est caractérisée en ce que le co-polyaminoacide est issu d'un polyaminoacide obtenu par dépolymérisation d'un polyaminoacide de plus haut poids moléculaire.

Dans un mode de réalisation, la composition selon l'invention est caractérisée en ce que le co-polyaminoacide est issu d'un polyaminoacide obtenu par dépolymérisation enzymatique d'un polyaminoacide de plus haut poids moléculaire.

Dans un mode de réalisation, la composition selon l'invention est caractérisée en ce que le co-polyaminoacide est issu d'un polyaminoacide obtenu par dépolymérisation chimique d'un polyaminoacide de plus haut poids moléculaire.

Dans un mode de réalisation, la composition selon l'invention est caractérisée en ce que le co-polyaminoacide est issu d'un polyaminoacide obtenu par dépolymérisation enzymatique et chimique d'un polyaminoacide de plus haut poids moléculaire.

Dans un mode de réalisation, la composition selon l'invention est caractérisée en ce que le co-polyaminoacide est issu d'un polyaminoacide obtenu par dépolymérisation d'un polyaminoacide de plus haut poids moléculaire choisi dans le groupe constitué par le polyglutamate de sodium et le polyaspartate de sodium.

Dans un mode de réalisation, la composition selon l'invention est caractérisée en ce que le co-polyaminoacide est issu d'un polyaminoacide obtenu par dépolymérisation d'un polyglutamate de sodium de plus haut poids moléculaire.

Dans un mode de réalisation, la composition selon l'invention est caractérisée en ce que le co-polyaminoacide est issu d'un polyaminoacide obtenu par dépolymérisation d'un polyaspartate de sodium de plus haut poids moléculaire.

Dans un mode de réalisation, la composition selon l'invention est caractérisée en ce que le co-polyaminoacide est obtenu par greffage d'un groupe hydrophobe sur un poly-L-glutamique acide ou poly-L-aspartique acide en utilisant les procédés de formation de liaison amide bien connus de l'homme de l'art.

Dans un mode de réalisation, la composition selon l'invention est caractérisée en ce que le co-polyaminoacide est obtenu par greffage d'un groupe hydrophobe sur un poly-L-glutamique acide ou poly-L-aspartique acide en utilisant les procédés de formation de liaison amide utilisés pour la synthèse peptidique.

Dans un mode de réalisation, la composition selon l'invention est caractérisée en ce que le co-polyaminoacide est obtenu par greffage d'un groupe hydrophobe sur un poly-L-glutamique acide ou poly-L-aspartique acide comme décrit dans le brevet FR 2,840,614 (Chan, Y.P. ; et al.).

Dans la suite, les unités utilisées sont pour les insulines celles recommandées par les pharmacopées dont les correspondances en mg/ml sont données dans le tableau ci-après :

| **Insuline** | **Pharmacopée EP 8.0 (2014)** | **Pharmacopée** US - USP38 (2015**)** |
|---|---|---|
| Aspart | 1U = 0,0350 mg d'insuline aspart | 1 USP = 0,0350 mg d'insuline aspart |
| Lispro | 1U = 0,0347 mq d'insuline lispro | 1 USP = 0,0347 mg d'insuline lispro |
| Humaine | 1UI = 0,0347 mg d'insuline humaine | 1 USP = 0,0347 mg d'insuline humaine |
| Glargine | 1U = 0,0364 mg d'insuline glargine | 1 USP = 0,0364 mg d'insuline glargine |
| Porcine | UI = 0,0345 mg d'insuline porcine | 1 USP = 0,0345 mg d'insuline porcine |
| Bovine | 1UI = 0,0342 mg d'insuline bovine | 1 USP = 0,0342 mg d'insuline bovine |

On entend par insuline basale dont le point isoélectrique est compris entre 5,8 et 8,5 une insuline insoluble à pH 7 et dont la durée d'action est comprise entre 8 et 24 heures ou supérieure dans les modèles standards de diabète.

Ces insulines basales dont le point isoélectrique est compris entre 5,8 et 8,5 sont des insulines recombinantes dont la structure primaire a été modifiée principalement par introduction d'aminoacides basiques comme l'Arginine ou la Lysine. Elles sont décrites par exemple dans les brevets, demandes de brevets ou publications suivants WO 2003/053339, WO 2004/096854, US 5,656,722 et US 6,100,376 dont le contenu est incorporé par référence.

Dans un mode de réalisation, l'insuline basale dont le point isoélectrique est compris entre 5,8 et 8,5 est l'insuline glargine. L'insuline glargine est commercialisée sous la marque Lantus® (100 U/ml) ou Toujeo® (300 U/ml) par SANOFI.

Dans un mode de réalisation, l'insuline basale dont le point isoélectrique est compris entre 5,8 et 8,5 est une insuline glargine biosimilaire.

Une insuline glargine biosimilaire est en voie de commercialisation sous la marque Abasaglar® ou Basaglar® par ELI LILLY.

Dans un mode de réalisation, les compositions selon l'invention comprennent entre 40 et 500 U/mL d'insuline basale dont le point isoélectrique est compris entre 5,8 et 8,5.

Dans un mode de réalisation, les compositions selon l'invention comprennent 40 U/mL d'insuline basale dont le point isoélectrique est compris entre 5,8 et 8,5.

Dans un mode de réalisation, les compositions selon l'invention comprennent 100 U/mL (soit environ 3,6 mg/mL) d'insuline basale dont le point isoélectrique est compris entre 5,8 et 8,5.

Dans un mode de réalisation, les compositions selon l'invention comprennent 150 U/mL d'insuline basale dont le point isoélectrique est compris entre 5,8 et 8,5.

Dans un mode de réalisation, les compositions selon l'invention comprennent 200 U/mL d'insuline basale dont le point isoélectrique est compris entre 5,8 et 8,5.

Dans un mode de réalisation, les compositions selon l'invention comprennent 225 U/mL d'insuline basale dont le point isoélectrique est compris entre 5,8 et 8,5.

Dans un mode de réalisation, les compositions selon l'invention comprennent 250 U/mL d'insuline basale dont le point isoélectrique est compris entre 5,8 et 8,5.

Dans un mode de réalisation, les compositions selon l'invention comprennent 300 U/mL d'insuline basale dont le point isoélectrique est compris entre 5,8 et 8,5.

Dans un mode de réalisation, les compositions selon l'invention comprennent 400 U/mL d'insuline basale dont le point isoélectrique est compris entre 5,8 et 8,5.

Dans un mode de réalisation, les compositions selon l'invention comprennent 500 U/mL d'insuline basale dont le point isoélectrique est compris entre 5,8 et 8,5.

Dans un mode de réalisation, le ratio massique entre l'insuline basale, dont le point isoélectrique est compris entre 5,8 et 8,5, et le co-polyaminoacide, soit co-polyaminoacide /insuline basale, est compris entre 0,2 et 8.

Dans un mode de réalisation, le ratio massique est compris entre 0,2 et 6.

Dans un mode de réalisation, le ratio massique est compris entre 0,2 et 5.

Dans un mode de réalisation, le ratio massique est compris entre 0,2 et 4.

Dans un mode de réalisation, le ratio massique est compris entre 0,2 et 3.

Dans un mode de réalisation, le ratio massique est compris entre 0,2 et 2.

Dans un mode de réalisation, le ratio massique est compris entre 0,2 et 1.

Dans un mode de réalisation, la concentration en co-polyaminoacide porteur de charges carboxylates et de radicaux hydrophobes est au plus de 60 mg/mL.

Dans un mode de réalisation, la concentration en co-polyaminoacide porteur de charges carboxylates et de radicaux hydrophobes est au plus de 40 mg/mL.

Dans un mode de réalisation, la concentration en co-polyaminoacide porteur de charges carboxylates et de radicaux hydrophobes est au plus de 20 mg/mL.

Dans un mode de réalisation, la concentration en co-polyaminoacide porteur de charges carboxylates et de radicaux hydrophobes est au plus de 10 mg/mL.

Dans un mode de réalisation, la concentration en co-polyaminoacide porteur de charges carboxylates et de radicaux hydrophobes est au plus 5 mg/ml.

Dans un mode de réalisation, la concentration en co-polyaminoacide porteur de charges carboxylates et de radicaux hydrophobes est au plus 2,5 mg/ml.

Dans un mode de réalisation, les compositions selon l'invention comprennent en outre une insuline prandiale. Les insulines prandiales sont solubles à pH 7.

On entend par insuline prandiale une insuline dite rapide ou « regular ».

Les insulines prandiales dites rapides sont des insulines qui doivent répondre aux besoins provoqués par l'ingestion des protéines et des glucides durant un repas, elles doivent agir en moins de 30 minutes.

Dans un mode de réalisation, l'insuline prandiale dite « regular » est de l'insuline humaine.

Dans un mode de réalisation, l'insuline prandiale est une insuline humaine recombinante telle que décrite dans la Pharmacopée Européenne et la Pharmacopée américaine.

L'insuline humaine est par exemple commercialisée sous les marques Humulin® (ELI LILLY) et Novolin® (NOVO NORDISK).

Les insulines prandiales dites très rapides (fast acting) sont des insulines qui sont obtenues par recombinaison et dont la structure primaire a été modifiée pour diminuer leur temps d'action.

Dans un mode de réalisation, les insulines prandiales dites très rapides (fast acting) sont choisies dans le groupe comprenant l'insuline lispro (Humalog®), l'insuline glulisine (Apidra®) et l'insuline aspart (NovoLog®).

Dans un mode de réalisation, l'insuline prandiale est l'insuline lispro.

Dans un mode de réalisation, l'insuline prandiale est l'insuline glulisine.

Dans un mode de réalisation, l'insuline prandiale est l'insuline aspart.

Dans un mode de réalisation, les compositions selon l'invention comprennent au total entre 60 et 800 U/mL d'insuline avec une combinaison d'insuline prandiale et d'insuline basale dont le point isoélectrique est compris entre 5,8 et 8,5.

Dans un mode de réalisation, les compositions selon l'invention comprennent au total entre 100 et 500 U/mL d'insuline avec une combinaison d'insuline prandiale et d'insuline basale dont le point isoélectrique est compris entre 5,8 et 8,5.

Dans un mode de réalisation, les compositions selon l'invention comprennent au total 800 U/mL d'insuline avec une combinaison d'insuline prandiale et d'insuline basale dont le point isoélectrique est compris entre 5,8 et 8,5.

Dans un mode de réalisation, les compositions selon l'invention comprennent au total 700 U/mL d'insuline avec une combinaison d'insuline prandiale et d'insuline basale dont le point isoélectrique est compris entre 5,8 et 8,5.

Dans un mode de réalisation, les compositions selon l'invention comprennent au total 600 U/mL d'insuline avec une combinaison d'insuline prandiale et d'insuline basale dont le point isoélectrique est compris entre 5,8 et 8,5.

Dans un mode de réalisation, les compositions selon l'invention comprennent au total 500 U/mL d'insuline avec une combinaison d'insuline prandiale et d'insuline basale dont le point isoélectrique est compris entre 5,8 et 8,5.

Dans un mode de réalisation, les compositions selon l'invention comprennent au total 400 U/mL d'insuline avec une combinaison d'insuline prandiale et d'insuline basale dont le point isoélectrique est compris entre 5,8 et 8,5.

Dans un mode de réalisation, les compositions selon l'invention comprennent au total 300 U/mL d'insuline avec une combinaison d'insuline prandiale et d'insuline basale dont le point isoélectrique est compris entre 5,8 et 8,5.

Dans un mode de réalisation, les compositions selon l'invention comprennent au total 266 U/mL d'insuline avec une combinaison d'insuline prandiale et d'insuline basale dont le point isoélectrique est compris entre 5,8 et 8,5.

Dans un mode de réalisation, les compositions selon l'invention comprennent au total 200 U/mL d'insuline avec une combinaison d'insuline prandiale et d'insuline basale dont le point isoélectrique est compris entre 5,8 et 8,5.

Dans un mode de réalisation, les compositions selon l'invention comprennent au total 100 U/mL d'insuline avec une combinaison d'insuline prandiale et d'insuline basale dont le point isoélectrique est compris entre 5,8 et 8,5.

Les proportions entre l'insuline basale dont le point isoélectrique est compris entre 5,8 et 8,5 et l'insuline prandiale sont par exemple en pourcentage de 25/75, 30/70, 40/60, 50/50, 60/40, 63/37, 70/30, 75/25, 80/20, 83/17, 90/10 pour des formulations telles que décrites ci-dessus comprenant de 60 à 800 U/mL. Cependant toute autre proportion peut être réalisée.

Dans un mode de réalisation, les compositions selon l'invention comprennent en outre une hormone gastrointestinale.

On entend par « hormones gastrointestinales », les hormones choisies dans le groupe constitué par le GLP-1 RA (Glucagon like peptide-1 receptor agonist) et le GIP (Glucose-dependent insulinotropic peptide), l'oxyntomoduline (un dérivé du proglucagon), le peptide YY, l'amyline, la cholecystokinine, le polypeptide pancréatique (PP), la ghreline et l'entérostatine, leurs analogues ou dérivés et/ou leurs sels pharmaceutiquement acceptables.

Dans un mode de réalisation, les hormones gastro-intestinales sont des analogues ou dérivés de GLP-1 RA choisis dans le groupe constitué par l'exenatide ou Byetta®(ASTRA-ZENECA), le liraglutide ou Victoza® (NOVO NORDISK), le lixisenatide ou Lyxumia® (SANOFI), l'albiglutide ou Tanzeum® (GSK) ou le dulaglutide ou Trulicity® (ELI LILLY & CO), leurs analogues ou dérivés et leurs sels pharmaceutiquement acceptables.

Dans un mode de réalisation, l'hormone gastro-intestinale est le pramlintide ou Symlin® ®(ASTRA-ZENECA).

Dans un mode de réalisation, l'hormone gastrointestinale est l'exenatide ou Byetta®, ses analogues ou dérivés et leurs sels pharmaceutiquement acceptables.

Dans un mode de réalisation, l'hormone gastrointestinale est le liraglutide ou Victoza®, ses analogues ou dérivés et leurs sels pharmaceutiquement acceptables.

Dans un mode de réalisation, l'hormone gastrointestinale est le lixisenatide ou Lyxumia®, ses analogues ou dérivés et leurs sels pharmaceutiquement acceptables.

Dans un mode de réalisation, l'hormone gastrointestinale est l'albiglutide ou Tanzeum®, ses analogues ou dérivés et leurs sels pharmaceutiquement acceptables.

Dans un mode de réalisation, l'hormone gastrointestinale est le dulaglutide ou Trulicity®, ses analogues ou dérivés et leurs sels pharmaceutiquement acceptables.

Dans un mode de réalisation, l'hormone gastrointestinale est le pramlintide ou Symlin®, ses analogues ou dérivés et leurs sels pharmaceutiquement acceptables.

On entend par « analogue », lorsqu'il est utilisé par référence à un peptide ou une protéine, un peptide ou une protéine, dans lequel un ou plusieurs résidus d'acides aminés constitutifs ont été substitués par d'autres résidus d'acides aminés et/ou dans lequel un ou plusieurs résidus d'acides aminés constitutifs ont été supprimés et/ou dans lequel un ou plusieurs résidus d'acides aminés constitutifs ont été ajoutés. Le pourcentage d'homologie admis pour la présente définition d'un analogue est de 50 %.

On entend par « dérivé », lorsqu'il est utilisé par référence à un peptide ou une protéine, un peptide ou une protéine ou un analogue chimiquement modifié par un substituant qui n'est pas présent dans le peptide ou la protéine ou l'analogue de référence, c'est-à-dire un peptide ou une protéine qui a été modifié par création de liaisons covalentes, pour introduire des substituants.

Dans un mode de réalisation, le substituant est choisi dans le groupe constitué des chaines grasses.

Dans un mode de réalisation, la concentration en hormone gastrointestinale est comprise dans un intervalle de 0,01 à 100 mg/mL.

Dans un mode de réalisation, la concentration en exenatide, ses analogues ou dérivés et leurs sels pharmaceutiquement acceptables est comprise dans un intervalle de 0,04 à 0,5 mg/mL.

Dans un mode de réalisation, la concentration en liraglutide, ses analogues ou dérivés et leurs sels pharmaceutiquement acceptables est comprise dans un intervalle de 1 à 10 mg/mL.

Dans un mode de réalisation, la concentration en lixisenatide, ses analogues ou dérivés et leurs sels pharmaceutiquement acceptables est comprise dans un intervalle de 0,01 à 1 mg/mL.

Dans un mode de réalisation, la concentration en albiglutide, ses analogues ou dérivés et leurs sels pharmaceutiquement acceptables est comprise entre 5 à 100 mg/mL.

Dans un mode de réalisation, la concentration en dulaglutide, ses analogues ou dérivés et leurs sels pharmaceutiquement acceptables est comprise entre 0,1 à 10 mg/mL.

Dans un mode de réalisation, la concentration en pramlintide, ses analogues ou dérivés et leurs sels pharmaceutiquement acceptables est comprise entre 0,1 à 5 mg/mL.

Dans un mode de réalisation, les compositions selon l'invention sont réalisées par mélange de solutions commerciales d'insuline basale dont le point isoélectrique est compris entre 5,8 et 8,5 et de solutions commerciales de GLP-1 RA, d'analogue ou de dérivé de GLP-1 RA en ratios volumiques compris dans un intervalle de 10/90 à 90/10.

Dans un mode de réalisation, la composition selon l'invention comprend une dose journalière d'insuline basale et une dose journalière d'hormone gastro-intestinale.

Dans un mode de réalisation, les compositions selon l'invention comprennent entre 40 U/mL et 500 U/mL d'insuline basale dont le point isoélectrique est compris entre 5,8 et 8,5 et, entre 0,05 et 0,5 mg/mL d'exenatide.

Dans un mode de réalisation, les compositions selon l'invention comprennent entre 40 U/mL et 500 U/mL d'insuline basale dont le point isoélectrique est compris entre 5,8 et 8,5 et, de 1 à 10 mg/mL de liraglutide.

Dans un mode de réalisation, les compositions selon l'invention comprennent entre 40 U/mL et 500 U/mL d'insuline basale dont le point isoélectrique est compris entre 5,8 et 8,5 et, de 0,01 à 1 mg/mL de lixisenatide.

Dans un mode de réalisation, les compositions selon l'invention comprennent entre 40 U/mL et 500 U/mL d'insuline basale dont le point isoélectrique est compris entre 5,8 et 8,5 et, de 5 à 100 mg/mL d'albiglutide.

Dans un mode de réalisation, les compositions selon l'invention comprennent entre 40 U/mL et 500 U/mL d'insuline basale dont le point isoélectrique est compris entre 5,8 et 8,5 et, de 0,1 à 10 mg/mL de dulaglutide.

Dans un mode de réalisation, les compositions selon l'invention comprennent 500 U/mL d'insuline basale dont le point isoélectrique est compris entre 5,8 et 8,5 et, de 0,04 à 0,5 mg/mL d'exenatide.

Dans un mode de réalisation, les compositions selon l'invention comprennent 500 U/mL d'insuline basale dont le point isoélectrique est compris entre 5,8 et 8,5 et, de 1 à 10 mg/mL de liraglutide.

Dans un mode de réalisation, les compositions selon l'invention comprennent 500 U/mL d'insuline basale dont le point isoélectrique est compris entre 5,8 et 8,5 et, de 0,01 à 1 mg/mL de lixisenatide.

Dans un mode de réalisation, les compositions selon l'invention comprennent 500 U/mL d'insuline basale dont le point isoélectrique est compris entre 5,8 et 8,5 et, de 5 à 100 mg/mL d'albiglutide.

Dans un mode de réalisation, les compositions selon l'invention comprennent 500 U/mL d'insuline basale dont le point isoélectrique est compris entre 5,8 et 8,5 et, de 0,1 à 10 mg/mL de dulaglutide.

Dans un mode de réalisation, les compositions selon l'invention comprennent 400 U/mL d'insuline basale dont le point isoélectrique est compris entre 5,8 et 8,5 et, de 0,04 à 0,5 mg/mL d'exenatide.

Dans un mode de réalisation, les compositions selon l'invention comprennent 400 U/mL d'insuline basale dont le point isoélectrique est compris entre 5,8 et 8,5 et, de 1 à 10 mg/mL de liraglutide.

Dans un mode de réalisation, les compositions selon l'invention comprennent 400 U/mL d'insuline basale dont le point isoélectrique est compris entre 5,8 et 8,5 et, de 0,01 à 1 mg/mL de lixisenatide.

Dans un mode de réalisation, les compositions selon l'invention comprennent 400 U/mL d'insuline basale dont le point isoélectrique est compris entre 5,8 et 8,5 et, de 5 à 100 mg/mL d'albiglutide.

Dans un mode de réalisation, les compositions selon l'invention comprennent 400 U/mL d'insuline basale dont le point isoélectrique est compris entre 5,8 et 8,5 et, de 0,1 à 10 mg/mL de dulaglutide.

Dans un mode de réalisation, les compositions selon l'invention comprennent 300 U/mL d'insuline basale dont le point isoélectrique est compris entre 5,8 et 8,5 et, de 0,04 à 0,5 mg/mL d'exenatide.

Dans un mode de réalisation, les compositions selon l'invention comprennent 300 U/mL d'insuline basale dont le point isoélectrique est compris entre 5,8 et 8,5 et, de 1 à 10 mg/mL de liraglutide.

Dans un mode de réalisation, les compositions selon l'invention comprennent 300 U/mL d'insuline basale dont le point isoélectrique est compris entre 5,8 et 8,5 et, de 0,01 à 1 mg/mL de lixisenatide.

Dans un mode de réalisation, les compositions selon l'invention comprennent 300 U/mL d'insuline basale dont le point isoélectrique est compris entre 5,8 et 8,5 et, de 5 à 100 mg/mL d'albiglutide.

Dans un mode de réalisation, les compositions selon l'invention comprennent 300 U/mL d'insuline basale dont le point isoélectrique est compris entre 5,8 et 8,5 et, de 0,1 à 10 mg/mL de dulaglutide.

Dans un mode de réalisation, les compositions selon l'invention comprennent 225 U/mL d'insuline basale dont le point isoélectrique est compris entre 5,8 et 8,5 et, de 0,04 à 0,5 mg/mL d'exenatide.

Dans un mode de réalisation, les compositions selon l'invention comprennent 225 U/mL d'insuline basale dont le point isoélectrique est compris entre 5,8 et 8,5 et, de 1 à 10 mg/mL de liraglutide.

Dans un mode de réalisation, les compositions selon l'invention comprennent 225 U/mL d'insuline basale dont le point isoélectrique est compris entre 5,8 et 8,5 et, de 0,01 à 1 mg/mL de lixisenatide.

Dans un mode de réalisation, les compositions selon l'invention comprennent 225 U/mL d'insuline basale dont le point isoélectrique est compris entre 5,8 et 8,5 et, de 5 à 100 mg/mL d'albiglutide.

Dans un mode de réalisation, les compositions selon l'invention comprennent 225 U/mL d'insuline basale dont le point isoélectrique est compris entre 5,8 et 8,5 et, de 0,1 à 10mg/mL de dulaglutide.

Dans un mode de réalisation, les compositions selon l'invention comprennent 200 U/mL d'insuline basale dont le point isoélectrique est compris entre 5,8 et 8,5 et, de 0,04 à 0,5 mg/mL d'exenatide.

Dans un mode de réalisation, les compositions selon l'invention comprennent 200 U/mL d'insuline basale dont le point isoélectrique est compris entre 5,8 et 8,5 et, de 1 à 10 mg/mL de liraglutide.

Dans un mode de réalisation, les compositions selon l'invention comprennent 200 U/mL d'insuline basale dont le point isoélectrique est compris entre 5,8 et 8,5 et, de 0,01 à 1 mg/mL de lixisenatide.

Dans un mode de réalisation, les compositions selon l'invention comprennent 200 U/mL d'insuline basale dont le point isoélectrique est compris entre 5,8 et 8,5 et, de 5 à 100 mg/mL d'albiglutide.

Dans un mode de réalisation, les compositions selon l'invention comprennent 200 U/mL d'insuline basale dont le point isoélectrique est compris entre 5,8 et 8,5 et, de 0,1 à 10 mg/mL de dulaglutide.

Dans un mode de réalisation, les compositions selon l'invention comprennent 100 U/mL (soit environ 3,6 mg/mL) d'insuline basale dont le point isoélectrique est compris entre 5,8 et 8,5 et, de 0,04 à 0,5 mg/mL d'exenatide.

Dans un mode de réalisation, les compositions selon l'invention comprennent 100 U/mL (soit environ 3,6 mg/mL) d'insuline basale dont le point isoélectrique est compris entre 5,8 et 8,5 et, de 1 à 10 mg/mL de liraglutide.

Dans un mode de réalisation, les compositions selon l'invention comprennent 100 U/mL (soit environ 3,6 mg/mL) d'insuline basale dont le point isoélectrique est compris entre 5,8 et 8,5 et, de 0,01 à 1 mg/mL de lixisenatide.

Dans un mode de réalisation, les compositions selon l'invention comprennent 100 U/mL d'insuline basale dont le point isoélectrique est compris entre 5,8 et 8,5 et, de 5 à 100 mg/mL d'albiglutide.

Dans un mode de réalisation, les compositions selon l'invention comprennent 100 U/mL d'insuline basale dont le point isoélectrique est compris entre 5,8 et 8,5 et, de 0,1 à 10 mg/mL de dulaglutide.

Dans un mode de réalisation, les compositions selon l'invention comprennent 40 U/mL d'insuline basale dont le point isoélectrique est compris entre 5,8 et 8,5 et, de 0,04 à 0,5 mg/mL d'exenatide.

Dans un mode de réalisation, les compositions selon l'invention comprennent 40 U/mL d'insuline basale dont le point isoélectrique est compris entre 5,8 et 8,5 et, de 1 à 10 mg/mL de liraglutide.

Dans un mode de réalisation, les compositions selon l'invention comprennent 40 U/mL d'insuline basale dont le point isoélectrique est compris entre 5,8 et 8,5 et, de 0,01 à 1 mg/mL de lixisenatide.

Dans un mode de réalisation, les compositions selon l'invention comprennent 40 U/mL d'insuline basale dont le point isoélectrique est compris entre 5,8 et 8,5 et, de 5 à 100 mg/mL d'albiglutide.

Dans un mode de réalisation, les compositions selon l'invention comprennent 40 U/mL d'insuline basale dont le point isoélectrique est compris entre 5,8 et 8,5 et, de 0,1 à 10 mg/mL de dulaglutide.

Dans un mode de réalisation, les compositions selon l'invention comprennent en outre des sels de zinc à une concentration comprise entre 0 et 5000 µM.

Dans un mode de réalisation, les compositions selon l'invention comprennent en outre des sels de zinc à une concentration comprise entre 0 et 4000 µM.

Dans un mode de réalisation, les compositions selon l'invention comprennent en outre des sels de zinc à une concentration comprise entre 0 et 3000 µM.

Dans un mode de réalisation, les compositions selon l'invention comprennent en outre des sels de zinc à une concentration comprise entre 0 et 2000 µM.

Dans un mode de réalisation, les compositions selon l'invention comprennent en outre des sels de zinc à une concentration comprise entre 0 et 1000 µM.

Dans un mode de réalisation, les compositions selon l'invention comprennent en outre des sels de zinc à une concentration comprise entre 50 et 600 µM.

Dans un mode de réalisation, les compositions selon l'invention comprennent en outre des sels de zinc à une concentration comprise entre 100 et 500 µM.

Dans un mode de réalisation, les compositions selon l'invention comprennent en outre des sels de zinc à une concentration comprise entre 200 et 500 µM.

Dans un mode de réalisation, les compositions selon l'invention comprennent en outre des tampons.

Dans un mode de réalisation, les compositions selon l'invention comprennent des tampons à des concentrations comprises entre 0 et 100 mM.

Dans un mode de réalisation, les compositions selon l'invention comprennent des tampons à des concentrations comprises entre 15 et 50 mM.

Dans un mode de réalisation, les compositions selon l'invention comprennent un tampon choisi dans le groupe constitué par un tampon phosphate, le Tris (trishydroxyméthylaminométhane) et le citrate de sodium.

Dans un mode de réalisation, le tampon est le phosphate de sodium.

Dans un mode de réalisation, le tampon est le Tris (trishydroxyméthylaminométhane).

Dans un mode de réalisation, le tampon est le citrate de sodium.

Dans un mode de réalisation, les compositions selon l'invention comprennent en outre des conservateurs.

Dans un mode de réalisation, les conservateurs sont choisis dans le groupe constitué par le m-crésol et le phénol, seuls ou en mélange.

Dans un mode de réalisation, la concentration des conservateurs est comprise entre 10 et 50 mM.

Dans un mode de réalisation, la concentration des conservateurs est comprise entre 10 et 40 mM.

Dans un mode de réalisation, les compositions selon l'invention comprennent en outre un tensioactif.

Dans un mode de réalisation, le tensioactif est choisi dans le groupe constitué par le propylène glycol et le polysorbate.

Les compositions selon l'invention peuvent en outre comprendre des additifs tels que des agents de tonicité.

Dans un mode de réalisation, les agents de tonicité sont choisis dans le groupe constitué par la glycérine, le chlorure de sodium, le mannitol et la glycine.

Les compositions selon l'invention peuvent comprendre en outre tous les excipients conformes aux pharmacopées et compatibles avec les insulines utilisées aux concentrations d'usage.

L'invention concerne également une formulation pharmaceutique selon l'invention, caractérisée en ce qu'elle est obtenue par séchage et/ou lyophilisation.

Dans le cas des libérations locale et systémique, les modes d'administration envisagés sont par voie intraveineuse, sous-cutanée, intradermique ou intramusculaire.

Les voies d'administration transdermique, orale, nasale, vaginale, oculaire, buccale, pulmonaire sont également envisagées.

L'invention concerne également des formulations unidoses à pH compris entre 6,0 et 8,0 comprenant une insuline basale dont le point isoélectrique est compris entre 5,8 et 8,5.

L'invention concerne également des formulations unidoses à pH compris entre 6,0 et 8,0 comprenant une insuline basale dont le point isoélectrique est compris entre 5,8 et 8,5 et une insuline prandiale.

L'invention concerne également des formulations unidoses à pH compris entre 6,0 et 8,0 comprenant une insuline basale dont le point isoélectrique est compris entre 5,8 et 8,5 et une hormone gastrointestinale, telle que définie précédemment.

L'invention concerne également des formulations unidoses à pH compris entre 6,0 et 8,0 comprenant une insuline basale dont le point isoélectrique est compris entre 5,8 et 8,5, une insuline prandiale et une hormone gastrointestinale, telle que définie précédemment.

L'invention concerne également des formulations unidoses à pH compris entre 6,6 et 7,8 comprenant une insuline basale dont le point isoélectrique est compris entre 5,8 et 8,5.

L'invention concerne également des formulations unidoses à pH compris entre 6,6 et 7,8 comprenant une insuline basale dont le point isoélectrique est compris entre 5,8 et 8,5 et une insuline prandiale.

L'invention concerne également des formulations unidoses à pH compris entre 6,6 et 7,8 comprenant une insuline basale dont le point isoélectrique est compris entre 5,8 et 8,5 et une hormone gastrointestinale, telle que définie précédemment.

L'invention concerne également des formulations unidoses à pH compris entre 6,6 et 7,8 comprenant une insuline basale dont le point isoélectrique est compris entre 5,8 et 8,5, une insuline prandiale et une hormone gastrointestinale, telle que définie précédemment.

L'invention concerne également des formulations unidoses à pH compris entre 6,6 et 7,6 comprenant une insuline basale dont le point isoélectrique est compris entre 5,8 et 8,5.

L'invention concerne également des formulations unidoses à pH compris entre 6,6 et 7,6 comprenant une insuline basale dont le point isoélectrique est compris entre 5,8 et 8,5 et une insuline prandiale.

L'invention concerne également des formulations unidoses à pH compris entre 6,6 et 7,6 comprenant une insuline basale dont le point isoélectrique est compris entre 5,8 et 8,5 et une hormone gastrointestinale, telle que définie précédemment.

L'invention concerne également des formulations unidoses à pH compris entre 6,6 et 7,6 comprenant une insuline basale dont le point isoélectrique est compris entre 5,8 et 8,5 , une insuline prandiale et une hormone gastrointestinale, telle que définie précédemment.

Dans un mode de réalisation, les formulations unidoses comprennent en outre un co-polyaminoacide tel que défini précédemment.

Dans un mode de réalisation, les formulations sont sous forme d'une solution injectable.

Dans un mode de réalisation, l'insuline basale dont le point isoélectrique est compris entre 5,8 et 8,5 est l'insuline glargine.

Dans un mode de réalisation, l'insuline prandiale est l'insuline humaine.

Dans un mode de réalisation, l'insuline est une insuline humaine recombinante telle que décrite dans la Pharmacopée Européenne et la Pharmacopée américaine.

Dans un mode de réalisation, l'insuline prandiale est choisie dans le groupe comprenant l'insuline lispro (Humalog®), l'insuline glulisine (Apidra®) et l'insuline aspart (NovoLog®).

Dans un mode de réalisation, l'insuline prandiale est l'insuline lispro.

Dans un mode de réalisation, l'insuline prandiale est l'insuline glulisine.

Dans un mode de réalisation, l'insuline prandiale est l'insuline aspart.

Dans un mode de réalisation, le GLP-1 RA, analogue ou dérivé de GLP-1 RA est choisi dans le groupe comprenant exenatide (Byetta®), liraglutide (Victoza®), lixisenatide (Lyxumia®), albiglutide (Tanzeum®), dulaglutide (Trulicity®) ou l'un de leurs dérivés.

Dans un mode de réalisation, l'hormone gastrointestinale est l'exenatide.

Dans un mode de réalisation, l'hormone gastrointestinale est le liraglutide.

Dans un mode de réalisation, l'hormone gastrointestinale est le lixisenatide.

Dans un mode de réalisation, l'hormone gastrointestinale est l'albiglutide.

Dans un mode de réalisation, l'hormone gastrointestinale est le dulaglutide.

La solubilisation à pH compris entre 6,0 et 8,0 des insulines basales dont le point isoélectrique est compris entre 5,8 et 8,5, par les co-polyaminoacides porteurs de charges carboxylates et d'au moins un radical hydrophobe selon l'invention, peut être constatée et contrôlée de manière simple, à l'œil nu, grâce à un changement d'aspect de la solution.

La solubilisation à pH compris entre 6,6 et 7,8 des insulines basales dont le point isoélectrique est compris entre 5,8 et 8,5, par les co-polyaminoacides porteurs de charges carboxylates et d'au moins un radical hydrophobe selon l'invention, peut être constatée et contrôlée de manière simple, à l'oeil nu, grâce à un changement d'aspect de la solution.

Par ailleurs et de façon toute aussi importante, la demanderesse a pu vérifier qu'une insuline basale dont le point isoélectrique est compris entre 5,8 et 8,5, solubilisée à pH compris entre 6,0 et 8,0 en présence d'un co-polyaminoacide porteur de charges carboxylates et d'au moins un radical hydrophobe selon l'invention conserve son action d'insuline lente que ce soit seule ou en combinaison avec une insuline prandiale ou une hormone gastrointestinale.

La demanderesse a également pu vérifier qu'une insuline prandiale mélangée à pH compris entre 6,0 et 8,0 en présence d'un co-polyaminoacide porteur de charges carboxylates et d'au moins un radical hydrophobe selon l'invention et d'une insuline basale dont le point isoélectrique est compris entre 5,8 et 8,5, conserve son action d'insuline rapide.

La préparation d'une composition selon l'invention présente l'avantage de pouvoir être réalisée par simple mélange d'une solution aqueuse d'insuline basale dont le point isoélectrique est compris entre 5,8 et 8,5, et d'un co-polyaminoacide porteur de charges carboxylates et d'au moins un radical hydrophobe selon l'invention, en solution aqueuse ou sous forme lyophilisée. Si nécessaire, le pH de la préparation est ajusté à pH compris entre 6 et 8.

La préparation d'une composition selon l'invention présente l'avantage de pouvoir être réalisée par simple mélange d'une solution aqueuse d'insuline basale dont le point isoélectrique est compris entre 5,8 et 8,5, d'une solution d'insuline prandiale, et d'un co-polyaminoacide porteur de charges carboxylates et d'au moins un radical hydrophobe selon l'invention, en solution aqueuse ou sous forme lyophilisée. Si nécessaire, le pH de la préparation est ajusté à pH compris entre 6 et 8.

La préparation d'une composition selon l'invention présente l'avantage de pouvoir être réalisée par simple mélange d'une solution aqueuse d'insuline basale dont le point isoélectrique est compris entre 5,8 et 8,5, d'une solution de GLP-1 RA, un analogue ou un dérivé de GLP-1 RA, et d'un co-polyaminoacide porteur de charges carboxylates et d'au moins un radical hydrophobe selon l'invention, en solution aqueuse ou sous forme lyophilisée. Si nécessaire, le pH de la préparation est ajusté à pH compris entre 6 et 8.

La préparation d'une composition selon l'invention présente l'avantage de pouvoir être réalisée par simple mélange d'une solution aqueuse d'insuline basale dont le point isoélectrique est compris entre 5,8 et 8,5, d'une solution d'insuline prandiale, d'une solution de GLP-1 RA ou d'un analogue ou dérivé de GLP-1 RA et d'un co-polyaminoacide porteur de charges carboxylates et d'au moins un radical hydrophobe selon l'invention, en solution aqueuse ou sous forme lyophilisée. Si nécessaire, le pH de la préparation est ajusté à pH compris entre 6 et 8.

Dans un mode de réalisation, le mélange d'insuline basale et de co-polyaminoacide est concentré par ultrafiltration avant le mélange avec l'insuline prandiale en solution aqueuse ou sous forme lyophilisée.

Si nécessaire, la composition du mélange est ajustée en excipients tels que glycérine, m-crésol, chlorure de zinc, et polysorbate (Tween®) par ajout de solutions concentrées de ces excipients au sein du mélange. Si nécessaire, le pH de la préparation est ajusté à pH compris entre 6 et 8.

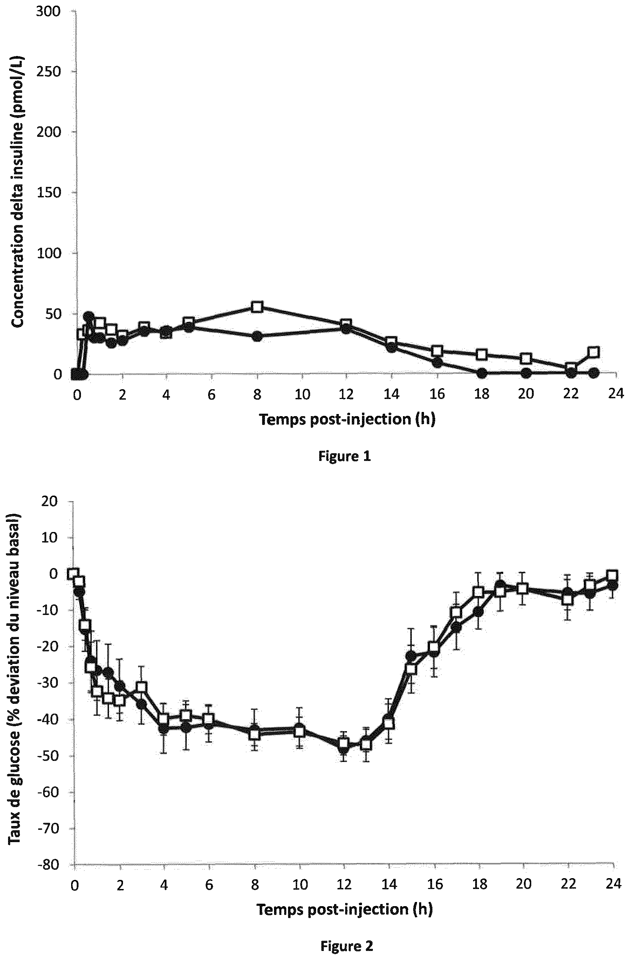

Figure 1 : Courbes médianes (en abscisse le temps post-injection exprimé en heures et en ordonnée la concentration delta en insuline exprimé en pmol/L) de déviation du niveau basal d'insuline après l'administration de la composition DB3k de l'exemple DB6 (200 U/ml, 0,5 U/kg d'insuline) (carrés vides) en comparaison après l'administration de la composition C4 (Lantus®) (100 U/mL, 0,50 U/kg) (cercles pleins); les administrations ayant été réalisées sur le chien (n=10), par injection sous-cutanée.
Figure 2 : Courbes moyennes (en abscisse le temps post-injection exprimé en heures et en ordonnée le taux de glucose exprimé en pourcentage de déviation du niveau basal)) de glycémie en pourcents de déviation du niveau basal ± erreur standard de la moyenne après l'administration de la composition DB3k de l'exemple DB6 (200 U/ml, 0,5 U/kg d'insuline) (carrés vides) en comparaison après l'administration de la composition C4 (Lantus®) (100 U/mL, 0,50 U/kg) (cercles pleins); les administrations ayant été réalisées sur le chien (n=10), par injection sous-cutanée.

### Exemples

L'invention est décrite plus en détails au travers des exemples suivants de manière non-limitative.

### Partie A

### AA : Synthèse des molécules hydrophobes dans lesquelles p = 1

Les radicaux sont représentés dans le tableau la suivant par la molécule hydrophobe correspondante avant greffage sur le co-polyaminoacide.

**Tableau la : liste et structure de molécules hydrophobes synthétisées selon l'invention.**

| **N°** | **Structure de la molécule hydrophobe avant greffage sur le co-polyaminoacide** |
|---|---|
| AA4 | 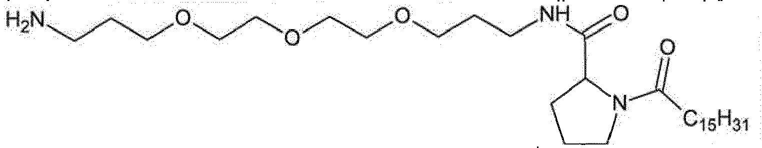 |
| AA7 | 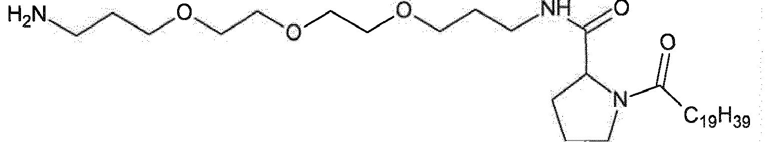 |
| AA10 | 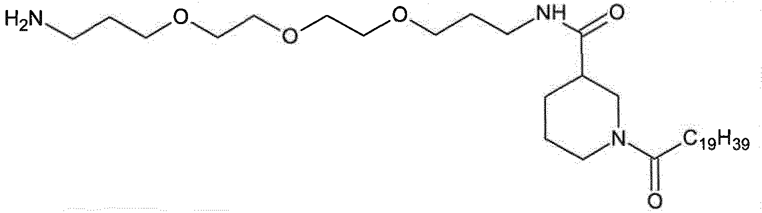 |

### Exemple AA4 : molécule AA4

### Molécule A1: produit obtenu par la réaction entre le chlorure de palmitoyle et la L-proline.

A une solution de L-proline (10,6 g, 92,1 mmol) dans une solution aqueuse de soude 1 N (230 mL ; 230 mmol) est ajoutée goutte à goutte pendant 90 minutes une solution de chlorure de palmitoyle (23,0 g, 83,7 mmol) dans l'acétone (167 mL). Après 14 h d'agitation à température ambiante, le mélange hétérogène est refroidi à 0 °C, puis filtré sur fritté pour donner un solide blanc qui est lavé avec de l'eau (2 x 100 mL) puis du diisopropyléther (100 mL). Le solide est séché sous pression réduite. Le solide est alors dissous à reflux dans 200 mL d'eau puis 8 mL d'une solution d'acide chlorhydrique à 37% sont ajoutés pour obtenir un pH de 1. Le milieu réactionnel opalescent est alors refroidi à 0 °C. Le précipité obtenu est filtré sur fritté puis lavé avec de l'eau (5 x 50 mL) jusqu'à obtenir des filtrats de pH neutre puis est ensuite séché à 50 °C dans une étuve sous vide pendant une nuit. Le produit est purifié par recristallisation dans le diisopropyléther. Un solide blanc est obtenu.

Rendement : 22,7 g (77%).

RMN ¹H (CDCl₃, ppm) : 0,88 (3H) ; 1,19-1,45 (24H) ; 1,58-1,74 (2H) ; 1,88-2,14 (3H) ; 2,15-2,54 (3H) ; 3,47 (1H) ; 3,58 (1H) ; 4,41 (0,1H) ; 4,61 (0,9H) 6,60-8,60 (1H).

Molécule A2: produit obtenu par la réaction entre la molécule A1 et la Boc-1-amino-4,7,10-trioxa-13-tridécane amine. A une solution de molécule A1 (4,5 g, 12,7 mmol) dans 90 mL de chloroforme sont ajoutés successivement à température ambiante de la N,N-diisopropyléthylamine (DIPEA) (4,1 g, 31,8 mmol), du 1-hydroxybenzotriazole (HOBt) (2,2 g, 16,4 mmol) puis du N-(3-dimethylaminopropyl)-N'-ethylcarbodiimide (EDC) (3,2 g, 16,6 mmol). Après 15 minutes d'agitation à température ambiante, une solution de Boc-1-amino-4,7,10-trioxa-13-tridécane amine (4,5 g, 14,0 mmol) dans 5 mL de chloroforme est additionnée. Après 18 h d'agitation à température ambiante, une solution de HCl 0,1 N (100 mL), puis une solution saturée de NaCl (50 mL) sont ajoutées. Les phases sont séparées puis la phase organique est lavée successivement avec une solution de HCl 0,1 N / NaCl saturée (100 mL/50 mL), une solution de NaCl saturée (100 mL), une solution de NaHCO₃ saturée (100 mL), puis une solution de NaCl saturée (100 mL). La phase organique est séchée sur sulfate de sodium anhydre, filtrée puis concentrée sous pression réduite. Une huile jaune est obtenue après purification par chromatographie flash (méthanol, dichlorométhane).

Rendement : 7,7 g (92%).

RMN ¹H (CDCl₃, ppm) : 0,88 (3H) ; 1,22-1,37 (24H) ; 1,44 (9H) ; 1,59-1,67 (2H) ; 1,67-2,00 (6H) ; 2,06-2,45 (4H) ; 3,18-3,76 (18H) ; 4,28 (0,2H) ; 4,52 (0,8H) ; 4,69-5,04 (1H) ; 6,77 (0,2H) ; 7,20 (0,8H).

### Molécule AA4

A une solution de molécule A2 (7,7 g, 11,8 mmol) dans 90 mL de dichlorométhane est ajoutée goutte à goutte et à 0 °C une solution d'acide chlorhydrique 4 M dans le dioxane (29,5 mL, 118 mmol). Après 3h30 d'agitation à température ambiante, la solution est concentrée sous pression réduite. Le résidu est purifié par chromatographie flash (méthanol, dichlorométhane) pour donner une huile jaune. Une coévaporation avec du diisopropyléther et un séchage à 50 °C sous vide permet d'obtenir la molécule AA4 sous forme de sel de chlorhydrate.

Rendement : 5,4 g (76%).

RMN ¹H (CDCl₃, ppm) : 0,88 (3H) ; 1,08-1,40 (24H) ; 1,49-1,65 (2H) ; 1,76-2,39 (10H) ; 3,07-3,28 (3H) ; 3,34-3,80 (15H) ; 4,34 (0,05H) ; 4,64 (0,95H) ; 7,35 (0,05H) ; 7,66-8,58 (3,95H).

LC/MS (ESI) : 556,7 ; (calculé ([M+H]⁺) : 556,5).

### Exemple AA7 : molécule AA7

### Molécule A3 : produit obtenu par réaction entre l'acide arachidique et la L-proline.

A une solution d'acide arachidique (15,51 g, 49,63 mmol) dans le THF (500 mL) à 0°C sont ajoutés successivement du dicyclohexyle carbodiimide (DCC) (10,45 g, 50,6mmol) et du N-hydroxysuccinimide (NHS) (5,83 g, 50,6 mmol). Après 17 h d'agitation à température ambiante, le milieu est refroidi à 0°C pendant 20 min, filtré sur fritté. De la L-proline (6 g, 52,11 mmol), de la DIPEA (60,5 mL) et de l'eau (50 mL) sont ajoutées au filtrat. Après 48 h d'agitation à température ambiante, le milieu est traité avec une solution aqueuse d'HCl 1N jusqu'à pH 1 et le solide résultant est filtré sur fritté, lavé à l'eau jusqu'à pH neutre des eaux-mères puis séché sous vide pour donner un solide jaunâtre. Après purification par chromatographie sur gel de silice (cyclohexane, acétate d'éthyle), un solide blanc est obtenu.

Rendement : 10,96 g (54%).

RMN ¹H (CDCl₃, ppm) : 0,88 (3H) ; 1,28 (34H) ; 1,66 (2H) ; 1,95-2,15 (2H) ; 2,34 (2H) ; 2,45 (1H) ; 3,47 (1H) ; 3,56 (1H) ; 4,60 (1H).

LC/MS (ESI): 410,4 ; (calculé ([M+H]⁺): 410,6).

### Molécule A4 : produit obtenu par la réaction entre la molécule A3 et la Boc-1-amino-4,7,10-trioxa-13-tridécane.

A une solution de molécule A3 (10,96 g, 26,75 mmol) dans du THF(135 mL) sont ajoutés de la DIPEA (9,32 mL) et du 2-(1H-benzotriazol-1-yl)-1,1,3,3-tétraméthyluronium tétrafluoroborate (TBTU) (9,45 g, 29,4 mmol) à température ambiante. Après 30 min d'agitation, de la Boc-1-amino-4,7,10-trioxa-13-tridécane (10,29 g, 32,11 mmol) est ajoutée. Après agitation à température ambiante pendant 18 h, le solvant est évaporé sous pression réduite et le résidu est dilué avec de l'acétate d'éthyle (400 mL). La phase organique est lavée avec une solution saturée de NaHCO₃, une solution aqueuse de HCl 1N, de l'eau, une solution aqueuse saturée en NaCl puis séchée sur Na₂SO₄, filtrée et concentrée sous vide.Le résidu est purifié par chromatographie flash (cyclohexane, acétate d'éthyle, méthanol) pour donner une huile incolore qui se solidifie. Un solide est obtenu.

Rendement : 14,2 g (75%).

RMN ¹H (CDCl₃, ppm) : 0,88 (3H) ; 1,24 (32H) ; 1,43 (9H) ; 1,57-2,00 (8H) ; 2,10-2,45 (4H) ; 3,20-3,75 (18H) ; 4,30 (0,20H) ; 4,55 (0,80H) ; 5,03 (1H) ; 6,75 (0,20H) ; 7,20 (0,80H).

LC/MS (ESI): 712,8 ; (calculé ([M+H]⁺): 713,1).

### Molécule AA7

A une solution de la molécule A4 (14,25 g, 20,01 mmol) dans le dichlorométhane (100 mL) à 0 °C est ajoutée une solution de HCl 4N dans le dioxane (25 mL). Après 20 h d'agitation à 0 °C et 4 h d'agitation à 25 °C, le milieu est concentré sous vide. Par quatre fois, le résidu est repris dans le méthanol et évaporé sous pression réduite pour donner un solide blanc de molécule AA7 sous forme de sel de chlorhydrate.

Rendement : 12,7 g (98%).

RMN ¹H (DMSO-d₆, ppm) : 0,85 (3H) ; 1,23 (32H) ; 1,45 (2H) ; 1,64 (2H) ; 1,70-2,05 (6H) ; 2,10-2,30 (2H) ; 2,82 (2H) ; 3,08 (2H) ; 3,30-3,60 (14H) ; 4,15-4,30 (1H) ; 7,73-8,13 (4H).

LC/MS (ESI): 612,7 ; (calculé ([M+H]⁺): 612,9).

### Exemple AA10 : molécule AA10

### Molécule A23 : produit obtenu par la réaction entre l'acide nipécotique et l'acide arachidique.

Par un procédé similaire à celui utilisé pour la préparation de la molécule A3 appliqué à l'acide arachidique (2,30 g, 7,37 mmol) et à l'acide nipécotique (1,00 g, 7,74 mmol), un solide blanc est obtenu après filtration de la phase aqueuse acidifiée jusqu'à pH 1 et lavage du solide à l'eau puis au dichlorométhane.
Rendement : 1,65 g (53 %)
RMN ¹H (CDCl₃, ppm) : 0,88 (3H) ; 1,07-1,88 (37H) ; 2,10 (1H) ; 2,28-2,45 (2H) ; 2,52 (1H) ; 2,91-3,17 (1,5H) ; 3,42 (0,5H) ; 3,72 (0,5H) ; 3,84 (0,5H) ; 4,08 (0,5H) ; 4,56 (0,5H).
LC/MS (ESI) : 424,4 ; 848,0 ; (calculé ([M+H]⁺) : 424,4 ; ([2M+H]⁺) : 847,8).

### Molécule A24 : produit obtenu par la réaction entre la molécule A23 et la Boc-1-amino-4,7,10-trioxa-13-tridécane amine.

Par un procédé similaire à celui utilisé pour la préparation de la molécule A4 appliqué à la molécule A23 (1,65 g, 3,89 mmol) et au TBTU (1,01 g, 7,79 mmol), un solide blanc de molécule de molécule A24 est obtenu.
Rendement : 1,97 g (70 %)
RMN ¹H (CDCl₃, ppm) : 0,88 (3H) ; 1,15-2,70 (54H) ; 3,10-3,46 (6H) ; 3,46-3,71 (12,6H) ; 3,92 (0,4H) ; 4,17 (0,6H) ; 4,49 (0,4H) ; 4,80-5,16 (1H) ; 6,35-6,76 (1H). LC/MS (ESI) : 726,8 ; (calculé ([M+H]⁺) : 726,6).

### Molécule AA10

Par un procédé similaire à celui utilisé pour la préparation de la molécule AA4 appliqué à la molécule A24 (1,97 g, 2,71 mmol), un solide blanc de molécule AA10 est obtenu après évaporation du solvant, trituration dans l'acétone, filtration et lavage à l'acétone puis séchage sous pression réduite à 50 °C.
Rendement : 1,66 g (92 %)
RMN ¹H (DMSO-d₆, ppm) : 0,86 (3H) ; 1,09-1,90 (42H) ; 2,05-2,68 (5H) ; 2,45-2,68 (1H) ; 2,78-3,19 (6H) ; 3,36-3,44 (2H) ; 3,44-3,60 (10H) ; 3,69-3,87 (1H) ; 4,20 (0,4H) ; 4,35 (0,6H).
LC/MS (ESI) : 626,7 ; (calculé ([M+H]⁺) : 626,5).

### AB : Synthèse des co-polyaminoacides

### Co-polyaminoacides à greffage statistiques (formule VII ou VIIa)

**Tableau 1b: liste de co-polyaminoacides synthétisés selon l'invention**

| **N°** | **co-polyaminoacides porteur de charges carboxylates et de radicaux hydrophobes** |
|---|---|
| AB6 | 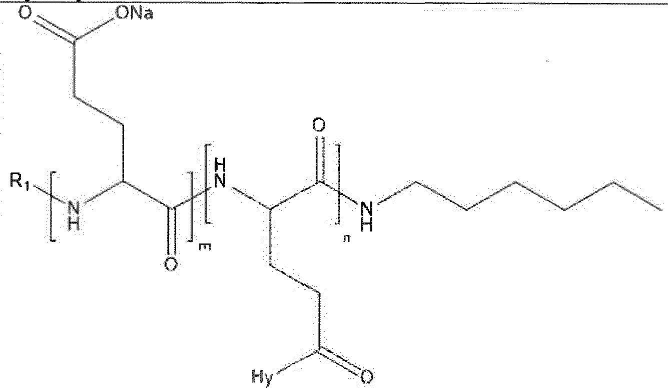 |
| | i = 0,025, DP (m + n) = 20 |
| | 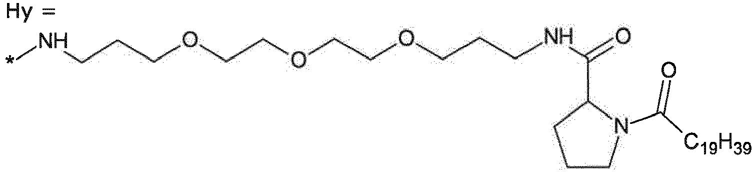 |
| | R₁ = H ou pyroglutamate |
| AB7 | 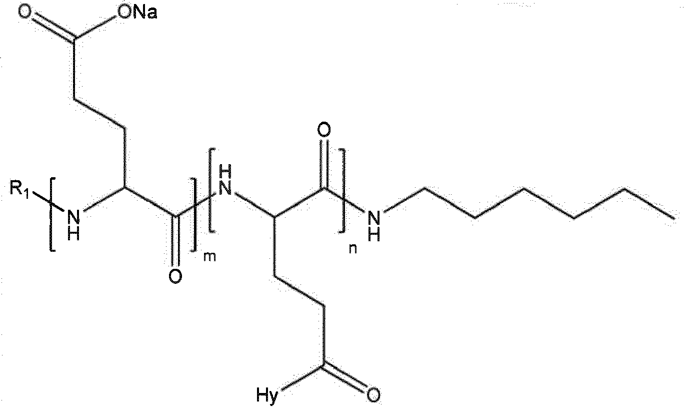 |
| | i = 0,03, DP (m + n) = 21 |
| | 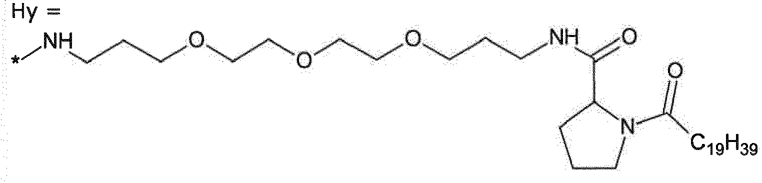 |
| | R1=CH₃-C(O)-, H ou pyroqlutamate |
| AB8 | 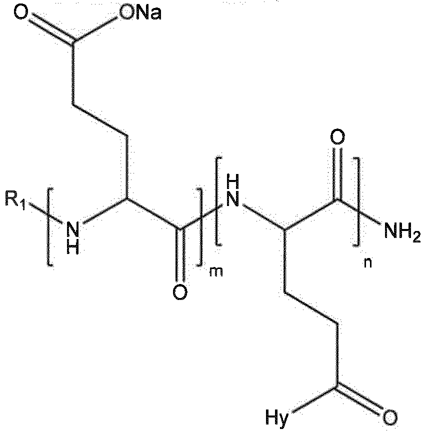 |
| | i = 0,03, DP (m + n) = 24 |
| | 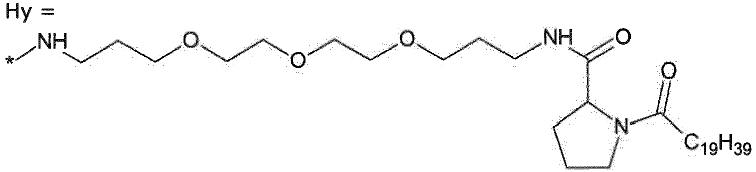 |
| | R₁ = H ou pyroglutamate |
| AB10 | 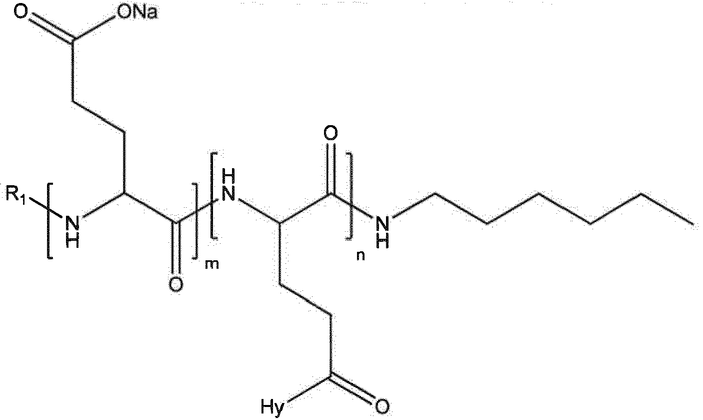 |
| | i = 0,08, DP (m + n) = 25 |
| | 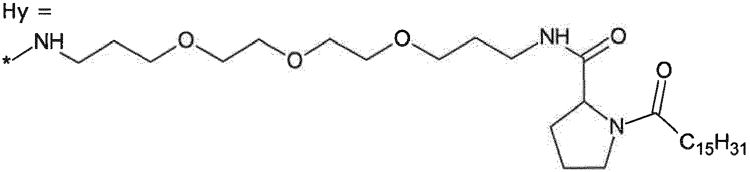 |
| | R₁ = H ou pyroglutamate |
| AB21 | 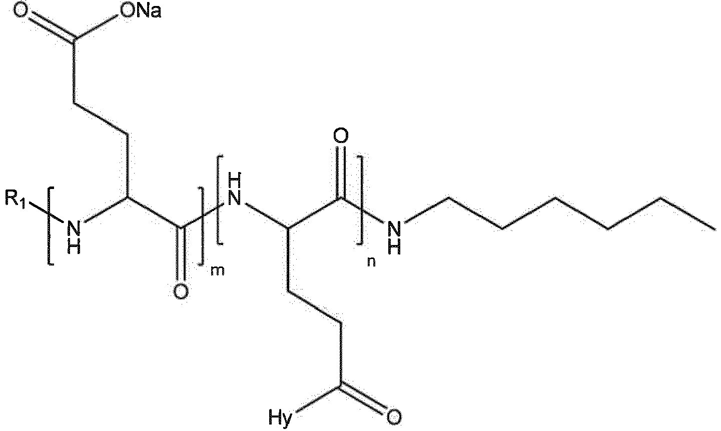 |
| | i = 0,056, DP (m + n) = 22 |
| | 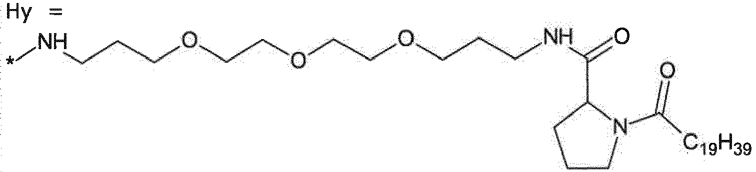 |
| | R₁ = H ou pyroglutamate |

### Co-polyaminoacides à greffage défini (formule VII ou VIIb)

**Tableau 1c : liste de co-polyaminoacides synthétisés selon l'invention.**

| **N°** | **co-polyaminoacides porteur de charges carboxylates et de radicaux hydrophobes** |
|---|---|
| AB17 | 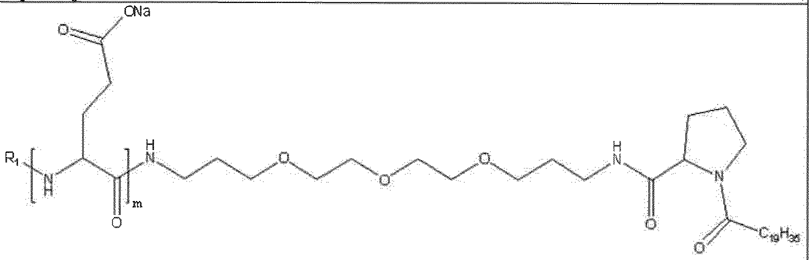 |
| | i = 0,038, DP (m) = 26 |
| | R₁ = H ou pyroglutamate |
| AB18 | 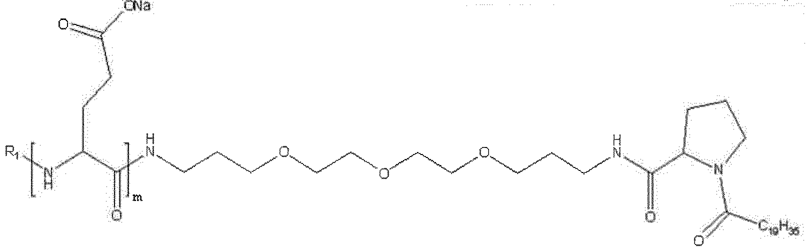 |
| | i = 0,045, DP (m) = 22 |
| | R₁ = H ou pyroglutamate |
| AB21' | 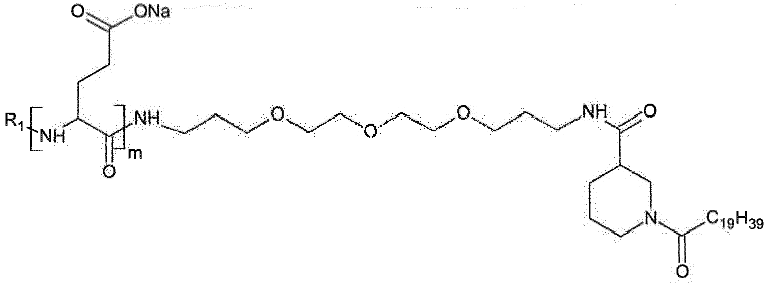 i = U,U4, DP (m) = 25 |
| | R₁ = H ou pyroglutamate |

### Exemple AB6 : Co-polyaminoacide AB6 - poly-L-glutamate de sodium modifié par la molécule AA7 et ayant une masse molaire moyenne en nombre (Mn) de 4000 g/mol

Co-polyaminoacide AB6-1 : acide poly-L-glutamique de masse molaire moyenne en nombre (Mn) relative 3500 g/mol issu de la polymérisation du γ-benzyl-L-glutamate N-carboxyanhydride initiée par l'hexylamine.

Dans un ballon préalablement séché à l'étuve est placé sous vide du γ-benzyl-L-glutamate N-carboxyanhydride (89,9 g, 341 mmol) pendant 30 min, puis du DMF anhydre (200 mL) est introduit. Le mélange est alors agité sous argon jusqu'à complète dissolution, refroidi à 4 °C, puis de l'hexylamine (2,05 mL, 15,5 mmol) est introduite rapidement. Le mélange est agité entre 4 °C et température ambiante pendant 2 jours. Le milieu réactionnel est ensuite chauffé à 65 °C pendant 2 h, refroidi à température ambiante puis coulé goutte à goutte dans du diisopropyléther (3 L) sous agitation. Le précipité blanc est récupéré par filtration, lavé avec du diisopropyléther (2 x 200 mL) puis séché sous vide à 30 °C pour donner un acide poly(gamma-benzyl-L-glutamique) (PBLG).

A une solution de PBLG (74,8 g) dans l'acide trifluoroacétique (TFA, 340 mL) à 4 °C est ajoutée goutte à goutte une solution d'acide bromhydrique (HBr) à 33% dans l'acide acétique (240 mL, 1,37 mol). Le mélange est agité à température ambiante pendant 2 h, puis coulé goutte à goutte sur un mélange 1:1 (v/v) de diisopropyléther et d'eau sous agitation (4 L). Après 2 h d'agitation, le mélange hétérogène est laissé au repos pendant une nuit. Le précipité blanc est récupéré par filtration, lavé avec un mélange 1:1 (v/v) de diisopropyléther et d'eau (340 mL) puis avec de l'eau (340 mL).

Le solide obtenu est alors solubilisé dans de l'eau (1,5 L) en ajustant le pH à 7 par ajout d'une solution aqueuse de soude 10 N puis une solution aqueuse de soude 1 N. Après solubilisation, la concentration théorique est ajustée à 20 g/L théorique par addition d'eau pour obtenir un volume final de 2,1 L.

La solution est filtrée sur filtre 0,45 µm puis purifiée par ultrafiltration contre une solution de NaCl 0,9 %, puis de l'eau jusqu'à ce que la conductimétrie du perméat soit inférieure à 50 µS/cm. La solution de co-polyaminoacide est ensuite concentrée jusqu'à obtenir un volume final de 1,8 L.

La solution aqueuse est alors acidifiée par ajout de solution d'acide chlorhydrique 37% jusqu'à atteindre un pH de 2. Après 4 h d'agitation, le précipité obtenu est filtré, lavé avec de l'eau (2 x 340 mL) puis séché sous vide à 30°C pour donner un acide poly-L-glutamique de masse molaire moyenne en nombre (Mn) 3500 g/mol par rapport à un standard de polyoxyéthylène (PEG).

### Co-polyaminoacide AB6

Le co-polyaminoacide AB6-1 de masse molaire moyenne en nombre (Mn) 3500 g/mol (10,0 g) est solubilisé dans le DMF (420 mL) à 30°C-40°C puis maintenu à cette température. En parallèle, le sel de chlorhydrate de la molécule AA7 (1,47 g, 2,3 mmol) est mis en suspension dans du DMF (12 mL) et de la triéthylamine (0,23 g, 2,3 mmol) est ajoutée, puis le mélange est légèrement chauffé sous agitation jusqu'à complète dissolution. A la solution de co-polyaminoacide dans le DMF, de la NMM (7,6 g, 75 mmol), la solution de AA7 puis de la N-oxyde de 2-hydroxypyridine (HOPO, 0,84 g, 7,5 mmol) sont ajoutées successivement. Le milieu réactionnel est alors refroidi à 0°C, puis du EDC (1,44 g, 7,5 mmol) est ajouté et le milieu est remonté à température ambiante durant 2 h. Le milieu réactionnel est filtré sur filtre tissé 0,2 mm et coulé au goutte-à-goutte sur 3,5 L d'eau contenant du NaCl à 15% massique et du HCl (pH 2) sous agitation. A la fin de l'ajout, le pH est réajusté à 2 avec une solution de HCl 37%, et la suspension est laissée reposer une nuit. Le précipité est collecté par filtration, puis rincé par 100 mL d'eau. Le solide blanc obtenu est solubilisé dans 500 mL d'eau par ajout lent d'une solution aqueuse de NaOH 1 N jusqu'à pH 7 sous agitation, puis la solution est filtrée sur filtre 0,45 µm. La solution limpide obtenue est purifiée par ultrafiltration contre une solution de NaCl 0,9% puis de l'eau, jusqu'à ce que la conductimétrie du perméat soit inférieure à 50 µS/cm. La solution est filtrée sur filtre 0,2 µm et stockée à 2-8 °C.

Extrait sec : 21,6 mg/g.

DP (estimé d'après la RMN ¹H : 20.

D'après la RMN ¹H : i = 0,025.

La masse molaire moyenne calculée du co-polyaminoacide AB6 est de 3369 g/mol.

HPLC-SEC aqueuse (calibrant PEG) : Mn = 4000 g/mol.

### Exemple AB7 : Co-polyaminoacide AB7 - poly-L-glutamate de sodium cappé à une de ses extrémités par un groupement acétyle, modifié par la molécule AA7 et ayant une masse molaire moyenne en nombre (Mn) de 3300 g/mol

Co-polyaminoacide AB7-1 : acide poly-L-glutamique de masse molaire moyenne en nombre (Mn) relative 3600 g/mol et de DP 21 issu de la polymérisation du γ-benzyl-L-glutamate N-carboxyanhydride initiée par l'hexylamine et cappé à l'une de ses extrémités par un groupement acétyle.

Dans un ballon préalablement séché à l'étuve est placé sous vide du γ-benzyl-L-glutamate N-carboxyanhydride (Glu(OBn)-NCA, 100,0 g, 380 mmol) pendant 30 minutes puis du DMF anhydre (225 mL) est introduit. Le mélange est alors agité sous argon jusqu'à complète dissolution, refroidi à 4°C, puis de l'hexylamine (1,78 g, 17 mmol) est introduite rapidement. Le mélange est agité entre 4°C et température ambiante pendant 2 jours puis précipité dans du diisopropyléther (3,4 L). Le précipité est récupéré par filtration, lavé deux fois avec du diisopropyléther (225 mL) puis séché pour donner un solide blanc qui est dissous dans 450 mL de THF. A cette solution sont ajoutés successivement de la DIPEA, (31 mL, 176 mmol) puis de l'anhydride acétique (17 mL, 176 mmol). Après une nuit d'agitation à température ambiante, la solution est versée lentement dans du diisopropyléther (3 L) sous agitation. Après 1 h d'agitation, le précipité est filtré, lavé deux fois avec du diisopropyléther (250 mL) puis séché sous vide à 30 °C pour donner un acide poly(gamma-benzyl-L-glutamique) cappé à une de ses extrémités par un groupement acétyle.

A une solution du co-polyaminoacide ci-dessus (72 g) dans l'acide trifluoroacétique (TFA, 335 mL) à 4°C est ajoutée goutte à goutte une solution d'acide bromhydrique (HBr) à 33% dans l'acide acétique (235 mL). Le mélange est agité à température ambiante pendant 3 h 30, puis coulé goutte à goutte sur un mélange 1:1 (v/v) de diisopropyléther et d'eau sous agitation (4 L). Après 2 h d'agitation, le mélange hétérogène est laissé au repos pendant une nuit. Le précipité blanc est récupéré par filtration, lavé avec un mélange 1:1 (v/v) de diisopropyléther et d'eau (340 mL) puis avec de l'eau (340 mL).

Le solide obtenu est alors solubilisé dans de l'eau (1,5 L) en ajustant le pH à 7 par ajout d'une solution aqueuse de soude 10N puis une solution aqueuse de soude 1N. Après solubilisation, la solution est diluée par addition d'eau pour obtenir un volume final de 2,1 L. La solution est filtrée sur filtre 0,45 µm puis purifiée par ultrafiltration contre une solution de NaCl 0,9 %, puis de l'eau jusqu'à ce que la conductimétrie du perméat soit inférieure à 50 µS/cm. La solution de co-polyaminoacide est ensuite concentrée jusqu'à obtenir un volume final de 1,8 L.

La solution aqueuse est alors acidifiée par ajout de solution d'acide chlorhydrique 37% jusqu'à atteindre un pH de 2. Après 4 h d'agitation, le précipité obtenu est filtré, lavé avec de l'eau (330 mL) puis séché sous vide à 30°C pour donner un acide poly-L-glutamique de masse molaire moyenne en nombre (Mn) 3600 g/mol par rapport à un standard de polyoxyéthylène (PEG), et de degré moyen de polymérisation 21.

### Co-polyaminoacide AB7

Par un procédé similaire à celui utilisé pour la préparation du co-polyaminoacide AB6 appliqué au sel de chlorhydrate de la molécule AA7 (1,43 g, 2,2 mmol) et au co-polyaminoacide AB7-1 (10,0 g), un acide poly-L-glutamate de sodium modifié par la molécule AA7 est obtenu.

Extrait sec : 24,3 mg/g.

DP (estimé d'après la RMN ¹H) : 21.

D'après la RMN ¹H : i = 0,03.

La masse molaire moyenne calculée du co-polyaminoacide AB7 est de 3677 g/mol.

HPLC-SEC aqueuse (calibrant PEG) : Mn = 3300 g/mol.

### Exemple AB8 : Co-polyaminoacide AB8 - poly-L-glutamate de sodium modifié par la molécule AA7 et ayant une masse molaire moyenne en nombre (Mn) de 3600 g/mol

Co-polyaminoacide AB8-1 : acide poly-L-glutamique de masse molaire moyenne en nombre (Mn) 3800 g / mol issu de la polymérisation du γ-méthyl-L-glutamate N-carboxyanhydride initiée par l'ammoniac.

Par un procédé similaire à celui décrit dans la demande de brevet FR-A-2 801 226 appliqué au γ-méthyl-L-acide glutamique N-carboxyanhydride (25,0 g, 133,6 mmol) et à une solution d'ammoniaque 0,5 N dans le dioxane (12,1 mL, 6,05 mmol), un acide poly-L-glutamique est obtenu.

Co-polyaminoacide AB8

Par un procédé similaire à celui utilisé pour la préparation du co-polyaminoacide AB6 appliqué au sel de chlorhydrate de la molécule AA7 (2,1 g, 3,24 mmol) et au co-polyaminoacide AB8-1 (14,3 g), un poly-L-glutamate de sodium modifié par la molécule AA7 est obtenu.

Extrait sec : 25,2 mg/g.

DP (estimé d'après la RMN ¹H) : 24.

D'après la RMN ¹H : i = 0,03.

La masse molaire moyenne calculée du co-polyaminoacide AB8 est de 4099 g/mol.

HPLC-SEC aqueuse (calibrant PEG) : Mn = 3600 g/mol.

### Exemple AB10: Co-polyaminoacide AB10 - poly-L-glutamate de sodium modifié par la molécule AA4 et ayant une masse molaire moyenne en nombre (Mn) de 2600 g/mol

Par un procédé similaire à celui utilisé pour la préparation du co-polyaminoacide AB7 appliqué au sel de chlorhydrate de la molécule AA4 et à un acide poly-L-glutamique obtenu par un procédé similaire à celui utilisé pour la préparation du co-polyaminoacide AB6-1, un poly-L-glutamate de sodium modifié par la molécule AA4 est obtenu.

Extrait sec : 18,3 mg/g.

DP (estimé d'après la RMN ¹H) : 25.

D'après la RMN ¹H : i = 0,08.

La masse molaire moyenne calculée du co-polyaminoacide AB10 est de 4870 g/mol.

HPLC-SEC aqueuse (calibrant PEG) : Mn = 2600 g/mol.

### Exemple AB21: Co-polyaminoacide AB21 - poly-L-glutamate de sodium modifié par la molécule AA7 et ayant une masse molaire moyenne en nombre (Mn) de 3400 g/mol

Par un procédé similaire à celui utilisé pour la préparation du co-polyaminoacide AB6 appliqué au sel de chlorhydrate de la molécule AA7 (2,44 g, 2,4 mmol) et à un acide poly-L-glutamique (10 g) obtenu par un procédé similaire à celui utilisé pour la préparation du co-polyaminoacide AB6-1, un poly-L-glutamate de sodium modifié par la molécule AA7 est obtenu.

Extrait sec : 22,7 mg/g.

DP (estimé d'après la RMN ¹H) : 22.

D'après la RMN ¹H : i = 0,056.

La masse molaire moyenne calculée du co-polyaminoacide AB21 est de 4090 g/mol.

HPLC-SEC aqueuse (calibrant PEG) : Mn = 3400 g/mol.

### Exemple AB17: Co-polyaminoacide AB17 - poly-L-glutamate de sodium modifié à une de ses extrémités par la molécule AA7 et ayant une masse molaire moyenne en nombre (Mn) de 3500 g/mol

Dans un contenant adapté sont introduits successivement le sel de chlorhydrate de la molécule AA7 (2,80 g, 4,32 mmol), du chloroforme (5 mL), du tamis moléculaire 4Â (1,3 g), ainsi que de la résine échangeuse d'ion Amberlite IRN 150 (1,3 g). Après 1 h d'agitation sur rouleaux, le milieu est filtré et la résine est rincée avec du chloroforme. Le mélange est évaporé puis co-évaporé avec du toluène. Le résidu est solubilisé dans du DMF anhydre (30 mL) pour être utilisé directement dans la réaction de polymérisation.

Dans un ballon préalablement séché à l'étuve, du γ-benzyl-L-glutamate N-carboxyanhydride (25,0 g, 94,9 mmol) est placé sous vide pendant 30 min puis du DMF anhydre (140 mL) est introduit. Le mélange est agité sous argon jusqu'à solubilisation complète, refroidi à 4 °C, puis la solution de molécule AA7 préparée comme décrit précédemment est introduite rapidement. Le mélange est agité entre 4 °C et température ambiante pendant 2 jours, puis chauffé à 65 °C pendant 2 h. Le mélange réactionnel est alors refroidi à température ambiante puis versé goutte à goutte dans du diisopropyléther (1,7 L) sous agitation. Le précipité blanc est récupéré par filtration, lavé deux fois avec du diisopropyléther (140 mL) puis séché sous vide à 30 °C pour obtenir un solide blanc. Le solide est dilué dans du TFA (160 mL), et une solution d'acide bromhydrique (HBr) à 33% dans de l'acide acétique (62 mL, 354 mmol) est alors ajoutée goutte à goutte et à 0 °C. La solution est agitée pendant 2 h à température ambiante puis est coulée goutte à goutte sur un mélange 1:1 (v/v) de diisopropyléther / eau et sous agitation (1,9 L). Après 2 h d'agitation, le mélange hétérogène est laissé au repos pendant une nuit. Le précipité blanc est récupéré par filtration, lavé successivement avec un mélange 1:1 (v/v) de diisopropyléther et d'eau (280 mL) puis avec de l'eau (140 mL). Le solide obtenu est solubilisé dans de l'eau (530 mL) en ajustant le pH à 7 par ajout d'une solution aqueuse de soude 10 N puis une solution aqueuse de soude 1 N. Après solubilisation, la concentration théorique est ajustée à 20 g/L théorique par addition d'eau pour obtenir un volume final de 800 mL. Le mélange est filtré sur filtre 0,45 µm puis est purifié par ultrafiltration contre une solution de NaCl 0,9 % puis de l'eau jusqu'à ce que la conductimétrie du perméat soit inférieure à 50 µS/cm. La solution de co-polyaminoacide est ensuite concentrée à environ 30 g/L théorique et le pH est ajusté à 7,0. La solution aqueuse est filtrée sur 0,2 µm et conservée à 4 °C.

Extrait sec : 25,2 mg/g.

DP (estimé par RMN ¹H) = 26 donc i = 0,038.

La masse molaire moyenne calculée du co-polyaminoacide AB17 est de 4500 g/mol.

HPLC-SEC aqueuse (calibrant PEG) : Mn = 3500 g/mol.

### Exemple AB18: Co-polyaminoacide AB18 - poly-L-glutamate de sodium modifié à une de ses extrémités par la molécule AA7 et ayant une masse molaire moyenne en nombre (Mn) de 3700 g/mol

Un poly-L-glutamate de sodium modifié à une de ses extrémités par la molécule AA7 est obtenu par polymérisation du γ-méthyl N-carboxyanhydride d'acide glutamique (25,0 g, 133,6 mmol) en utilisant le sel chlorhydrate de la molécule AA7 (2,80 g, 4,32 mmol) comme initiateur et en effectuant une déprotection des esters méthyliques par utilisation d'une solution d'acide chlorhydrique à 37% selon le procédé décrit dans la demande de brevet FR-A-2 801 226.

Extrait sec : 44,3 mg/g.

DP (estimé par RMN ¹H) = 22 donc i = 0,045.

La masse molaire moyenne calculée du co-polyaminoacide AB18 est de 3896 g/mol.

HPLC-SEC aqueuse (calibrant PEG) : Mn = 3700 g/mol.

### Exemple AB21' : Co-polyaminoacide AB21' - poly-L-glutamate de sodium modifié à une de ses extrémités par la molécule AA10 et ayant une masse molaire moyenne en nombre (Mn) de 3500 g/mol

Par un procédé similaire à celui utilisé pour la préparation du co-polyaminoacide AB17 appliqué au sel de chlorhydrate de la molécule AA10 (0,916 g, 1,38 mmol) et au γ-benzyl-L-glutamate *N*-carboxyanhydride (7,19 g, 27,3 mmol), un poly-L-glutamate de sodium modifié à une de ses extrémités par la molécule AA10 est obtenu.

Extrait sec : 14,8 mg/g

DP (estimé par RMN ¹H) = 25 donc i = 0,04

La masse molaire moyenne calculée du co-polyaminoacide AB21' est de 4364 g/mol.

HPLC-SEC aqueuse (calibrant PEG) : Mn = 3500 g/mol.

### Partie B: radicaux hydrophobes

### BB: Synthèse des molécules hydrophobes dans lesquelles p = 2

Les radicaux sont représentés dans le tableau 1c' suivant par la molécule hydrophobe correspondante avant greffage sur le co-polyaminoacide.

**Tableau 1c' : liste de molécules hydrophobes synthétisées selon l'invention.**

| **N°** | **Structure de la molécule hydrophobe avant greffage sur le co-polyaminoacide** |
|---|---|
| BA1 | 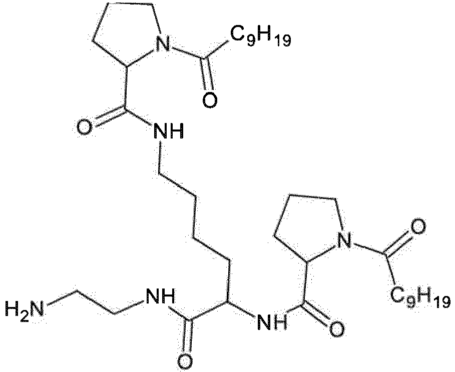 |
| BA2 | 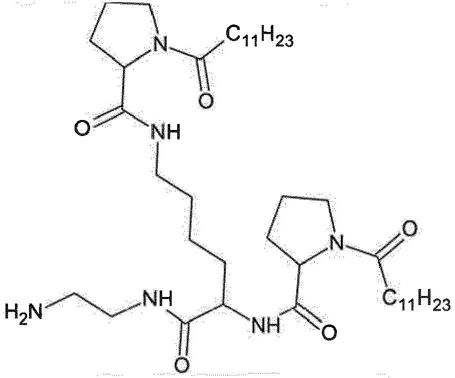 |
| BA3 | 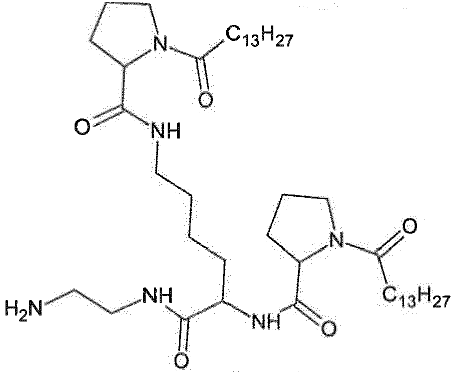 |
| BA4 | 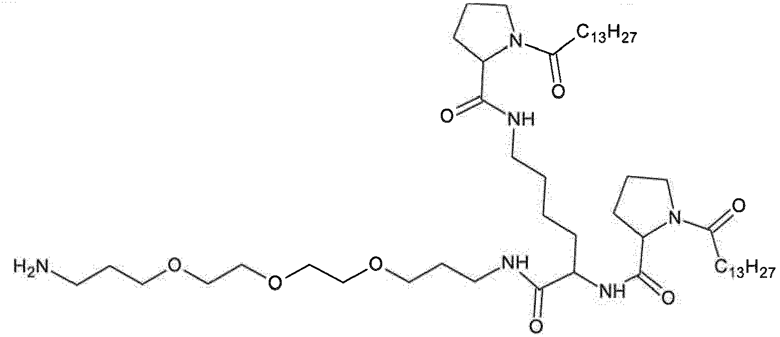 |
| BA5 | 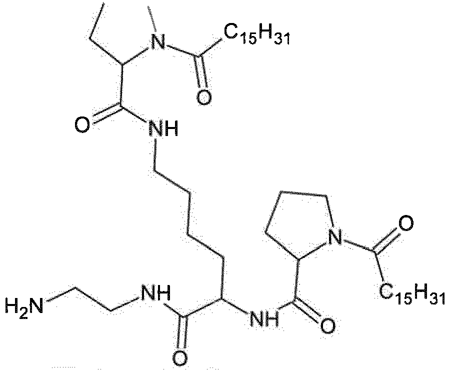 |
| BA6 | 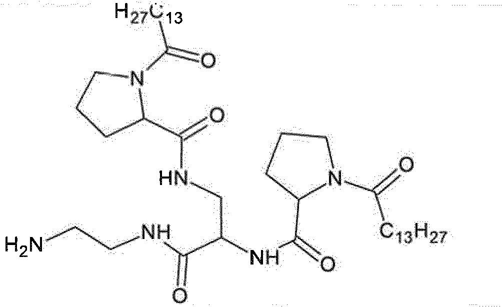 |
| BA7 | 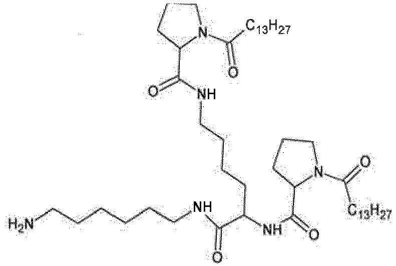 |

### Exemple BA1 : molécule BA1

### Molécule B1: produit obtenu par la réaction entre l'acide décanoïque et la L-proline.

A une solution d'acide décanoïque (14,28 g, 82,91 mmol) dans le THF (520 mL) à 0°C sont ajoutés successivement du dicyclohexyle carbodiimide (DCC) (16,29 g, 78,96 mmol) et du N-hydroxysuccinimide (NHS) (9,09 g, 78,96 mmol). Après 60 h d'agitation à température ambiante, le milieu est refroidi à 0°C pendant 20 min, filtré sur fritté. De la L-proline (10 g, 86,86 mmol), de la diisopropyléthylamine (DIPEA) (68,8 mL) et de l'eau (60 mL) sont ajoutés au filtrat. Après 24 h d'agitation à température ambiante, le milieu est dilué avec de l'eau (300 mL). La phase aqueuse est lavée avec de l'acétate d'éthyle (2 x 250 mL), acidifiée jusqu'à pH ∼1 avec une solution aqueuse d'HCl 1N puis extraite avec du dichlorométhane (3 x 150 mL). Les phases organiques combinées sont séchées sur Na₂SO₄, filtrées, concentrées sous vide et le résidu est purifié par chromatographie sur gel de silice (cyclohexane, acétate d'éthyle).

Rendement : 14,6 g (69%).

RMN ¹H (CDCl₃, ppm) : 0,87 (3H) ; 1,26 (12H) ; 1,65 (2H) ; 2,02 (3H) ; 2,34 (2H) ; 2,41 (1H) ; 3,48 (1H) ; 3,56 (1H) ; 4,58 (1H).

LC/MS (ESI): 270,2; (calculé ([M+H]⁺): 270,4).

### Molécule B2 : produit obtenu par la réaction entre la molécule B1 et la L-lysine.

Par un procédé similaire à celui utilisé pour la préparation de la molécule B1 appliqué à la molécule B1 (14,57 g, 54,07 mmol) et à la L-lysine (4,15 g, 28,39 mmol), une huile jaune est obtenue.

Rendement : 16,4 g (93%).

RMN ¹H (CDCl₃, ppm) : 0,88 (6H) ; 1,26 (24H) ; 1,35-1,65 (8H) ; 1,85-2,35 (12H) ; 2,53 (0,2H) ; 2,90 (0,8H) ; 3,45-3,75 (5H) ; 4,50-4,70 (3H) ; 7,82 (1H).

LC/MS (ESI): 649,6; (calculé ([M+H]⁺): 649,9).

### Molécule B3 : produit obtenu par réaction entre la molécule B2 et la Boc-éthylènediamine.

A une solution de molécule B2 (16,4 g, 25,27 mmol) dans le THF (170 mL) sont ajoutés de la DIPEA (8,80 mL) et du 2-(1H-benzotriazol-1-yl)-1,1,3,3-tétraméthyluronium tétrafluoroborate (TBTU, 8,52 g, 26,54 mmol) à température ambiante. Après 30 min d'agitation, de la Boc-éthylènediamine (4,45 g, 27,8 mmol) est ajoutée. Après agitation à température ambiante pendant 2 h, le solvant est évaporé sous pression réduite et le résidu est dilué avec de l'acétate d'éthyle (400 mL). La phase organique est lavée avec de l'eau (250 mL), une solution aqueuse saturée de NaHCO₃ (250 ml), une solution aqueuse de 1 N HCl (250 mL), une solution aqueuse saturée en NaCl (250 mL) et est séchée sur Na₂SO₄. Après filtration et concentration sous vide, le résidu obtenu est purifié par chromatographie sur gel de silice (acétate d'éthyle, méthanol) pour donner une huile incolore.

Rendement : 12,8 g (64%).

RMN ¹H (CDCl₃, ppm) : 0,87 (6H) ; 1,25-1,60 (42H) ; 1,80-2,05 (4H) ; 2,15-2,45 (9H) ; 3,10-3,75 (10H) ; 4,30 (1H) ; 4,50 (2H) ; 5,50 (0,6H) ; 5,89 (0,2H) ; 6,15 (0,2H) ; 7,03 (1H) ; 7,47 (1H).

LC/MS (ESI): 791,8; (calculé ([M+H]+): 792,1).

### Molécule BA1

A une solution de la molécule B3 (12,78 g, 16,15 mmol) dans le dichlorométhane (110 mL) à 5°C est ajoutée une solution de HCl 4N dans le dioxane (20,2 mL). Après 20 h d'agitation à 5°C, le milieu est concentré sous vide. Le résidu obtenu est dissous dans le méthanol et évaporé sous vide, cette opération étant répétée 4 fois pour donner un solide blanc de molécule BA1 sous forme de sel de chlorhydrate.

Rendement : 11,4 g (97%).

RMN ¹H (DMSO-d₆, ppm) : 0,85 (6H) ; 1,25-1,50 (33H) ; 1,57 (1H) ; 1,70-2,40 (12H) ; 2,82 (2H) ; 3,00 (2H) ; 3,25-3,70 (6H) ; 4,05-4,50 (3H) ; 7,75-8,45 (6H).

LC/MS (ESI): 691,6 ; (calculé ([M+H]⁺): 692,0).

### Exemple BA2 : molécule BA2

### Molécule B4 : produit obtenu par la réaction entre l'acide laurique et la L-proline.

Par un procédé similaire à celui utilisé pour la préparation de la molécule B1 appliqué à l'acide laurique (31,83 g, 157,9 mmol) et à la L-proline (20 g, 173,7 mmol), une huile jaune est obtenue.

Rendement : 34,3 g (73%).

RMN ¹H (CDCl₃, ppm) : 0,87 (3H) ; 1,26 (16H) ; 1,70 (2H) ; 1,90-2,10 (3H) ; 2,35 (2H) ; 2,49 (1H) ; 3,48 (1H) ; 3,56 (1H) ; 4,60 (1H).

LC/MS (ESI): 298,2 ; (calculé ([M+H]⁺): 298,4).

### Molécule B5 : produit obtenu par la réaction entre la molécule B4 et la L-lysine.

Par un procédé similaire à celui utilisé pour la préparation de la molécule B1 appliqué à la molécule B4 (33,72 g, 113,36 mmol) et à la L-lysine (8,70 g, 59,51 mmol), un solide blanc est obtenu.

Rendement : 26,2 g (66%).

RMN ¹H (CDCl₃, ppm) : 0,88 (6H) ; 1,26 (32H) ; 1,35-1,65 (8H) ; 1,85-2,35 (15H) ; 2,87 (1H) ; 3,40-3,75 (5H) ; 4,50-4,75 (3H) ; 7,87 (1H).

LC/MS (ESI): 705,6 ; (calculé ([M+H]⁺): 706,0).

### Molécule B6 : produit obtenu par réaction entre la Boc-éthylènediamine et la molécule B5.

Par un procédé similaire à celui utilisé pour la préparation de la molécule B3 appliqué à la molécule B5 (25,74 g, 36,51 mmol) et à la Boc-éthylènediamine (6,43 g, 40,16 mmol), une huile incolore est obtenue.

Rendement : 30,9 g (quantitatif).

RMN ¹H (CDCl₃, ppm) : 0,88 (6H) ; 1,35-1,65 (50H) ; 1,85-2,35 (13H) ; 3,05-3,75 (10H) ; 4,25-4,65 (3H) ; 5,50 (0,4H) ; 5,88 (0,2H) ; 6,16 (0,2H) ; 7,08 (1H) ; 7,26 (1H) ; 7,49 (0,2H).

LC/MS (ESI): 847,8 ; (calculé ([M+H]⁺): 848,2).

### Molécule BA2

Après un procédé similaire à celui utilisé pour la préparation de la molécule BA1 appliqué à la molécule B6 (30,9 g, 36,47 mmol), le résidu obtenu après concentration sous vide est dissous dans le méthanol et évaporé sous vide, cette opération étant répétée 4 fois pour donner un solide blanc de molécule BA2 sous forme de sel de chlorhydrate après séchage sous pression réduite.

Rendement : 27,65 g (97%).

RMN ¹H (DMSO-d₆, ppm) : 0,85 (6H) ; 1,10-2,40 (54H) ; 2,75-3,15 (4H) ; 3,25-3,60 (6H) ; 4,05-4,50 (3H) ; 7,50-8,50 (6H).

LC/MS (ESI): 747,6 ; (calculé ([M+H]⁺): 748,1).

### Exemple BA3 : molécule BA3

### Molécule B7 : produit obtenu par la réaction entre l'acide myristique et la L-proline.

Par un procédé similaire à celui utilisé pour la préparation de la molécule B1 appliqué à l'acide myristique (18,93 g, 82,91 mmol) et à la L-proline (10 g, 86,86 mmol), une huile jaunâtre est obtenue.

Rendement : 20 g (78%).

RMN ¹H (CDCl₃, ppm) : 0,88 (3H) ; 1,28 (20H) ; 1,70 (2H) ; 1,90-2,10 (3H) ; 2,36 (2H) ; 2,51 (1H) ; 3,47 (1H) ; 3,56 (1H) ; 4,61 (1H).

LC/MS (ESI): 326,2; (calculé ([M+H]⁺): 326,6).

### Molécule B8 : produit obtenu par la réaction entre la molécule B7 et la L-lysine

Par un procédé similaire à celui utilisé pour la préparation de la molécule B1 appliqué à la molécule B7 (20,02 g, 61,5 mmol) et à la L-lysine (4,72 g, 32,29 mmol), un solide blanc est obtenu.

Rendement : 12,3 g (53%).

RMN ¹H (DMSO-d₆, ppm) : 0,85 (6H) ; 1,26 (40H) ; 1,35-1,50 (6H) ; 1,50-2,10 (10H) ; 2,10-2,25 (4H) ; 3,01 (2H) ; 3,31-3,55 (4H) ; 4,10-4,40 (3H) ; 7,68 (0,6H) ; 7,97 (1H) ; 8,27 (0,4H) ; 12,50 (1H).

LC/MS (ESI): 761,8 ; (calculé ([M+H]⁺): 762,1).

### Molécule B9 : produit obtenu par la réaction entre la Boc-éthylènediamine et la molécule B8.

Par un procédé similaire à celui utilisé pour la préparation de la molécule B3 appliqué à la molécule B8 (12 g, 15,77 mmol) et à la Boc-éthylènediamine (3,03 g, 18,92 mmol), une huile incolore est obtenue après purification par colonne chromatographique sur gel de silice (acétate d'éthyle, méthanol).

Rendement : 12,5 g (88%).

RMN ¹H (DMSO-d₆, ppm) : 0,85 (6H) ; 1,20-1,55 (55H) ; 1,50-2,25 (14H) ; 2,95-3,10 (6H) ; 3,31-3,55 (4H) ; 4,10-4,40 (3H) ; 6,74 (1H) ; 7,60-8,25 (3H).

LC/MS (ESI): 904,1 ; (calculé ([M+H]⁺): 904,3).

### Molécule BA3

Après un procédé similaire à celui utilisé pour la préparation de la molécule BA1 appliqué à la molécule B9 (12,5 g, 13,84 mmol), le résidu obtenu après concentration sous vide est dissous dans le méthanol et évaporé sous vide, cette opération étant répétée 4 fois pour donner un solide blanc de molécule BA3 sous forme de sel de chlorhydrate après séchage sous pression réduite.

Rendement : 9,2 g (79%).

RMN ¹H (DMSO-d₆, ppm) : 0,85 (6H) ; 1,10-1,65 (48H) ; 1,70-2,35 (12H) ; 2,85 (2H) ; 3,01 (2H) ; 3,25-3,65 (6H) ; 4,10-4,50 (3H) ; 7,70-8,40 (6H).

LC/MS (ESI): 803,9 ; (calculé ([M+H]⁺): 804,2).

### Exemple BA4 : molécule BA4

### Molécule B10 : produit obtenu par la réaction entre la molécule B8 et le Boc-1-amino-4,7,10-trioxa-13-tridécane.

Par un procédé similaire à celui utilisé pour la préparation de la molécule B3 appliqué à la molécule B8 (29,80 g, 39,15 mmol) et au Boc-1-amino-4,7,10-trioxa-13-tridécane (15,05 g, 46,96 mmol), une huile épaisse incolore est obtenue.

Rendement : 25,3 g (61%).

RMN ¹H (DMSO-d₆, ppm) : 0,85 (6H) ; 1,25-2,35 (75H) ; 2,85-3,20 (6H) ; 3,25-3,65 (16H) ; 4,10-4,45 (3H) ; 6,38 (0,1H) ; 6,72 (0,9H) ; 7,50-8,25 (3H).

LC/MS (ESI): 1064,2 ; (calculé ([M+H]⁺): 1064,5).

### Molécule BA4

Après un procédé similaire à celui utilisé pour la préparation de la molécule BA1 appliqué à la molécule B10 (25,3 g, 23,8 mmol), le résidu obtenu après concentration sous vide est dissous dans le méthanol et évaporé sous vide, cette opération étant répétée 4 fois pour donner un solide blanc de molécule BA4 sous forme de sel de chlorhydrate après séchage sous pression réduite.

Rendement : 20,02 g (84%).

RMN ¹H (DMSO-d₆, ppm) : 0,85 (6H) ; 1,15-2,35 (66H) ; 2,80-3,20 (6H) ; 3,30-3,65 (16H) ; 4,10-4,45 (3H) ; 7,55-8,60 (6H).

LC/MS (ESI): 964,9 ; (calculé ([M+H]⁺): 964,6).

### Exemple BA5 : Molécule BA5

### Molécule B12 : produit obtenu par réaction entre la molécule A1 et la L-Lysine

Par un procédé similaire à celui utilisé pour la préparation de la molécule B1 appliqué à la molécule A1 (19,10 g, 54,02 mmol) et à la L-lysine (4,15 g, 28,36 mmol), un résidu huileux est obtenu après concentration du milieu réactionnel sous pression réduite. Ce résidu est dilué dans de l'eau (150 mL), lavé à l'acétate d'éthyle (2 x 75 mL) puis la phase aqueuse est acidifiée jusqu'à pH 1 par addition lente de HCl 6 N. Le produit est extrait 3 fois au dichlorométhane, la phase organique est séchée sur Na₂SO₄ puis filtrée et concentrée sous pression réduite pour donner 11,2 g de résidu huileux jaune. Parallèlement, la phase organique d'acétate d'éthyle précédente est lavée avec une solution aqueuse de HCl 2 N (2 x 75 mL), une solution aqueuse saturée en NaCl (75 mL), séchée sur Na₂SO₄, filtrée et concentrée pour donner 10,2 g de résidu huileux jaune. Un solide blanc est obtenu après recristallisation de chacun de ces résidus dans l'acétone.
Rendement : 11,83 g (54 %)
RMN ¹H (CDCl₃, ppm) : 0,87 (6H) ; 1,06-2,44 (70H) ; 2,78-2,96 (1H) ; 3,35-3,75 (5H) ; 4,28-4,43 (0,1H) ; 4,43-4,52 (0,2H) ; 4,52-4,61 (1,8H) ; 4,61-4,75 (0,9H) ; 7,74-8,02 (2H).
LC/MS (ESI) : 818,0 ; (calculé ([M+H]⁺) : 818,7).

### Molécule B13 : produit obtenu par couplage entre la molécule B12 et la Boc-éthylènediamine

A une solution de molécule B12 (18,00 g, 22,02 mmol) à température ambiante dans le THF (110 mL) est ajoutée de la DIPEA (3,42 g, 26,43 mmol). Le milieu réactionnel est refroidi à 0°C, puis sont ajoutés successivement du HOBt (337 mg, 2,20 mmol), de l'EDC (4,64 g, 24,23 mmol) puis de la Boc-éthylènediamine (4,23 g, 26,43 mmol). Le mélange réactionnel est agité pendant 1 h à 0 °C puis 24 h à température ambiante et concentré sous pression réduite. Le résidu est repris dans l'acétate d'éthyle (250 mL) et du dichlorométhane (40 mL), la phase organique est lavée avec une solution aqueuse de HCl 1 N (2 x 125 mL), une solution aqueuse saturée en NaCl (2 x 125 mL), séchée sur Na₂SO₄ et concentrée sous pression réduite. Un solide blanc est obtenu après recristallisation deux fois dans l'acétonitrile.

Rendement : 17,5 g (83%)

RMN ¹H (DMSO-d6, ppm) : 0,85 (6H) ; 1,15-2,29 (79H) ; 2,92-3,12 (6H) ; 3,30-3,59 (4H) ; 4,06-4,13 (0,65H) ; 4,16-4,29 (2H) ; 4,38-4,42 (0,35H) ; 6,71-6,76 (1H) ; 7,60-7,69 (1,3H) ; 7,76-7,81 (0,65H) ; 7,93-7,97 (0,35H) ; 8,00-8,04 (0,35H) ; 8,10-8,17 (0,35H).

LC/MS (ESI) : 960,4 ; (calculé ([M+H]⁺) : 960,8).

### Molécule BA5

Par un procédé similaire à celui utilisé pour la préparation de la molécule BA1 appliqué à la molécule B13 (24,4 g, 25,43 mmol), le résidu obtenu après concentration sous vide est solubilisé dans du dichlorométhane (150 mL), la phase organique est lavée 2 fois avec une solution aqueuse de soude 2M (90 mL). De l'acétonitrile (120 mL) est ajouté et le dichlorométhane est éliminé par concentration sous pression réduite. Le milieu est ensuite laissé au repos pendant 72 H et un solide blanc est obtenu après filtration et rinçage à l'acétonitrile puis séchage sous pression réduite. Cette opération est répétée 4 fois.

Rendement : 14,28 g (65 %)

RMN ¹H (DMSO-d6, ppm) : 0,85 (6H) ; 1,06-2,32 (70H) ; 2,53-2,63 (2H) ; 2,89-3,61 (10H) ; 4,04-4,43 (3H); 7,55-7,62 (0,65H) ; 7,65-7,72 (0,65H) ; 7,80 (0,65H) ; 7,91 (0,35H) ; 8,03 (0,35H) ; 8,14-8,23 (0,35H).

LC/MS (ESI) : 860,0 ; (calculé ([M+H]⁺) : 860,8).

### Exemple BA6 : molécule BA6

### Molécule B14 : produit obtenu par couplage entre la molécule B7 et l'acide 2,3-diaminopropionique

Par un procédé similaire à celui utilisé pour la préparation de la molécule B1 appliqué à la molécule B7 (80,00 g, 245,78 mmol) et au dichlorhydrate de l'acide 2,3-diaminopropionique (22,84 g, 129,04 mmol), un solide blanc est obtenu après recristallisation dans l'acétonitrile.

Rendement : 69 g (78 %)

RMN ¹H (DMSO-d6, ppm) : 0,86 (6H) ; 1,08-1,38 (40H) ; 1,40-1,55 (4H) ; 1,68-2,30 (12H) ; 3,16-3,66 (6H) ; 4,20-4,39 (3H) ; 7,67-8,31 (2H) ; 12,70 (1H).

LC/MS (ESI) : 719,4 ; 741,5 ; (calculé ([M+H]⁺) : 719,6 ; ([M+Na]⁺) : 741,6).

### Molécule B15 : produit obtenu par couplage entre la molécule B14 et la Boc-éthylènediamine

Par un procédé similaire à celui utilisé pour la préparation de la molécule B13 appliqué à la molécule B14 (32,00 g, 44,50 mmol) en solution dans le dichlorométhane et à la Boc-éthylènediamine (8,56 g, 53,40 mmol), une huile incolore est obtenue après purification par chromatographie sur gel de silice (acétate d'éthyle, méthanol).

Rendement : 24,5 g (64 %)

RMN ¹H (DMSO-d6, ppm) : 0,85 (6H) ; 1,16-2,42 (65H) ; 2,89-3,14 (4H) ; 3,17-3,66 (6H) ; 4,11-4,43 (3H) ; 6,77 (1H) ; 7,38-8,23 (3H).

LC/MS (ESI) : 861,7 ; (calculé ([M+H]⁺) : 861,7).

### Molécule BA6

Par un procédé similaire à celui utilisé pour la préparation de la molécule BA5 appliqué à la molécule B15 (24,50 g, 28,45 mmol), un solide blanc est obtenu après recristallisation dans l'acétonitrile.

Rendement : 19,7 g (91 %)

RMN ¹H (DMSO-d6, ppm) : 0,85 (6H) ; 1,10-2,40 (58H) ; 2,51-2,62 (2H) ; 2,90-3,16 (2H) ; 3,16-3,67 (6H) ; 4,04-4,47 (3H) ; 7,33-8,27 (3H).

LC/MS (ESI) : 761,5 ; (calculé ([M+H]⁺) : 761,6).

### Exemple BA7 : molécule BA7

### Molécule B16 : produit obtenu par la réaction entre le N-(tert-butoxycarbonyl)-1,6-diaminohexane et la molécule B8.

Par un procédé similaire à celui utilisé pour la préparation de la molécule B13 appliqué à la molécule B8 (10 g, 13,14 mmol) et au *N*-(*tert*-butoxycarbonyl)-1,6-diaminohexane (3,41 g, 15,77 mmol) dans le dichlorométhane, un solide blanc est obtenu après recristallisation dans l'acétonitrile.

Rendement : 10,7 g (85%)

RMN ¹H (CDCl₃, ppm) : 0,88 (6H) ; 1,17-2,40 (79H) ; 3,00-3,71 (10H) ; 4,26-4,58 (3H) ; 4,67 (1H) ; 6,74 (1H) ; 7,34-7,49 (2H).

LC/MS (ESI) : 959,9 ; (calculé ([M+H]⁺) : 959,8).

### Molécule BA7

Après un procédé similaire à celui utilisé pour la préparation de la molécule BA1 appliqué à la molécule B16 (10,5 g, 10,94 mmol), une solution aqueuse de NaOH 2N est ajoutée goutte à goutte au milieu réactionnel refroidi à 0°C. La phase aqueuse est extraite au dichlorométhane puis la phase organique est lavée 3 fois avec une solution aqueuse de NaCl 5%. Après séchage sur Na₂SO₄, la phase organique est filtrée, concentrée sous vide et le résidu est recristallisé dans l'acétonitrile.

Rendement : 5,4 g (58%)

RMN ¹H (CDCl₃, ppm) : 0,88 (6H) ; 1,19-2,40 (72H) ; 2,67 (2H) ; 3,03-3,70 (8H) ; 4,26-4,57 (3H) ; 6,71 (1H) ; 7,39-7,49 (2H).

LC/MS (ESI) : 859,8 ; (calculé ([M+H]⁺) : 859,7).

### BB : Synthèse des co-polyaminoacides

### Co-polyaminoacides à greffage statistiques (formule VII et VIIa)

**Tableau 1d: liste de co-polyaminoacides synthétisés selon l'invention**

| **N°** | **co-polyaminoacides porteurs de charges carboxylates et de radicaux hydrophobes** |
|---|---|
| BB1 | 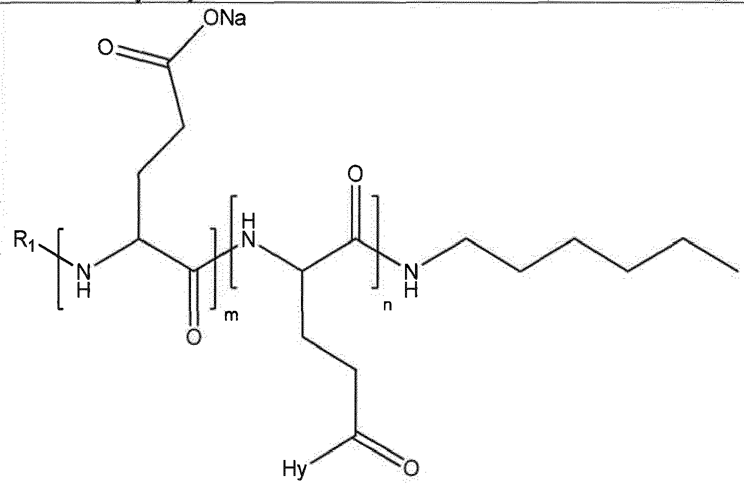 |
| | i = 0,05, DP (m + n) = 23 |
| | 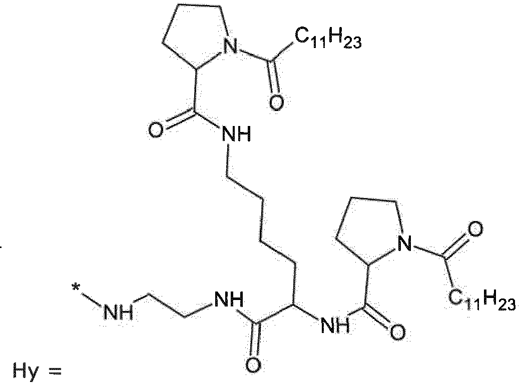 |
| | R₁ = H ou pyroglutamate |
| BB2 | 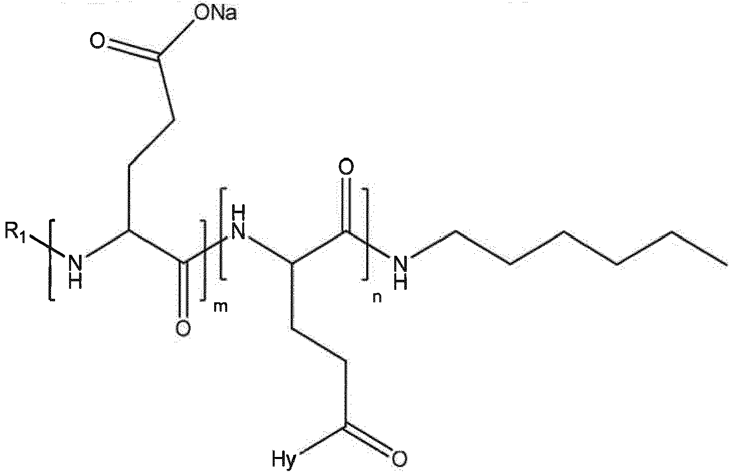 |
| | i = 0,047, DP (m + n) = 21 |
| | 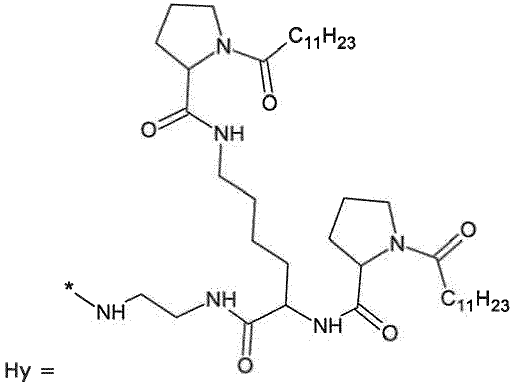 |
| | R₁ = H ou pyroglutamate |
| BB3 | 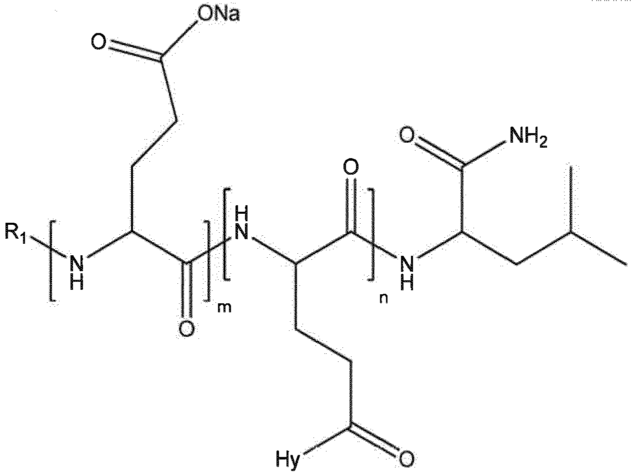 |
| | i = 0.049, DP (m + n) = 34 |
| | 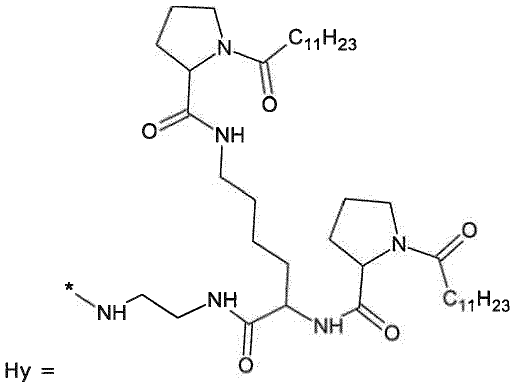 |
| | R1 = H ou pyroglutamate |
| BB4 | 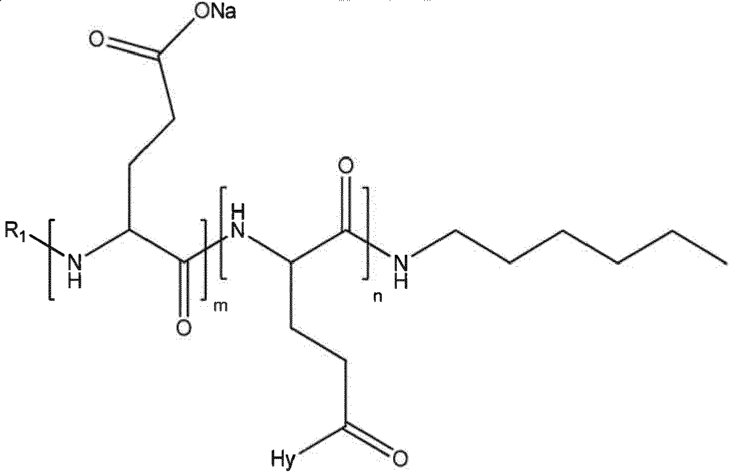 |
| | i = 0,04, DP (m + n) = 65 |
| | 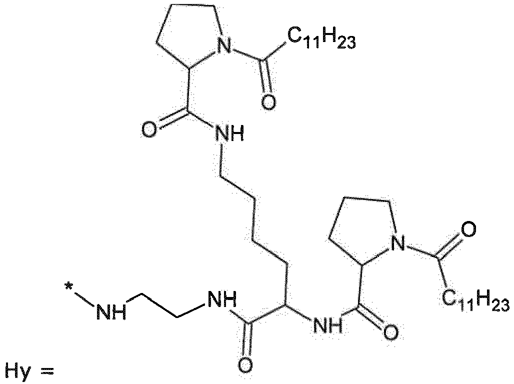 |
| | R1 = H ou pyroglutamate |
| BB5 | 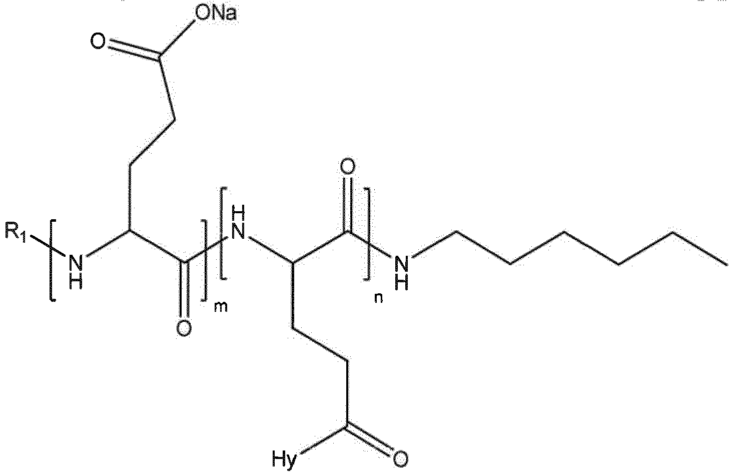 |
| | i = 0,042, DP (m + n) = 23 |
| | 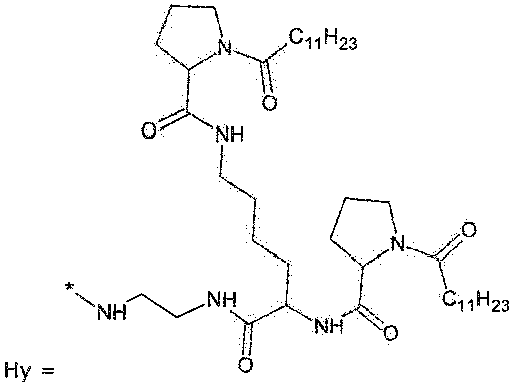 |
| | R₁= CH₃-C(O)-, H ou pyroglutamate |
| BB6 | 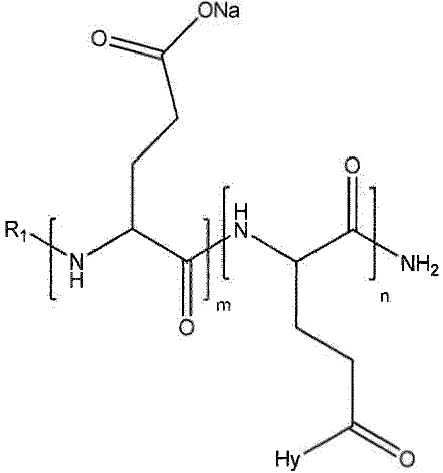 |
| | i = 0,04, DP (m + n) = 24 |
| | 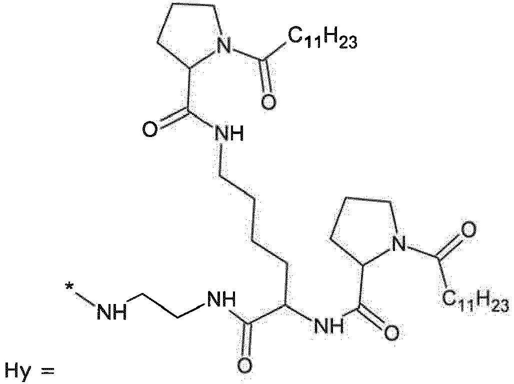 |
| | R₁= CH₃-C(O)-, H ou pyroglutamate |
| BB7 | 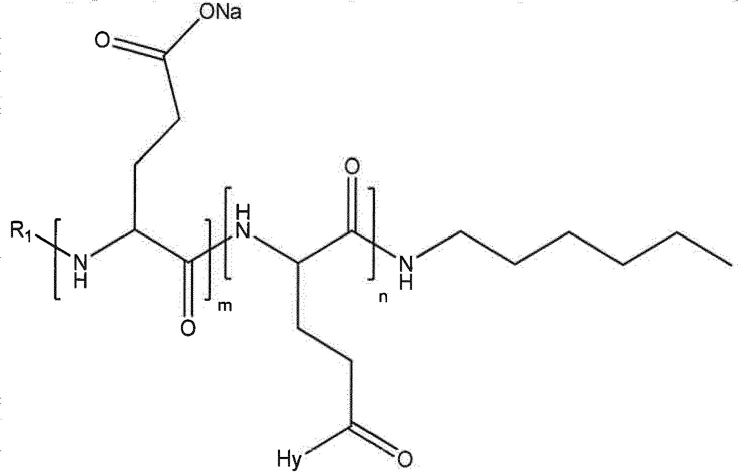 |
| | i = 0,042, DP (m + n) = 22 |
| | 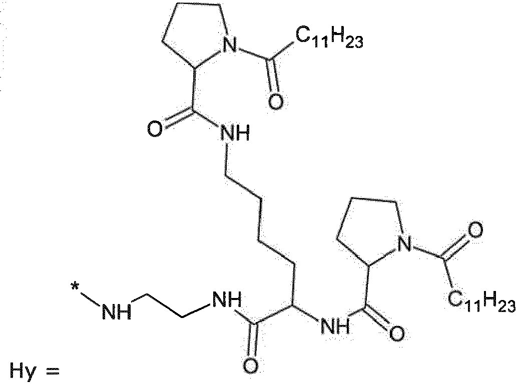 |
| | R₁ = H ou pyroglutamate |
| BB8 | 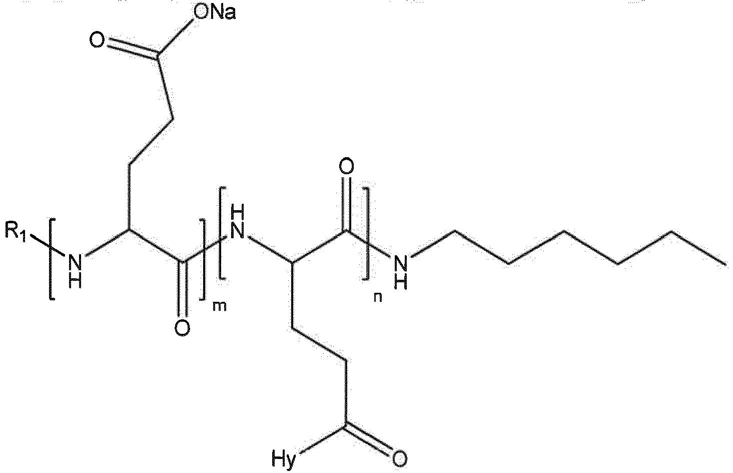 |
| | i = 0,026, DP (m + n) = 21 |
| | 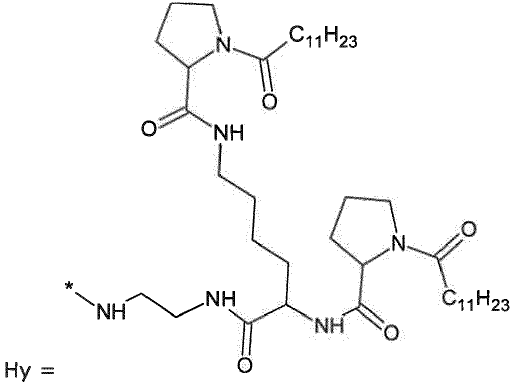 |
| | R₁ = H ou pyroglutamate |
| BB9 | 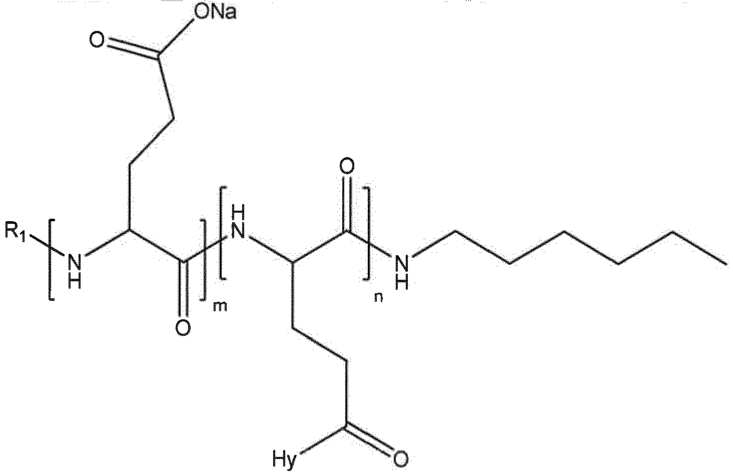 |
| | i = 0,05, DP (m + n) = 26 |
| | 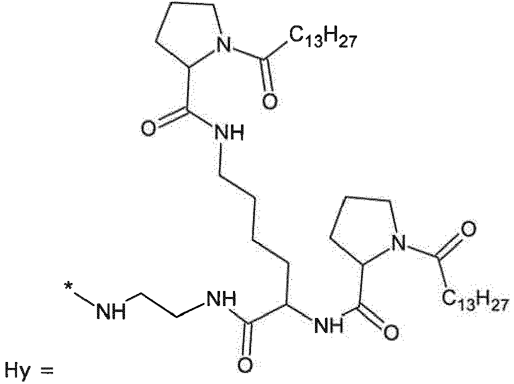 R₁ = H ou pyroglutamate |
| BB10 | 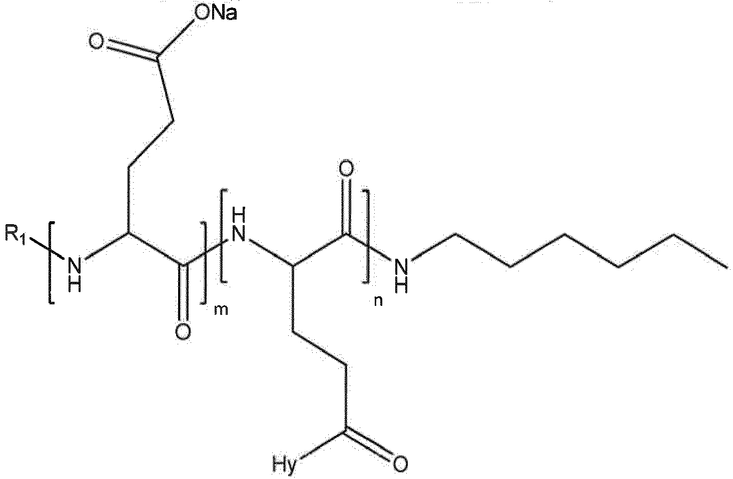 |
| | i = 0,029, DP (m + n) = 22 |
| | 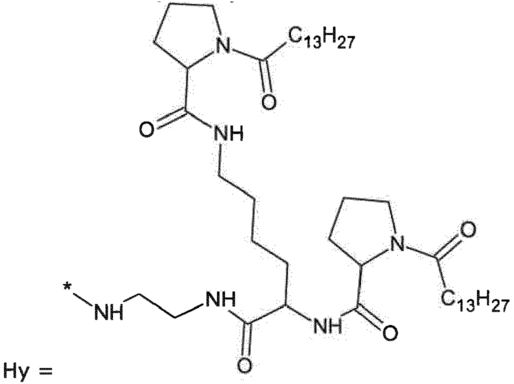 |
| | R1 = H ou pyroglutamate |
| BB11 | 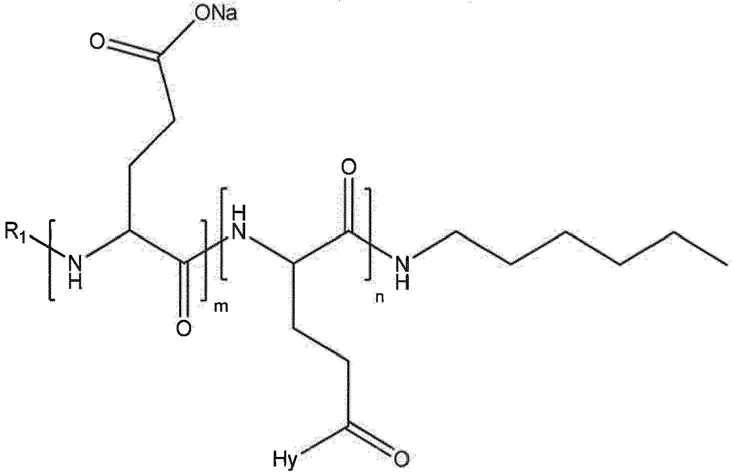 |
| | i = 0,032, DP (m + n) = 22 |
| | 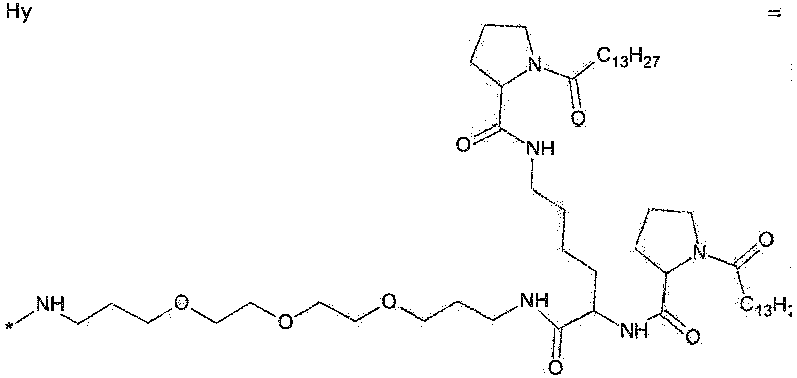 |
| | R₁= CH₃-C(O)-, H ou pyroglutamate |
| BB12 | 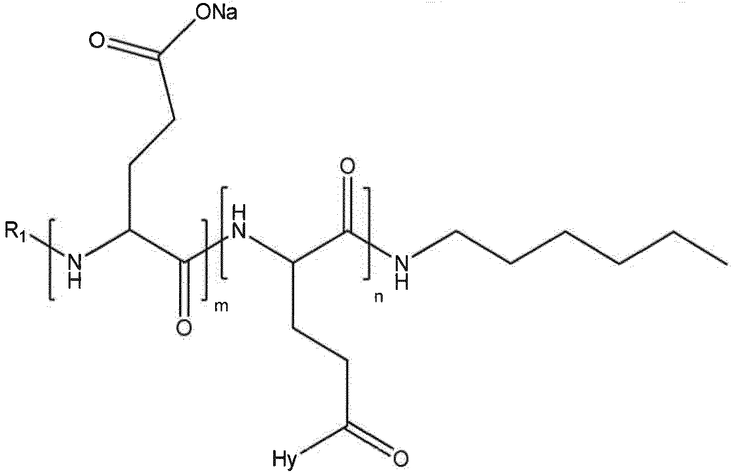 |
| | i = 0,03, DP (m + n) = 23 |
| | 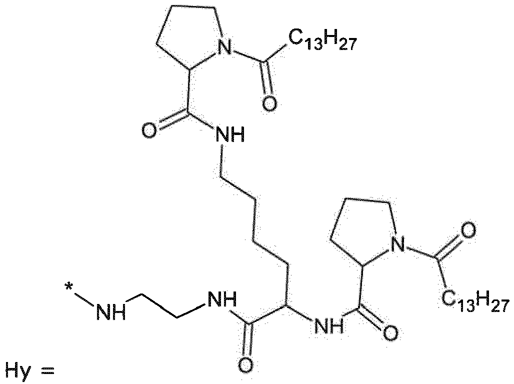 |
| | R₁= CH₃-C(O)-, H ou pyroglutamate |
| BB13 | 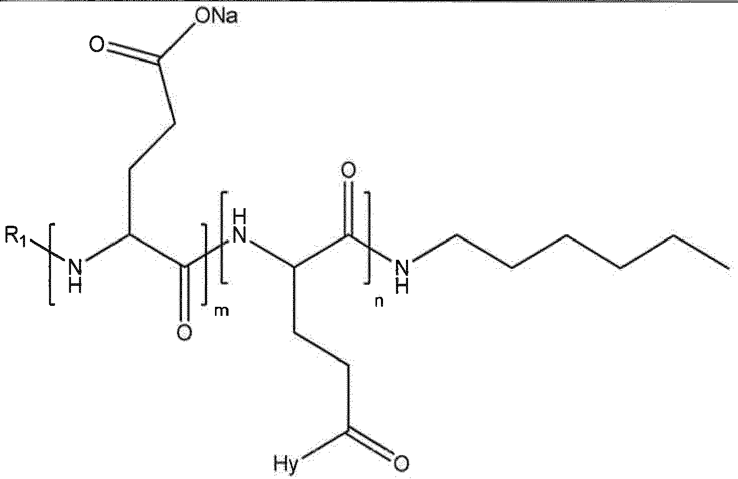 |
| | i = 0,08, DP = 25 |
| | 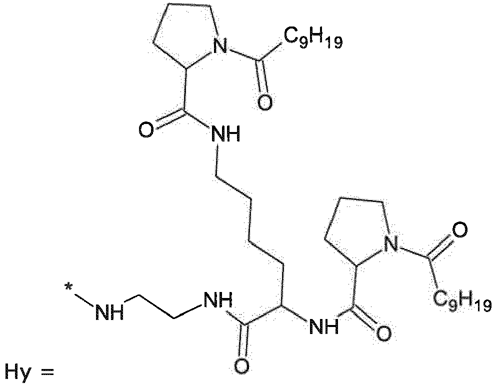 |
| | R₁ = H ou pyroglutamate |

### Co-polyaminoacides à greffage défini (formule VII et VIIb)

**Tableau 1e : liste de co-polyaminoacides synthétisés selon l'invention.**

| **N°** | **co-polyaminoacides porteur de charges carboxylates et de radicaux hydrophobes** |
|---|---|
| BB14 | 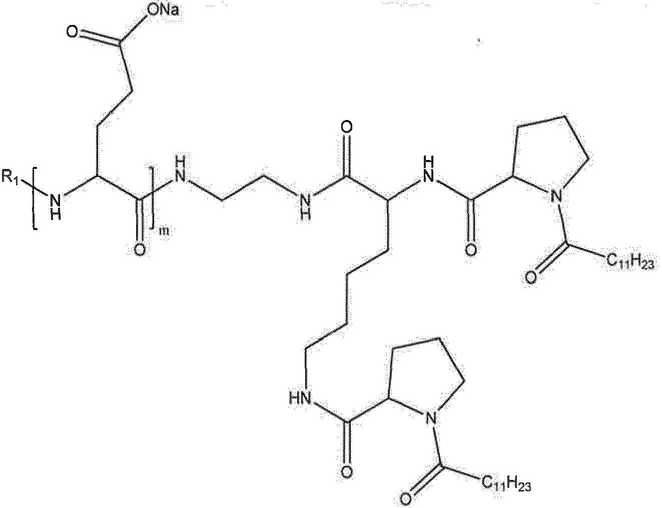 |
| | i = 0,034, DP (m) = 29 |
| | R₁ = H ou pyroglutamate |
| BB15 | 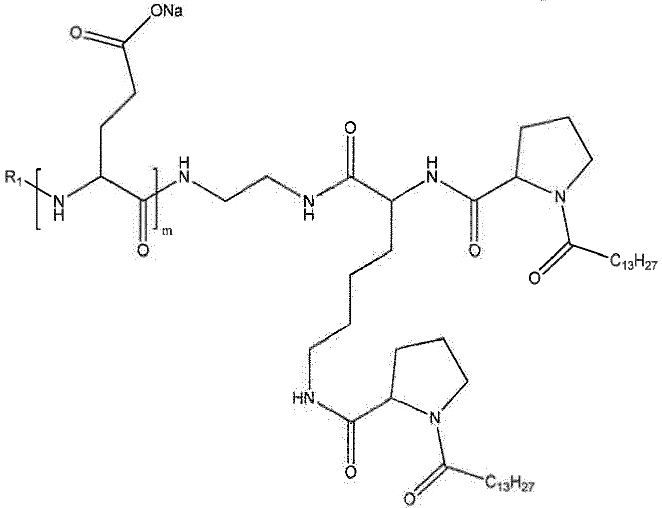 |
| | i = 0,042, DP (m) = 24 |
| | R₁ = H ou pyroglutamate |
| BB16 | 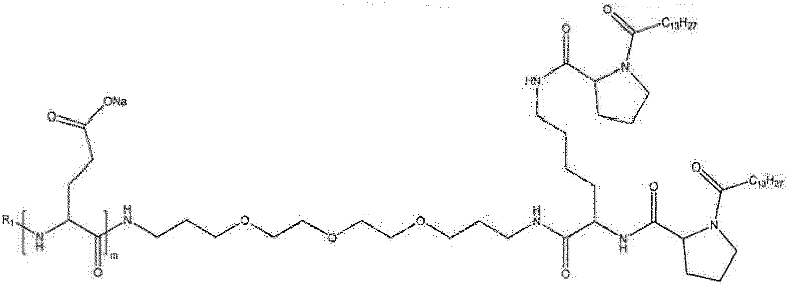 |
| | i = 0,043, DP (m) = 23 |
| | R₁ = H ou pyroglutamate |
| BB17 | 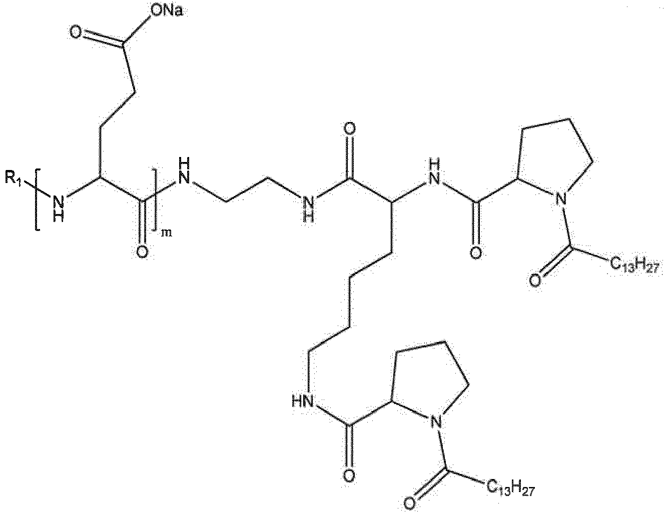 |
| | i = 0,015, DP (m) = 65 |
| | R₁ = H ou pyroglutamate |
| BB18 | 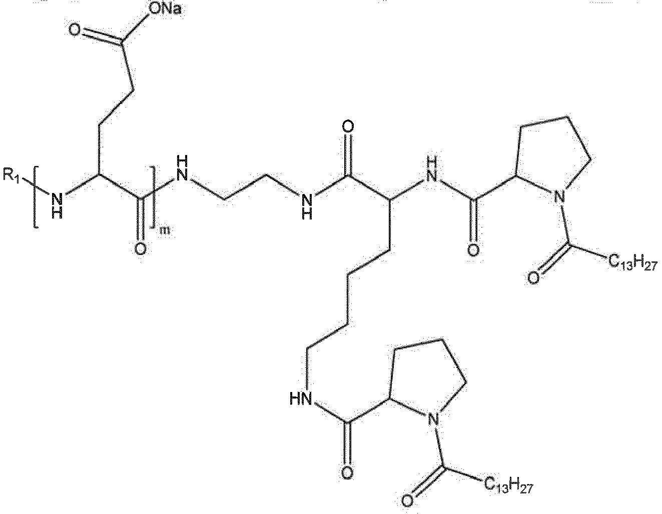 |
| | i = 0,025, DP (m) = 40 |
| | R₁= H ou pyroglutamate |
| **BB19** | 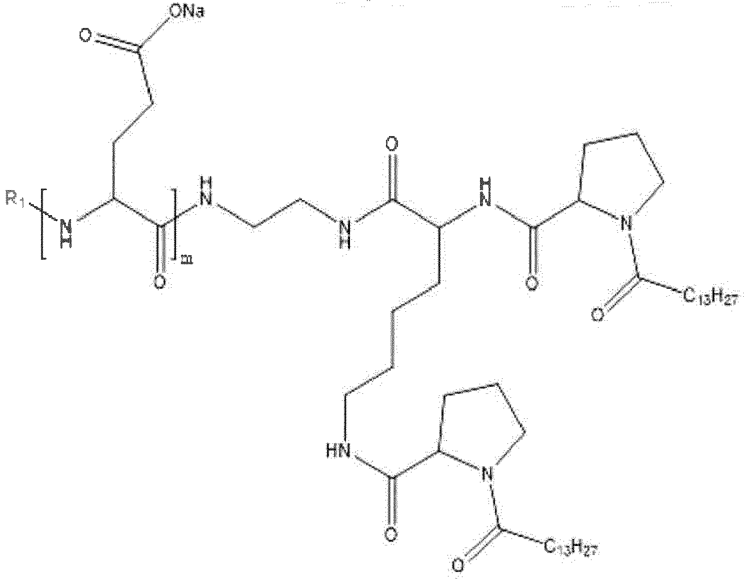 |
| | i = 0,04, DP (m) = 25 |
| | R₁ = H ou pyroglutamate |
| **BB20** | 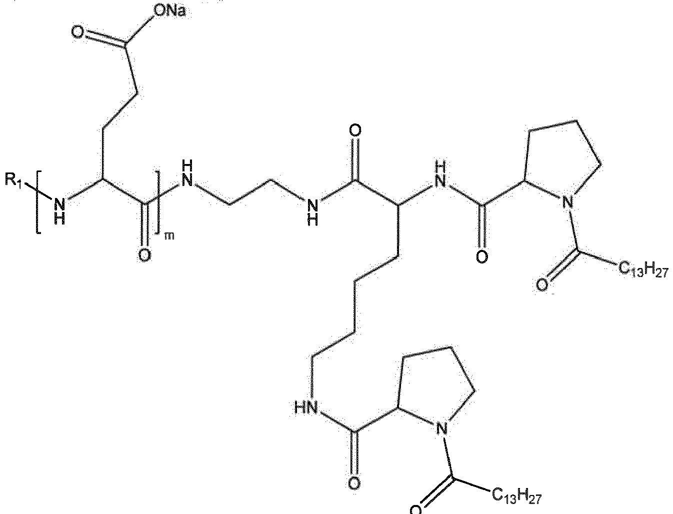 |
| | i = 0,059, DP (m) = 17 |
| | R₁ = H ou pyroglutamate |
| **BB21** | 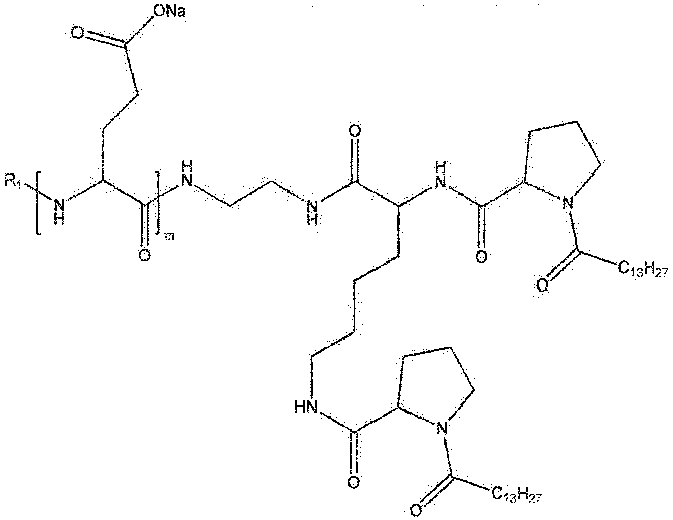 |
| | i = 0,11, DP (m) = 9 |
| | R₁ = H ou pyroglutamate |
| **BB22** | 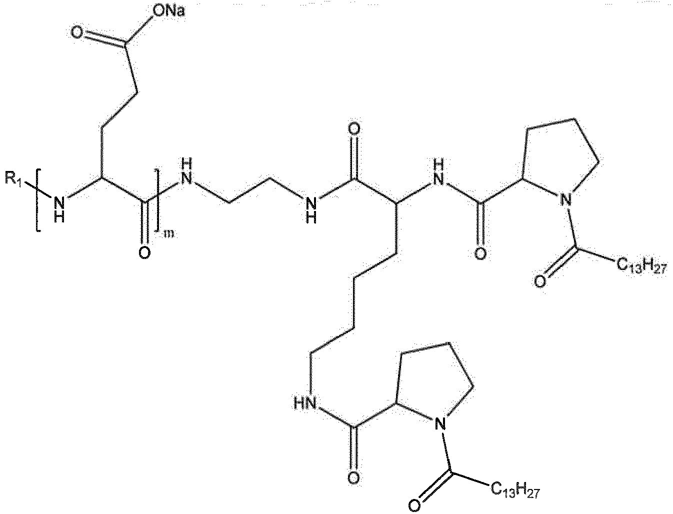 |
| | i = 0,048, DP (m) = 21 |
| | R₁ = H ou pyroglutamate |
| **BB23** | 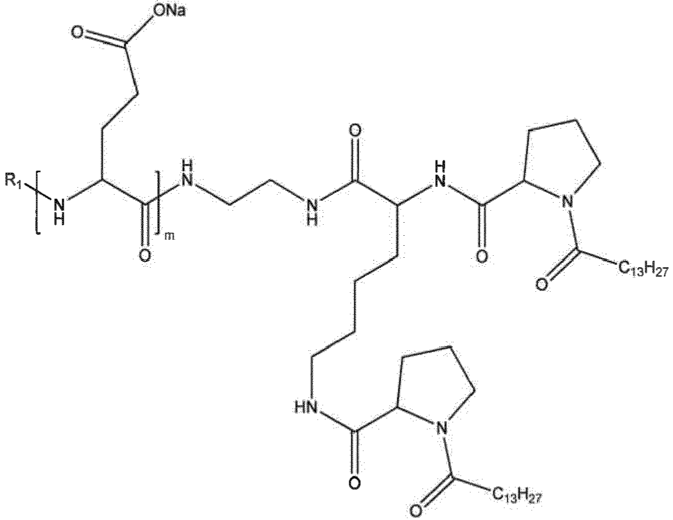 |
| | i = 0,048, DP (m) = 21 |
| | R₁ = H ou pyroglutamate |
| **BB24** | 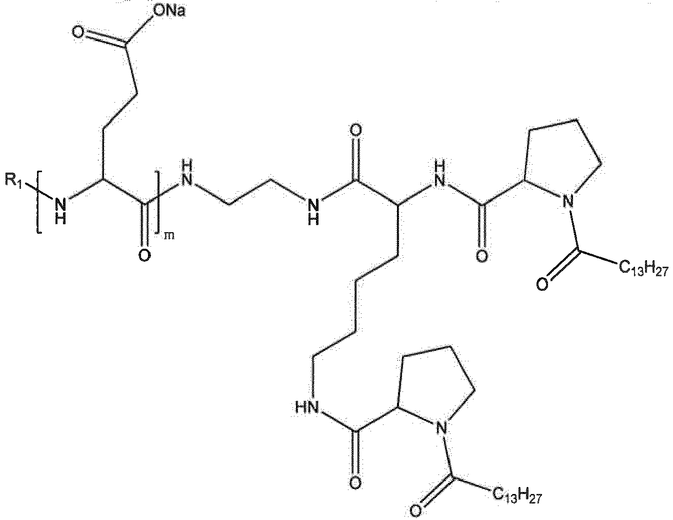 |
| | i = 0,040, DP (m) = 25 |
| | R₁ = H ou pyroglutamate |
| **BB25** | 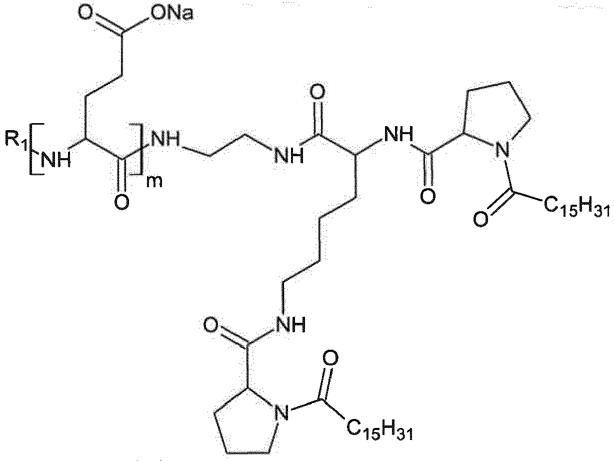 |
| | i = 0,043 , DP (m) = 23 |
| | R₁ = H ou pyroglutamate |
| **BB26** | 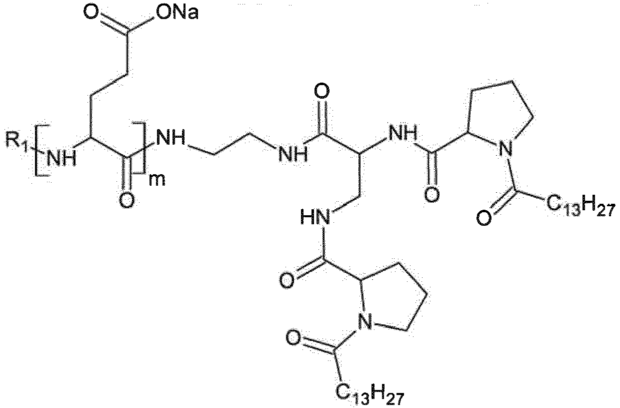 |
| | i = 0,048, DP (m) = 21 R₁ = H ou pyroglutamate |
| **BB42** | 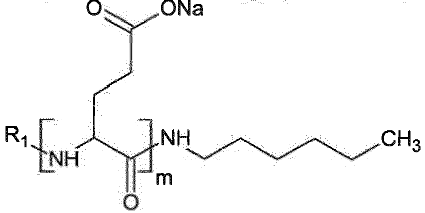 |
| | i = 0,045, DP (m) = 22 |
| | 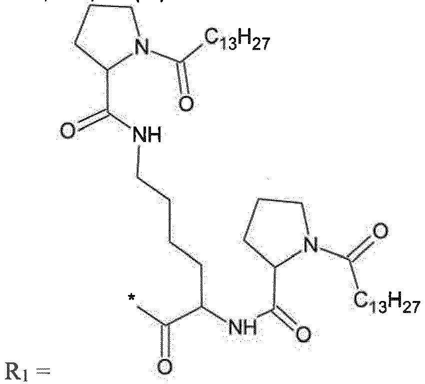 |
| **BB44** | 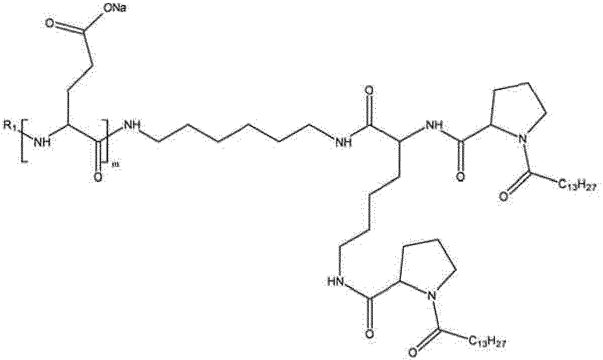 |
| | i = 0,04, DP (m) = 25 |
| | R₁ = H ou pyroglutamate |

### Exemple BB1 : Co-polyaminoacide BB1 - poly-L-glutamate de sodium modifié par la molécule BA2 et ayant une masse molaire moyenne en nombre (Mn) de 2400 g/mol

Co-polyaminoacide BB1-1 : acide poly-L-glutamique de masse molaire moyenne en nombre (Mn) relative 3860 g/mol issu de la polymérisation du γ-benzyl-L-glutamate N-carboxyanhydride initiée par l'hexylamine.

Dans un ballon préalablement séché à l'étuve est placé sous vide du γ-benzyl-L-glutamate N-carboxyanhydride (90,0 g, 342 mmol) pendant 30 min, puis du DMF anhydre (465 mL) est introduit. Le mélange est alors agité sous argon jusqu'à complète dissolution, refroidi à 4 °C, puis de l'hexylamine (1,8 mL, 14 mmol) est introduite rapidement. Le mélange est agité entre 4 °C et température ambiante pendant 2 jours. Le milieu réactionnel est ensuite chauffé à 65 °C pendant 4 h, refroidi à température ambiante puis coulé goutte à goutte dans du diisopropyléther froid (6 L) sous agitation. Le précipité blanc est récupéré par filtration, lavé avec du diisopropyléther (500 mL puis 250 mL) puis séché sous vide à 30 °C pour donner un acide poly(γ-benzyl-L-glutamique) (PBLG).

A une solution de PBLG (42,1 g) dans l'acide trifluoroacétique (TFA, 325 mL) à 4 °C est ajoutée goutte à goutte une solution d'acide bromhydrique (HBr) à 33% dans l'acide acétique (135 mL, 0,77 mol). Le mélange est agité à température ambiante pendant 2 h, puis coulé goutte à goutte sur un mélange 1:1 (v/v) de diisopropyléther et d'eau sous agitation (1,6 L). Après 1 h 30 d'agitation, le mélange hétérogène est laissé au repos pendant une nuit. Le précipité blanc est récupéré par filtration, lavé avec un mélange 1:1 (v/v) de diisopropyléther et d'eau (200 mL).

Le solide obtenu est alors solubilisé dans de l'eau (1 L) en ajustant le pH à 7 par ajout d'une solution aqueuse de soude 10 N puis une solution aqueuse de soude 1 N. Après solubilisation, la concentration théorique est ajustée à 25 g/L théorique par addition d'eau pour obtenir un volume final de 1,5 L.

La solution est filtrée sur filtre 0,45 µm puis purifiée par ultrafiltration contre une solution de NaCl 0,9 %, puis de l'eau jusqu'à ce que la conductimétrie du perméat soit inférieure à 50 µS/cm.

La solution aqueuse est alors acidifiée par ajout de solution d'acide chlorhydrique 37% jusqu'à atteindre un pH de 2. Après 4 h d'agitation, le précipité obtenu est filtré puis séché sous vide à 30 °C pour donner un acide poly-L-glutamique de masse molaire moyenne en nombre (Mn) 3860 g/mol par rapport à un standard de polyoxyéthylène (PEG).

### Co-polyaminoacide BB1

Le co-polyaminoacide BB1-1 (10,0 g) est solubilisé dans le DMF (700 mL) à 30-40 °C puis refroidi à 0 °C. Le sel de chlorhydrate de la molécule BA2 (2,95 g, 3,8 mmol) est mis en suspension dans du DMF (45 mL) et de la triéthylamine (0,39 g, 3,8 mmol) est ensuite ajoutée à cette suspension puis le mélange est légèrement chauffé sous agitation jusqu'à complète dissolution. A la solution de co-polyaminoacide à 0 °C, de la N-méthylmorpholine (NMM, 7,6 g, 75 mmol) dans le DMF (14 mL) et du chloroformate d'éthyle (ECF, 8,1 g, 75 mmol) sont ajoutés. Après 10 min à 0 °C, la solution de molécule BA2 est ajoutée et le milieu maintenu à 30 °C durant 1 h. Le milieu réactionnel est coulé goutte-à-goutte sur 6 L d'eau contenant du chlorure de sodium à 15% massique et du HCl (pH 2), puis laissé reposer une nuit. Le précipité est collecté par filtration, lavé par la solution de chlorure de sodium à pH 2 (1L) et séché sous vide pendant environ 1 h. Le solide blanc obtenu est repris dans de l'eau (600 mL) et le pH est ajusté à 7 par ajout lent d'une solution aqueuse de NaOH 1 N. Le volume est ajusté à 700 mL par ajout d'eau. Après filtration sur filtre 0,45 µm, la solution limpide obtenue est purifiée par ultrafiltration contre une solution de NaCl 0,9% puis de l'eau, jusqu'à ce que la conductimétrie du perméat soit inférieure à 50 µS/cm. Après déchargement, la solution est filtrée sur filtre 0,2 µm et stockée à 2-8 °C.

Extrait sec : 19,7 mg/g.

DP (estimé d'après la RMN ¹H) : 23.

D'après la RMN ¹H : i = 0,05.

La masse molaire moyenne calculée du co-polyaminoacide BB1 est de 4350 g/mol.

HPLC-SEC aqueuse (calibrant PEG) : Mn = 2400 g/mol.

### Exemple BB2 : co-polyaminoacide BB2 - poly-L-glutamate de sodium modifié par la molécule BA2 et ayant une mase molaire moyenne en nombre (Mn) de 4900 g/mol

Un acide poly-L-glutamique de masse molaire moyenne en nombre (Mn) 4100 g/mol (5,0 g) obtenu par un procédé similaire à celui utilisé pour la préparation du co-polyaminoacide BB1-1 est solubilisé dans le DMF (205 mL) à 30-40 °C puis maintenu à cette température. En parallèle, le sel de chlorhydrate de la molécule BA2 (1,44 g, 1,84 mmol) est mis en suspension dans du DMF (10 mL) et de la triéthylamine (0,19 g, 1,84 mmol) est ajoutée, puis le mélange est légèrement chauffé sous agitation jusqu'à complète dissolution. A la solution de co-polyaminoacide dans le DMF, de la NMM (3,7 g, 36,7 mmol), la solution de molécule BA2 puis de la N-oxyde de 2-hydroxypyridine (HOPO, 0,31 g, 2,76 mmol) sont ajoutées successivement. Le milieu réactionnel est alors refroidi à 0 °C, puis du EDC (0,53 g, 2,76 mmol) est ajouté et le milieu est remonté à température ambiante durant 3 h. Le milieu réactionnel est coulé au goutte-à-goutte sur 1,55 L d'eau contenant du NaCl à 15% massique et du HCl (pH 2) sous agitation. A la fin de l'ajout, le pH est réajusté à 2 avec une solution de HCl 1 N, et la suspension est laissée reposer une nuit. Le précipité est collecté par filtration, puis rincé par 100 mL d'eau. Le solide blanc obtenu est solubilisé dans 200 mL d'eau par ajout lent d'une solution aqueuse de NaOH 1 N jusqu'à pH 7 sous agitation, puis la solution est filtrée sur filtre 0,45 µm. La solution limpide obtenue est purifiée par ultrafiltration contre une solution de NaCl 0,9% puis de l'eau, jusqu'à ce que la conductimétrie du perméat soit inférieure à 50 µS/cm. La solution obtenue est filtrée sur filtre 0,2 µm et stockée à 2-8 °C.

Extrait sec : 16,3 mg/g.

DP (estimé d'après la RMN ¹H) : 21.

D'après la RMN ¹H : li = 0,047.

La masse molaire moyenne calculée du co-polyaminoacide BB2 est de 3932 g/mol.

HPLC-SEC aqueuse (calibrant PEG) : Mn = 4900 g/mol.

### Exemple BB3 : Co-polyaminoacide BB3 - poly-L-glutamate de sodium modifié par la molécule BA2 et ayant une masse molaire moyenne en nombre (Mn) de 6400 g/mol

Co-polyaminoacide BB3-1 : acide poly-L-glutamique de masse molaire moyenne en nombre (Mn) 17500 g/mol issu de la polymérisation du γ-méthyl-L-glutamate N-carboxyanhydride initiée par la L-leucinamide.

Un acide poly-L-glutamique de masse moyenne en nombre (Mn) 17500 g/mol relative à un standard polymétacrylate de méthyle (PMMA) est obtenu par polymérisation du γ-méthyl N-carboxyanhydride d'acide glutamique en utilisant la L-leucinamide comme initiateur et en effectuant une déprotection des esters méthyliques par utilisation d'une solution d'acide chlorhydrique à 37% selon le procédé décrit dans la demande de brevet FR-A-2 801 226.

Par un procédé similaire à celui utilisé pour la préparation du co-polyaminoacide BB2 appliqué au sel chlorhydrate de la molécule BA2 (3,23 g, 4,1 mmol) et au co-polyaminoacie BB3-1 (11 g), un poly-L-glutamate de sodium modifié par la molécule BA2 est obtenu.

Extrait sec : 27,5 mg/g.

DP (estimé d'après la RMN ¹H) : 34.

D'après la RMN ¹H : li = 0,049.

La masse molaire moyenne calculée du co-polyaminoacide BB3 est de 6405 g/mol.

HPLC-SEC aqueuse (calibrant PEG) : Mn = 6400 g/mol.

### Exemple BB4 : co-polyaminoacide BB4 - poly-L-glutamate de sodium modifié par la molécule BA2 et ayant une masse molaire moyenne en nombre (Mn) de 10500 g/mol

Par un procédé similaire à celui utilisé pour la préparation du co-polyaminoacide BB2 appliqué au sel de chlorhydrate de la molécule BA2 (5 g, 6,35 mmol) et à un acide poly-L-glutamique de masse molaire moyenne en nombre Mn = 10800 g / mol (21,7 g) obtenu par un procédé similaire à celui utilisé pour la préparation du co-polyaminoacide BB1-1, un poly-L-glutamate de sodium modifié par la molécule BA2 est obtenu.

Extrait sec : 28,2 mg/g.

DP (estimé d'après la RMN ¹H) : 65.

D'après la RMN ¹H : i = 0,04.

La masse molaire moyenne calculée du co-polyaminoacide BB4 est de 11721 g/mol.

HPLC-SEC aqueuse (calibrant PEG) : Mn = 10500 g/mol.

### Exemple BB5 : Co-polyaminoacide BB5 - poly-L-glutamate de sodium cappé à une de ses extrémités par un groupement acétyle et modifié par la molécule BA2 et ayant une masse molaire moyenne en nombre (Mn) de 3600 g/mol

Co-polyaminoacide BB5-1 : acide poly-L-glutamique de Mn 3700 g/mol issu de la polymérisation du γ-benzyl-L-glutamate N-carboxyanhydride initiée par l'hexylamine et cappé à une de ses extrémités par un groupement acétyle.

Dans un ballon préalablement séché à l'étuve est placé sous vide du γ-benzyl-L-glutamate N-carboxyanhydride (100,0 g, 380 mmol) pendant 30 minutes puis du DMF anhydre (250 mL) est introduit. Le mélange est alors agité sous argon jusqu'à complète dissolution, refroidi à 4 °C, puis de l'hexylamine (2,3 mL, 17 mmol) est introduite rapidement. Le mélange est agité entre 4 °C et température ambiante pendant 2 jours puis précipité dans du diisopropyléther (3,4 L). Le précipité est récupéré par filtration, lavé deux fois avec du diisopropyléther (225 mL) puis séché pour donner un solide blanc qui est dissout dans 450 mL de THF. A cette solution sont ajoutés successivement de la N,N-diisopropyléthylamine (DIPEA, 31 mL, 176 mmol) puis de l'anhydride acétique (17 mL, 176 mmol). Après une nuit d'agitation à température ambiante, la solution est versée lentement dans du diisopropyléther (3 L) sur une durée de 30 min et sous agitation. Après 1 h d'agitation, le précipité est filtré, lavé deux fois avec du diisopropyléther (200 mL) puis séché sous vide à 30 °C pour donner un acide poly(γ-benzyl-L-glutamique) cappé à une de ses extrémités par un groupement acétyle.

A une solution du co-polyaminoacide cappé (72 g) dans l'acide trifluoroacétique (TFA, 335 mL) à 4°C est ajoutée goutte à goutte une solution d'acide bromhydrique (HBr) à 33% dans l'acide acétique (235 mL, 1,34 mol). Le mélange est agité à température ambiante pendant 3 h 30, puis coulé goutte à goutte sur un mélange 1:1 (v/v) de diisopropyléther et d'eau sous agitation (4 L). Après 2 h d'agitation, le mélange hétérogène est laissé au repos pendant une nuit. Le précipité blanc est récupéré par filtration, lavé avec un mélange 1:1 (v/v) de diisopropyléther et d'eau (340 mL) puis avec de l'eau (340 mL). Le solide obtenu est alors solubilisé dans de l'eau (1,5 L) en ajustant le pH à 7 par ajout d'une solution aqueuse de soude 10 N puis une solution aqueuse de soude 1 N. Après solubilisation, la concentration théorique est ajustée à 20 g/L théorique par addition d'eau pour obtenir un volume final de 2,1 L. La solution est filtrée sur filtre 0,45 µm puis purifiée par ultrafiltration contre une solution de NaCl 0,9 %, puis de l'eau jusqu'à ce que la conductimétrie du perméat soit inférieure à 50 µS/cm. La solution de co-polyaminoacide est ensuite concentrée jusqu'à obtenir un volume final de 1,8 L. La solution aqueuse est alors acidifiée par ajout de solution d'acide chlorhydrique 37% jusqu'à atteindre un pH de 2. Après 4 h d'agitation, le précipité obtenu est filtré, lavé avec de l'eau (330 mL) puis séché sous vide à 30 °C pour donner un acide poly-L-glutamique de masse molaire moyenne en nombre (Mn) 3700 g/mol par rapport à un standard de polyoxyéthylène (PEG).

### Co-polyaminoacide BB5

Par un procédé similaire à celui utilisé pour la préparation du co-polyaminoacide BB2 appliqué au sel chlorhydrate de la molécule BA2 (6,92 g, 8,8 mmol) et au co-polyaminoacide BB5-1 (30,0 g), un poly-L-glutamate de sodium cappé à une de ses extrémités par un groupement acétyle et modifié par la molécule BA2 est obtenu.

Extrait sec : 29,4 mg/g.

DP (estimé d'après la RMN ¹H) : 23.

D'après la RMN ¹H : i = 0,042.

La masse molaire moyenne calculée du co-polyaminoacide BB5 est de 4302 g/mol.

HPLC-SEC aqueuse (calibrant PEG) : Mn = 3600 g/mol.

### Exemple BB6 : Co-polyaminoacide BB6 - poly-L-glutamate de sodium cappé à une de ses extrémités par un groupement acétyle et modifié par la molécule BA2 et ayant une masse molaire moyenne en nombre (Mn) de 4100 g/mol

Par un procédé similaire à celui utilisé pour la préparation du co-polyaminoacide BB2 appliqué au sel chlorhydrate de la molécule BA2 (5,8 g, 7,4 mmol) et à un acide poly-L-glutamique de masse molaire moyenne en nombre Mn = 3800 g / mol (25 g) obtenu par un procédé similaire à celui utilisé pour la préparation du co-polyaminoacide BB5-1 en utilisant l'ammoniac à la place de l'hexylamine, un poly-L-glutamate de sodium cappé à une de ses extrémités par un groupement acétyle et modifié par la molécule BA2 est obtenu.

Extrait sec : 27,6 mg/g.

DP (estimé d'après la RMN ¹H) : 24.

D'après la RMN ¹H : i = 0,04.

La masse molaire moyenne calculée du co-polyaminoacide BB6 est de 4387 g/mol.

HPLC-SEC aqueuse (calibrant PEG) : Mn = 4100 g/mol.

### Exemple BB7 : Co-polyaminoacide BB7 - poly-L-glutamate de sodium modifié par la molécule BA2 et ayant une masse molaire moyenne en nombre (Mn) de 4200 g/mol

Par un procédé similaire à celui utilisé pour la préparation du co-polyaminoacide BB2 appliqué au sel chlorhydrate de la molécule BA2 (7,07 g, 9,0 mmol) et à un acide poly-L-glutamique de masse molaire moyenne en nombre Mn = 3600 g / mol (30,0 g) obtenu par un procédé similaire à celui utilisé pour la préparation du co-polyaminoacide BB1-1, un poly-L-glutamate de sodium modifié par la molécule BA2 est obtenu.

Extrait sec : 28,3 mg/g.

DP (estimé d'après la RMN ¹H) : 22.

D'après la RMN ¹H : i = 0,042.

La masse molaire moyenne calculée du co-polyaminoacide BB7 est de 4039 g/mol.

HPLC-SEC aqueuse (calibrant PEG) : Mn = 4200 g/mol.

### Exemple BB8 : co-polyaminoacide BB8 - poly-L-glutamate de sodium modifié par la molécule BA2 et ayant une masse molaire moyenne en nombre (Mn) de 5200 g/mol

Par un procédé similaire à celui utilisé pour la préparation du co-polyaminoacide BB2 appliqué au sel chlorhydrate de la molécule BA2 (0,85 g, 1,1 mmol) et à un acide poly-L-glutamique de masse molaire moyenne en nombre Mn = 4100 g / mol (5,0 g) obtenu par un procédé similaire à celui utilisé pour la préparation du co-polyaminoacide BB1-1, un poly-L-glutamate de sodium modifié par la molécule BA2 est obtenu.

Extrait sec : 28,6 mg/g.

DP (estimé d'après la RMN ¹H) : 21.

D'après la RMN ¹H : i = 0,026.

La masse molaire moyenne calculée du co-polyaminoacide BB8 est de 3620 g/mol.

HPLC-SEC aqueuse (calibrant PEG) : Mn = 5200 g/mol.

### Exemple BB9 : co-polyaminoacide BB9 - poly-L-glutamate de sodium modifié par la molécule BA3 et ayant une masse molaire moyenne en nombre (Mn) de 4700 g/mol

Par un procédé similaire à celui utilisé pour la préparation du co-polyaminoacide BB2 appliqué au sel chlorhydrate de la molécule BA3 (3,05 g, 3,6 mmol) et à un acide poly-L-glutamique de masse molaire moyenne en nombre Mn = 4100 g / mol (10,0 g) obtenu par un procédé similaire à celui utilisé pour la préparation du co-polyaminoacide BB1-1, un poly-L-glutamate de sodium modifié par la molécule BA3 est obtenu.

Extrait sec : 28,6 mg/g.

DP (estimé d'après la RMN ¹H) : 26.

D'après la RMN ¹H : i = 0,05.

La masse molaire moyenne calculée du co-polyaminoacide BB9 est de 4982 g/mol.

HPLC-SEC aqueuse (calibrant PEG) : Mn = 4700 g/mol.

### Exemple BB10 : co-polyaminoacide BB10 - poly-L-glutamate de sodium modifié par la molécule BA3 et ayant une masse molaire moyenne en nombre (Mn) de 4200 g/mol

Par un procédé similaire à celui utilisé pour la préparation du co-polyaminoacide BB2 appliqué au sel chlorhydrate de la molécule BA3 (1,90 g, 2,3 mmol) et à un acide poly-L-glutamique de masse molaire moyenne en nombre Mn = 3500 g / mol (10,0 g) obtenu par un procédé similaire à celui utilisé pour la préparation du co-polyaminoacide BB1-1, un poly-L-glutamate de sodium modifié par la molécule BA3 est obtenu.

Extrait sec : 25,9 mg/g.

DP (estimé d'après la RMN ¹H) : 22.

D'après la RMN ¹H : i = 0,029.

La masse molaire moyenne calculée du co-polyaminoacide BB10 est de 3872 g/mol.

HPLC-SEC aqueuse (calibrant PEG) : Mn = 4200 g/mol.

### Exemple BB11 : co-polyaminoacide BB11 - poly-L-glutamate de sodium cappé à une de ses extrémités par un groupement acétyle et modifié par la molécule BA4 et ayant une masse molaire moyenne en nombre (Mn) de 3900 g/mol

Par un procédé similaire à celui utilisé pour la préparation du co-polyaminoacide BB2 appliqué au sel chlorhydrate de la molécule BA4 (2,21 g, 2,2 mmol) et à un acide poly-L-glutamique de masse moyenne en nombre Mn = 3700 g / mol (10 g) obtenu par un procédé similaire à celui utilisé pour la préparation du co-polyaminoacide BB5-1, un poly-L-glutamate de sodium cappé à une de ses extrémités par un groupement acétyle et modifié par la molécule BA4 est obtenu.

Extrait sec : 28,1 mg/g.

DP (estimé d'après la RMN ¹H) : 22.

D'après la RMN ¹H : i = 0,032.

La masse molaire moyenne calculée du co-polyaminoacide BB11 est de 4118 g/mol.

HPLC-SEC aqueuse (calibrant PEG) : Mn = 3900 g/mol.

### Exemple BB12 : co-polyaminoacide BB12 - poly-L-glutamate de sodium cappé à une de ses extrémités par un groupement acétyle et modifié par la molécule BA3 et ayant une masse molaire moyenne en nombre (Mn) de 3900 g/mol

Par un procédé similaire à celui utilisé pour la préparation du co-polyaminoacide BB2 appliqué au sel chlorhydrate de la molécule BA3 (1,9 g, 2,3 mmol) et à un acide poly-L-glutamique de masse moyenne en nombre Mn = 3600 g / mol (10 g) obtenu par un procédé similaire à celui utilisé pour la préparation du co-polyaminoacide BB5-1, un poly-L-glutamate de sodium cappé à une de ses extrémités par un groupement acétyle et modifié par la molécule BA3 est obtenu.

Extrait sec : 26,7 mg/g.

DP (estimé d'après la RMN ¹H) : 23.

D'après la RMN ¹H : i = 0,03.

La masse molaire moyenne calculée du co-polyaminoacide BB12 est de 4145 g/mol.

HPLC-SEC aqueuse (calibrant PEG) : Mn = 3900 g/mol.

### Exemple BB13 : co-polyaminoacide BB13 - poly-L-glutamate de sodium modifié par la molécule BA1 et ayant une masse molaire moyenne en nombre (Mn) de 2800 g/mol

Par un procédé similaire à celui utilisé pour la préparation du co-polyaminoacide BB1 appliqué au sel de chlorhydrate de la molécule BA1 (3,65 g, 5 mmol) et à un acide poly-L-glutamique de masse molaire moyenne en nombre Mn = 3600 g / mol (10 g) obtenu par un procédé similaire à celui utilisé pour la préparation du co-polyaminoacide BB1-1, un poly-L-glutamate de sodium modifié par la molécule BA1 est obtenu.

Extrait sec : 25,6 mg/g.

DP (estimé d'après la RMN ¹H) : 25.

D'après la RMN ¹H : i = 0,08.

La masse molaire moyenne calculée du co-polyaminoacide BB13 est de 5253 g/mol.

HPLC-SEC aqueuse (calibrant PEG) : Mn = 2800 g/mol.

### Exemple BB14 : co-polyaminoacide BB14 - poly-L-glutamate de sodium modifié à une de ses extrémités par la molécule BA2 et ayant une masse molaire moyenne en nombre (Mn) de 4020 g/mol

Dans un contenant adapté sont introduits successivement le sel de chlorhydrate de la molécule BA2 (2,12 g, 2,70 mmol), du chloroforme (40 mL), du tamis moléculaire 4Å (1,5 g), ainsi que de la résine échangeuse d'ion Amberlite IRN 150 (1,5 g). Après 1 h d'agitation sur rouleaux, le milieu est filtré et la résine est rincée avec du chloroforme. Le mélange est évaporé puis co-évaporé avec du toluène. Le résidu est solubilisé dans du DMF anhydre (20 mL) pour être utilisé directement dans la réaction de polymérisation.

Dans un ballon préalablement séché à l'étuve, du γ-benzyl-L-glutamate *N-*carboxyanhydride (18 g, 68,42 mmol) est placé sous vide pendant 30 min puis du DMF anhydre (100 mL) est introduit. Le mélange est agité sous argon jusqu'à solubilisation complète, refroidi à 4 °C, puis la solution de molécule BA2 préparée comme décrit précédemment est introduite rapidement. Le mélange est agité entre 4 °C et température ambiante pendant 2 jours, puis chauffé à 65 °C pendant 2 h. Le mélange réactionnel est alors refroidi à température ambiante puis versé goutte à goutte dans du diisopropyléther (1,2 L) sous agitation. Le précipité blanc est récupéré par filtration, lavé deux fois avec du diisopropyléther (100 mL) puis séché sous vide à 30 °C pour obtenir un solide blanc. Le solide est dilué dans du TFA (105 mL), et une solution d'acide bromhydrique (HBr) à 33% dans de l'acide acétique (38 mL, 220 mmol) est alors ajoutée goutte à goutte et à 0 °C. La solution est agitée pendant 2 h à température ambiante puis est coulée goutte à goutte sur un mélange 1:1 (v/v) de diisopropyléther / eau et sous agitation (600 mL). Après 2 h d'agitation, le mélange hétérogène est laissé au repos pendant une nuit. Le précipité blanc est récupéré par filtration, lavé successivement avec un mélange 1:1 (v/v) de diisopropyléther et d'eau (200 mL) puis avec de l'eau (100 mL). Le solide obtenu est solubilisé dans de l'eau (450 mL) en ajustant le pH à 7 par ajout d'une solution aqueuse de soude 10 N puis une solution aqueuse de soude 1 N. Le mélange est filtré sur filtre 0,45 µm puis est purifié par ultrafiltration contre une solution de NaCl 0,9 % puis de l'eau jusqu'à ce que la conductimétrie du perméat soit inférieure à 50 µS/cm. La solution de co-polyaminoacide est ensuite concentrée à environ 30 g/L théorique et le pH est ajusté à 7,0. La solution aqueuse est filtrée sur 0,2 µm et conservée à 4 °C.

Extrait sec : 22,3 mg/g.

DP (estimé par RMN ¹H = 29 donc i = 0,034.

La masse molaire moyenne calculée du co-polyaminoacide BB14 est de 5089 g/mol.

HPLC-SEC aqueuse (calibrant PEG) : Mn = 4020 g/mol.

### Exemple BB15 : co-polyaminoacide BB15 - poly-L-glutamate de sodium modifié à une de ses extrémités par la molécule BA3 et ayant une masse molaire moyenne en nombre (Mn) de 3610 g/mol

Par un procédé similaire à celui utilisé pour la préparation du co-polyaminoacide BB14 appliqué au sel chlorhydrate de la molécule BA3 (3,62 g, 4,32 mmol) et à 25,0 g (94,97 mmol) de γ-benzyl-L-glutamate N-carboxyanhydride, un poly-L-glutamate de sodium modifié à une de ses extrémités par la molécule BA3 est obtenu.

Extrait sec : 26,5 mg/g.

DP (estimé par RMN ¹H) = 24 donc i = 0,042.

La masse molaire moyenne calculée du co-polyaminoacide BB15 est de 4390 g/mol.

HPLC-SEC aqueuse (calibrant PEG) : Mn = 3610 g/mol.

### Exemple BB16 : co-polyaminoacide BB16 - poly-L-glutamate de sodium modifié à une de ses extrémités par la molécule BA4 et ayant une masse molaire moyenne en nombre (Mn) de 3300 g/mol

Par un procédé similaire à celui utilisé pour la préparation du co-polyaminoacide BB14 appliqué au sel chlorhydrate de la molécule BA4 (5,70 g, 5,70 mmol) et à 29,99 g (113,9 mmol) de γ-benzyl-L-glutamate N-carboxyanhydride, un poly-L-glutamate de sodium modifié à une de ses extrémités par la molécule BA4 est obtenu.

Extrait sec : 32,3 mg/g.

DP (estimé par RMN ¹H) = 23 donc i = 0,043.

La masse molaire moyenne calculée du co-polyaminoacide BB16 est de 4399 g/mol.

HPLC-SEC aqueuse (calibrant PEG) : Mn = 3300 g/mol.

### Exemple BB17 : co-polyaminoacide BB17 - poly-L-glutamate de sodium modifié à une de ses extrémités par la molécule BA3 et ayant une masse molaire moyenne en nombre de 10700 g/mol

Par un procédé similaire à celui utilisé pour la préparation du co-polyaminoacide BB14 appliqué au sel chlorhydrate de la molécule BA3 (2,51 g, 3 mmol) et à 52,7 g (200 mmol) de γ-benzyl-L-glutamate N-carboxyanhydride, un poly-L-glutamate de sodium modifié à une de ses extrémités par la molécule BA3 est obtenu.

Extrait sec : 24,5 mg/g.

DP (estimé par RMN ¹H) = 65 donc i = 0,015.

La masse molaire moyenne calculée du co-polyaminoacide BB17 est de 10585 g/mol.

HPLC-SEC aqueuse (calibrant PEG) : Mn = 10700 g/mol.

### Exemple BB18 : co-polyaminoacide BB18 - poly-L-glutamate de sodium modifié à une de ses extrémités par la molécule BA3 et ayant une masse molaire moyenne en nombre de 6600 g/mol

Par un procédé similaire à celui utilisé pour la préparation du co-polyaminoacide BB14 appliqué au sel chlorhydrate de la molécule BA3 (2,51 g, 3 mmol) et à 31,6 g (120 mmol) de γ-benzyl-L-glutamate N-carboxyanhydride, un poly-L-glutamate de sodium modifié à une de ses extrémités par la molécule BA3 est obtenu.

Extrait sec : 27,3 mg/g.

DP (estimé par RMN ¹H) = 40 donc i = 0,025.

La masse molaire moyenne calculée du co-polyaminoacide BB18 est de 6889 g/mol.

HPLC-SEC aqueuse (calibrant PEG) : Mn = 6600 g/mol.

### Exemple BB19 : co-polyaminoacide BB19 - poly-L-glutamate de sodium modifié à l'une de ses extrémités par la molécule BA3 et ayant une masse molaire moyenne en nombre (Mn) de 3400 g/mol

Par un procédé similaire à celui utilisé pour la préparation du co-polyaminoacide BB14 appliqué au sel chlorhydrate de la molécule BA3 (36,26 g, 43,2 mmol) et de γ-benzyl-L-glutamate N-carboxyanhydride (250,0 g, 949,7 mmol), un poly-L-glutamate de sodium modifié à une de ses extrémités par la molécule BA3 est obtenu.

Extrait sec : 22,4 mg/g

DP (estimé par RMN ¹H) = 25 donc i = 0,04

La masse molaire moyenne calculée du co-polyaminoacide BB19 est de 4540 g/mol.

HPLC-SEC aqueuse (calibrant PEG) : Mn = 3400 g/mol.

### Exemple BB20 : co-polyaminoacide BB20 - poly-L-glutamate de sodium modifié à l'une de ses extrémités par la molécule BA3 et ayant une masse molaire moyenne en nombre (Mn) de 2500 g/mol

Par un procédé similaire à celui utilisé pour la préparation du co-polyaminoacide BB14 appliqué à la molécule BA3 sous forme d'amine libre (1,017 g, 12,7 mmol) et de γ-benzyl-L-glutamate N-carboxyanhydride (5,0 g, 19,0 mmol), un poly-L-glutamate de sodium modifié à une de ses extrémités par la molécule BA3 est obtenu.

Extrait sec : 11,2 mg/g

DP (estimé par RMN ¹H) = 17 donc i = 0,059

La masse molaire moyenne calculée du co-polyaminoacide BB20 est de 3332 g/mol.

HPLC-SEC aqueuse (calibrant PEG) : Mn = 2500 g/mol.

### Exemple BB21 : co-polyaminoacide BB21 - poly-L-glutamate de sodium modifié à l'une de ses extrémités par la molécule BA3 et ayant une masse molaire moyenne en nombre (Mn) de 1100 g/mol

Par un procédé similaire à celui utilisé pour la préparation du co-polyaminoacide BB14 appliqué à la molécule BA3 sous forme d'amine libre (3,814 g, 4,75 mmol) et de γ-benzyl-L-glutamate N-carboxyanhydride (10,0 g, 38,0 mmol), un poly-L-glutamate de sodium modifié à une de ses extrémités par la molécule BA3 est obtenu.

Extrait sec : 16,1 mg/g

DP (estimé par RMN ¹H) = 9 donc i = 0,11

La masse molaire moyenne calculée du co-polyaminoacide BB21 est de 2123 g/mol.

HPLC-SEC aqueuse (calibrant PEG) : Mn = 1100 g/mol.

### Exemple BB22 : co-polyaminoacide BB22 - poly-D-glutamate de sodium modifié à l'une de ses extrémités par la molécule BA3 et ayant une masse molaire moyenne en nombre (Mn) de 2900 g/mol

Par un procédé similaire à celui utilisé pour la préparation du co-polyaminoacide BB14 appliqué à la molécule BA3 sous forme d'amine libre (2,77 g, 3,45 mmol) et de γ-benzyl-D-glutamate *N*-carboxyanhydride (20,0 g, 76,0 mmol), un poly-D-glutamate de sodium modifié à une de ses extrémités par la molécule BA3 est obtenu.

Extrait sec : 15,2 mg/g

DP (estimé par RMN ¹H = 21 donc i = 0,048

La masse molaire moyenne calculée du co-polyaminoacide BB22 est de 3936 g/mol.

HPLC-SEC aqueuse (calibrant PEG) : Mn = 2900 g/mol.

### Exemple BB23 : co-polyaminoacide BB23 - copolymère aléatoire d'unité D- ou L-glutamate de sodium modifié à l'une de ses extrémités par la molécule BA3 et ayant une masse molaire moyenne en nombre (Mn) de 2800 g/mol

Dans un ballon préalablement séché à l'étuve, du γ-benzyl-L-glutamate *N-*carboxyanhydride (20,0 g, 76,00 mmol) et du γ-benzyl-D-glutamate *N-*carboxyanhydride (20,0 g, 76,00 mmol) sont placés sous vide pendant 30 min puis du DMF anhydre (75 mL) est introduit. Le mélange est agité sous argon jusqu'à solubilisation complète, refroidi à 4 °C, puis une solution de molécule BA3 sous forme d'amine libre (5,55 g, 6,91 mmol) dans le chloroforme (14,5 mL) est introduite rapidement. Le mélange est agité entre 4 °C et température ambiante pendant 18 h, puis chauffé à 65 °C pendant 2 h. Le mélange réactionnel est alors refroidi à température ambiante puis versé goutte à goutte dans du diisopropyléther (1,2 L) sous agitation. Le précipité blanc est récupéré par filtration, lavé trois fois avec du diisopropyléther (80 mL) puis séché sous vide à 30 °C pour obtenir un solide blanc. Le solide est dilué dans du TFA (152 mL), et une solution d'acide bromhydrique (HBr) à 33% dans de l'acide acétique (106 mL, 220 mmol) est alors ajoutée goutte à goutte et à 0 °C. La solution est agitée pendant 3 h à température ambiante puis est coulée goutte à goutte sur un mélange 1:1 (v/v) de diisopropyléther / eau et sous agitation (1,84 L). La phase aqueuse est séparée dans une ampoule de coulée et le pH est ajusté à 7,2 par ajout d'une solution aqueuse de NaOH 10 N. Après ajout d'eau (250 mL), le mélange est filtré sur filtre 0,45 µm puis est purifié par ultrafiltration contre une solution de NaCl 0,9 % puis de l'eau jusqu'à ce que la conductimétrie du perméat soit inférieure à 50 µS/cm. La solution de co-polyaminoacide est ensuite concentrée à environ 25 g/L, filtrée sur 0,2 µm et conservée à 4 °C.

Extrait sec : 28,2 mg/g

DP (estimé par RMN ¹H) = 21 donc i = 0,048

La masse molaire moyenne calculée du co-polyaminoacide BB23 est de 3936 g/mol.

HPLC-SEC aqueuse (calibrant PEG) : Mn = 2800 g/mol.

### Exemple BB24 : co-polyaminoacide BB24 - copolymère à bloc de poly-D-glutamate et poly-L-glutamate de sodium modifié à l'une de ses extrémités par la molécule BA3 et ayant une masse molaire moyenne en nombre (Mn) de 2800 g/mol

Dans un ballon préalablement séché à l'étuve, du γ-benzyl-D-glutamate *N-*carboxyanhydride (13,5 g, 51,3 mmol) est placé sous vide pendant 30 min puis du DMF anhydre (52 mL) est introduit. Le mélange est agité sous argon jusqu'à solubilisation complète, refroidi à 0 °C, puis une solution de molécule BA3 sous forme d'amine libre (3,43 g, 4,27 mmol) dans du chloroforme (8,6 mL) est introduite rapidement. Le mélange est agité à 0 °C pendant 24 h, puis une solution de γ-*tert*-butyl-*L*-glutamate *N*-carboxyanhydride (13,5 g, 58,9 mmol) dans le DMF (15 mL) est ajoutée. Le mélange est alors agité entre 0°C et température ambiante pendant 21 h, puis chauffé à 65 °C pendant 2 h. Le mélange réactionnel est alors refroidi à température ambiante puis versé goutte à goutte dans du diisopropyléther (0,8 L) sous agitation. Le précipité blanc est récupéré par filtration, lavé trois fois avec du diisopropyléther (52 mL) puis séché sous vide à 30 °C pour obtenir un solide blanc. Le solide est dilué dans du TFA (96 mL), et une solution d'acide bromhydrique (HBr) à 33% dans de l'acide acétique (68 mL, 388 mmol) est alors ajoutée goutte à goutte et à 0 °C. La solution est agitée pendant 2 h à température ambiante puis est coulée goutte à goutte sur un mélange 1:1 (v/v) de diisopropyléther / eau et sous agitation (1,2 L). Après 2 h d'agitation, le mélange hétérogène est laissé au repos pendant une nuit. Le précipité blanc est récupéré par filtration, lavé successivement avec un mélange 1:1 (v/v) de diisopropyléther et d'eau (100 mL) puis avec de l'eau (100 mL). Le solide obtenu est solubilisé dans de l'eau (900 mL) en ajustant le pH à 7 par ajout d'une solution aqueuse de soude 10 N puis une solution aqueuse de soude 1 N. Le mélange est filtré sur filtre 0,45 µm puis est purifié par ultrafiltration contre une solution de NaCl 0,9 % puis de l'eau jusqu'à ce que la conductimétrie du perméat soit inférieure à 50 µS/cm. La solution de co-polyaminoacide est ensuite concentrée à environ 20 g/L théorique et le pH est ajusté à 7,0. La solution aqueuse est filtrée sur 0,2 µm et conservée à 4 °C.

Extrait sec : 23,9 mg/g

DP (estimé par RMN ¹H) = 25 donc i = 0,04

La masse molaire moyenne calculée du co-polyaminoacide BB24 est de 4541 g/mol.

HPLC-SEC aqueuse (calibrant PEG) : Mn = 2800 g/mol.

### Exemple BB25 : co-polyaminoacide BB25 - poly-L-glutamate de sodium modifié à l'une de ses extrémités par la molécule BA5 et ayant une masse molaire moyenne en nombre (Mn) de 2800 g/mol

Par un procédé similaire à celui utilisé pour la préparation du co-polyaminoacide BB14 appliqué à la molécule BA5 sous forme d'amine libre (1,70 g, 1,98 mmol) et de γ-benzyl-L-glutamate *N*-carboxyanhydride (11,46 g, 43,5 mmol), un poly-L-glutamate de sodium modifié à une de ses extrémités par la molécule BA5 est obtenu.

Extrait sec : 19,8 mg/g

DP (estimé par RMN ¹H) = 23 donc i = 0,043

La masse molaire moyenne calculée du co-polyaminoacide BB25 est de 4295 g/mol.

HPLC-SEC aqueuse (calibrant PEG) : Mn = 2800 g/mol.

### Exemple BB26 : co-polyaminoacide BB26 - poly-L-glutamate de sodium modifié à l'une de ses extrémités par la molécule BA6 et ayant une masse molaire moyenne en nombre (Mn) de 2900 g/mol

Par un procédé similaire à celui utilisé pour la préparation du co-polyaminoacide BB14 appliqué à la molécule BA6 sous forme d'amine libre (3,05 g, 4,01 mmol) et de γ-benzyl-L-glutamate *N*-carboxyanhydride (22,78 g, 86,5 mmol), un poly-L-glutamate de sodium modifié à une de ses extrémités par la molécule BA6 est obtenu.

Extrait sec : 16,9 mg/g

DP (estimé par RMN ¹H) = 21 donc i = 0,048

La masse molaire moyenne calculée du co-polyaminoacide BB26 est de 3894 g/mol.

HPLC-SEC aqueuse (calibrant PEG) : Mn = 2900 g/mol.

### Exemple BB42 : co-polyaminoacide BB42 - poly-L-glutamate de sodium modifié à l'une de ses extrémités par la molécule B8 et ayant une masse molaire moyenne en nombre (Mn) de 3200 g/mol

A une solution de molécule B8 (2,366 g, 3,11 mmol) dans le DMF (19,5 mL) sont introduits du DCC (0,659 g, 3,19 mmol) et du NHS (0,365 g, 3,17 mmol). Après 16 h d'agitation à température ambiante, la solution est filtrée pour être utilisée directement dans la réaction suivante.

Dans un ballon préalablement séché à l'étuve, du γ-benzyl-L-glutamate N-carboxyanhydride (18,0 g, 68,4 mmol) est placé sous vide pendant 30 min puis du DMF anhydre (40 mL) est introduit. Le mélange est alors agité sous argon jusqu'à complète dissolution, refroidi à 0°C, puis de l'hexylamine (0,411 mL, 3,11 mmol) est introduit rapidement. Après 30 h d'agitation à 0 °C, la solution de molécule B8 préparée précédemment est additionnée. La solution est agitée entre 0°C et température ambiante pendant 72 h puis coulée goutte à goutte dans du diisopropyléther (0,9 L) sous agitation. Le précipité est récupéré par filtration, lavé avec du diisopropyléther (5 fois 100 mL) puis séché sous vide à 30 °C pour donner un solide blanc. Le solide est dilué dans du TFA (69 mL), puis la solution est refroidie à 4 °C. Une solution de HBr à 33% dans l'acide acétique (48 mL, 0,274 mol) est alors ajoutée goutte à goutte. Le mélange est agité à température ambiante pendant 2 h, puis coulé goutte à goutte sur un mélange 1:1 (v/v) de diisopropyléther et d'eau sous agitation (0,8 L). Après 2 h d'agitation, le mélange hétérogène est laissé au repos pendant une nuit. Le précipité blanc est récupéré par filtration, lavé avec un mélange 1:1 (v/v) de diisopropyléther et d'eau (70 mL) puis avec de l'eau (70 mL). Le solide obtenu est alors solubilisé dans de l'eau (0,42 L) en ajustant le pH à 7 par ajout d'une solution aqueuse de soude 10 N puis une solution aqueuse de soude 1 N. Après solubilisation, la concentration théorique est ajustée à 20 g/L théorique par addition d'eau pour obtenir un volume final de 0,63 L. La solution est filtrée sur filtre 0,45 µm puis purifiée par ultrafiltration contre une solution de NaCl 0,9 %, puis de l'eau jusqu'à ce que la conductimétrie du perméat soit inférieure à 50 µS/cm. La solution obtenue est filtrée sur filtre 0,2 µm et stockée à 2-8 °C.

Extrait sec : 22,2 mg/g

DP (estimé d'après la RMN ¹H) : 22

D'après la RMN ¹H : i = 0,045

La masse molaire moyenne calculée du co-polyaminoacide BB42 est de 4160 g/mol.

HPLC-SEC aqueuse (calibrant PEG) : Mn = 3200 g/mol.

### Exemple BB44 : co-polyaminoacide BB44 - poly-L-glutamate de sodium modifié à l'une de ses extrémités par la molécule BA7 et ayant une masse molaire moyenne en nombre (Mn) de 3300 g/mol

Par un procédé similaire à celui utilisé pour la préparation du co-polyaminoacide BB14 appliqué à la molécule BA7 sous forme d'amine libre (4,45 g, 5,18 mmol) et à 30,0 g (113,96 mmol) de γ-benzyl-L-glutamate N-carboxyanhydride, un poly-L-glutamate de sodium modifié à une de ses extrémités par la molécule BA7 est obtenu.

Extrait sec : 29,0 mg/g

DP (estimé par RMN ¹H) = 25 donc i = 0,04

La masse molaire moyenne calculée du co-polyaminoacide BB44 est de 4597 g/mol.

HPLC-SEC aqueuse (calibrant PEG) : Mn = 3300 g/mol.

### Partie CE: co-polyaminoacides contre-exemples

### CEA : Synthèse des molécules hydrophobes contre-exemples

**Tableau if : molécules hydrophobes contre-exemples**

| | |
|---|---|
| CEA1 | 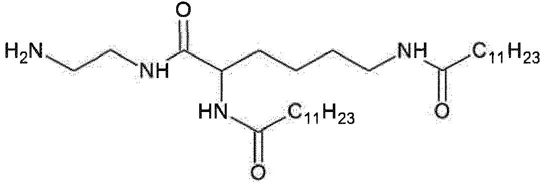 |
| CEA2 | 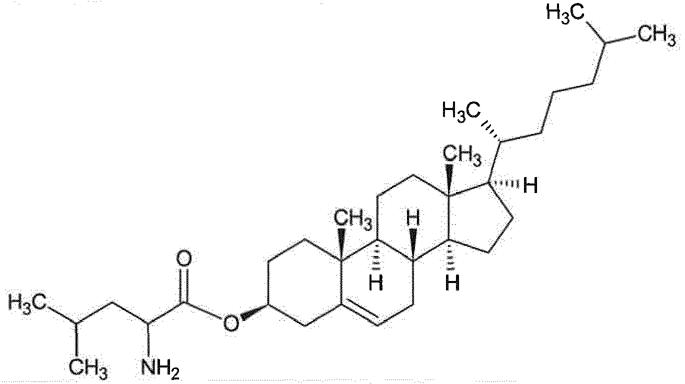 |
| CEA3 | 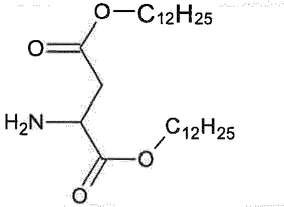 |
| CEA4 | 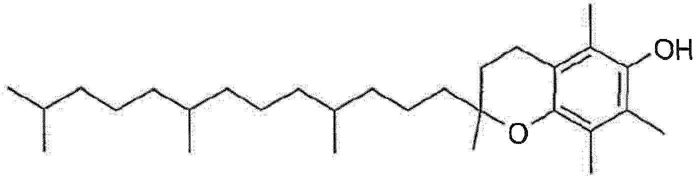 |

### Exemple CEA1 : molécule CEA1

### Molécule CE1 : produit obtenu par la réaction entre l'acide laurique et la L-lysine.

Par un procédé similaire à celui utilisé pour la préparation de la molécule B1 appliqué à l'acide laurique (19,58 g, 97,72 mmol) et à la L-lysine (7,5 g, 51,3 mmol), un solide beige est obtenu.

Rendement : 18,8 g (75%)

RMN ¹H (DMSO-d6, ppm) : 0,85 (6H) ; 1,12-1,78 (42H) ; 1,96-2,16 (4H) ; 2,92-3,07 (2H) ; 4,06-4,20 (1H) ; 7,70 (1H) ; 7,95 (1H) ; 12,40 (1H).

LC/MS (ESI): 511,5 ; (calculé ([M+H]⁺): 511,8).

### Molécule CE2 : produit obtenu par la réaction entre la molécule CE1 et la Boc-éthylènediamine.

Par un procédé similaire à celui utilisé pour la préparation de la molécule B3 appliqué à la molécule CE1 (18,70 g, 36,61 mmol), le résidu obtenu après concentration sous pression réduite du milieu réactionnel est trituré dans un mélange acétonitrile/THF (1/1 vol.), filtré et lavé à l'acétonitrile. Après séchage, un solide jaune pâle est obtenu.

Rendement : 13,7 g (57%)

RMN ¹H (CDCl₃, ppm) : 0,87 (6H) ; 1,09-1,89 (51H) ; 2,08-2,31 (4H) ; 3,13-3,48 (6H) ; 4,35 (1H) ; 5,20 (1H) ; 5,85 (1H) ; 6,46 (1H) ; 6,94 (1H).LC/MS (ESI): 653,5 ; (calculé ([M+H]⁺): 654,0).

### Molécule CEA1

Après un procédé similaire à celui utilisé pour la préparation de la molécule BA1 appliqué à la molécule CE2 (22,2 g, 34 mmol), le résidu obtenu après concentration sous vide est recristallisé dans le méthanol à chaud. Après refroidissement à température ambiante, le solide est filtré, lavé avec du méthanol froid puis de l'acétone et séché sous vide pour donner un solide jaune pâle.

Rendement : 16,3 g (81%).

RMN ¹H (MeOD, ppm) : 0,90 (6H) ; 1,20-1,85 (42H) ; 2,15-2,30 (4H) ; 3,06 (2H) ; 3,19 (2H) ; 3,40 (1H) ; 3,55 (1H) ; 4,13 (1H).

LC/MS (ESI): 553,5 ; (calculé ([M+H]⁺): 553,9).

### Molécule CEA2 et Molécule CEA3

Les molécules CEA2 et CEA3 ont été synthétisées selon le protocole décrit dans le brevet U.S. Pat. No. 4,826,818 (Kenji M. et Al.).

### Partie CEB : synthèse des co-polyaminoacides contre-exemples

**Tableau 1g : contre-exemples co-polyaminoacides**

| | |
|---|---|
| CEB1 | 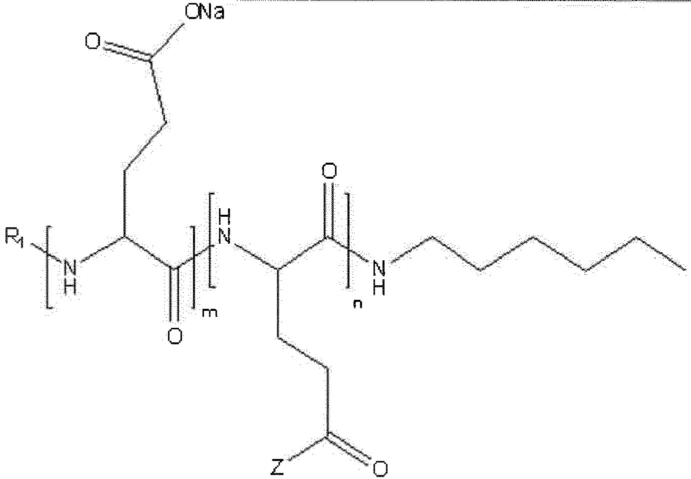 |
| | i = 0,024, DP (m + n) = 42 |
| | 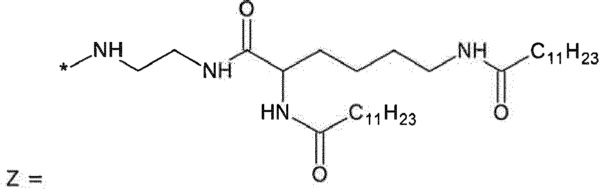 |
| | R₁ = H ou pyroglutamate |
| CEB2 | 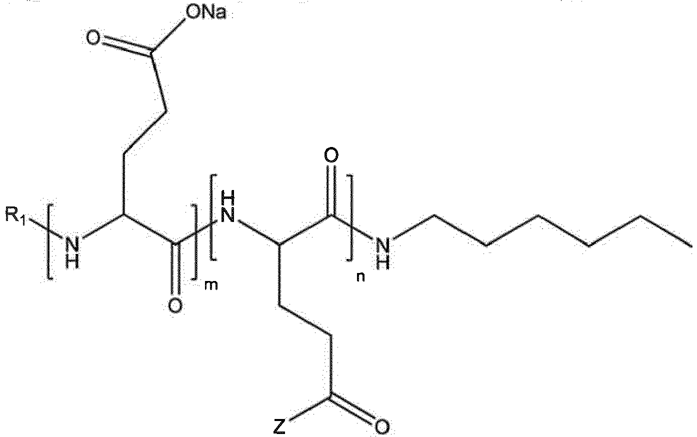 |
| | i = 0,05, DP (m + n) = 22 |
| | 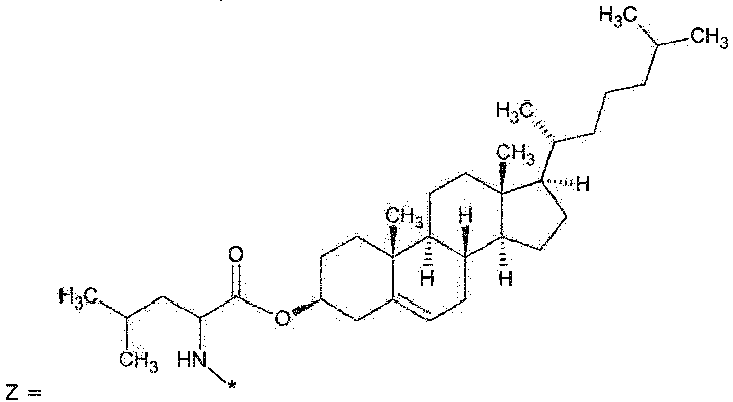 |
| | R₁ = CH₃-CO-, -H, ou pyroglutamate |
| CEB3 | 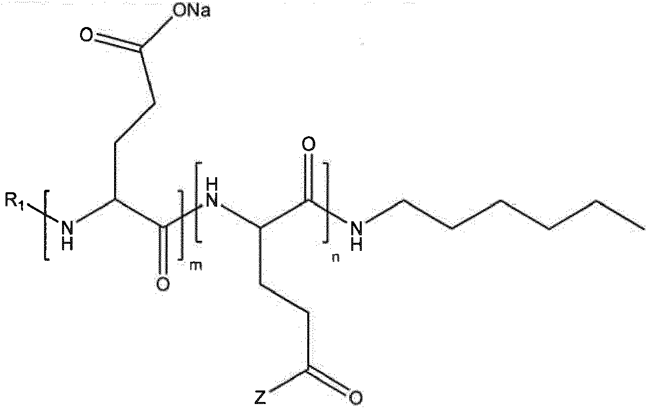 |
| | i = 0,05, DP (m + n) = 43 |
| | 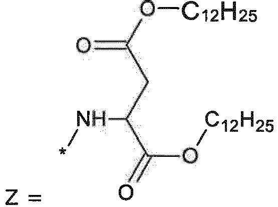 |
| | R₁ = CH₃-CO-, H ou pyroglutamate |
| CEB4 | 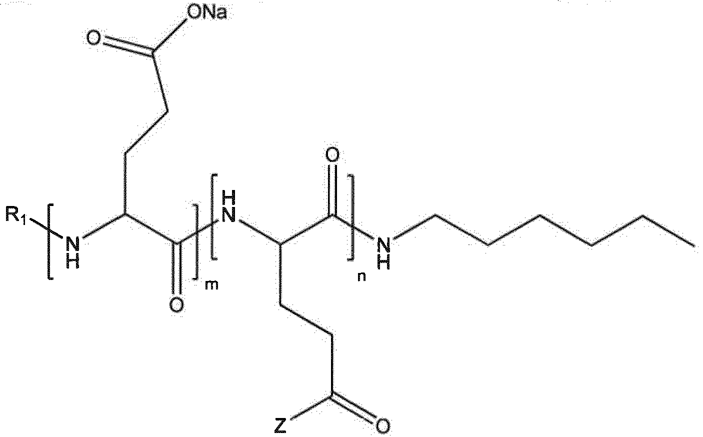 |
| | i = 0,05, DP (m + n) = 20 |
| | 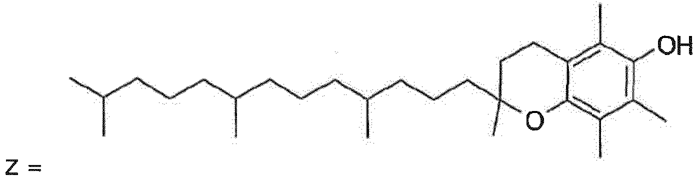 |
| | R₁ = H ou pyroglutamate |
| CEBX4 | 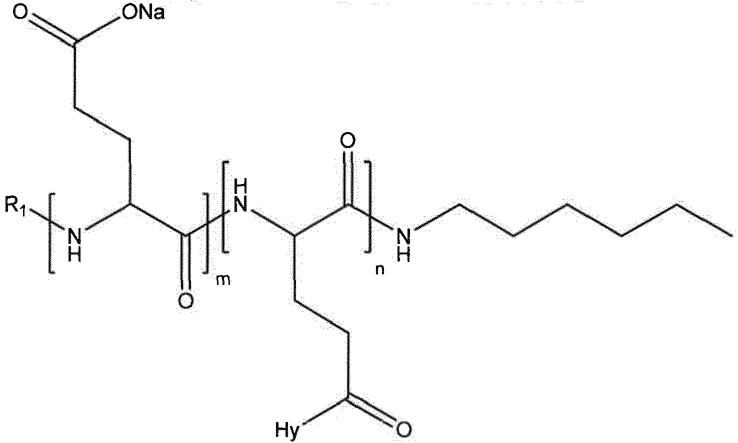 |
| | i = 0,042, DP (m + n) = 22 |
| | 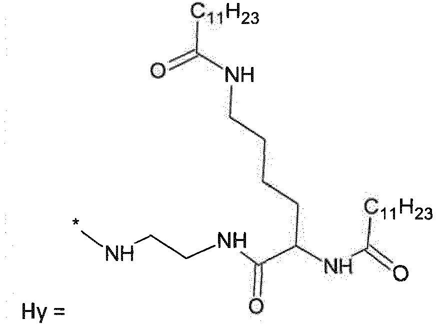 |
| | R₁ = CH₃-CO-, H ou pyroglutamate |

### Exemple CEB1 : co-polyaminoacide CEB1 - poly-L-glutamate de sodium modifié par la molécule CEA1 et ayant une masse molaire moyenne en nombre de 3800 g/mol

Un acide poly-L-glutamique de masse molaire moyenne en nombre (Mn) 8500 g/mol (2,0 g) obtenu par un procédé similaire à celui utilisé pour la préparation du co-polyaminoacide BB1-1 est solubilisé dans le DMF (27 mL) à 30-40 °C puis la solution est refroidie à 0°C. A cette solution, une solution de EDC (0,59 g, 3,06 mmol) dans le DMF (6 mL), une solution de HOBt (0,41 g, 3,06 mmol) dans le DMF (1 mL) et de la DIPEA (1,76 mL, 18,3 mmol) sont ajoutées. Une solution de molécule CEA1 (0,21 g, 1,84 mmol) dans le DMF (0,9 mL) est ensuite ajoutée et le mélange est agité à 25°C pendant 3 h. Le milieu réactionnel est dilué avec 11 mL d'eau ajoutée au goutte-à-goutte puis purifié par dialyse contre une solution aqueuse de NaCl à 0,9% puis de l'eau. La solution obtenue est filtrée sur filtre 0,2 µm et stockée à 2-8 °C.

Extrait sec : 16,3 mg/g.

DP (estimé d'après la RMN ¹H) : 42.

D'après la RMN ¹H : i = 0,024.

La masse molaire moyenne calculée du co-polyaminoacide CEB1 est de 6924 g/mol.

HPLC-SEC aqueuse (calibrant PEG) : Mn = 3800 g/mol.

### Exemple CEB2 : co-polyaminoacide CEB2 - poly-L-glutamate de sodium cappé à une de ses extrémités par un groupement acétyle et modifié par le leucinate de cholestéryle et ayant une masse molaire moyenne en nombre de 2575 g/mol

Le sel d'acide para-toluènesulfonique du leucinate de cholestéryle est préparé selon le procédé décrit dans le brevet U.S. Pat. No. 4,826,818 (Kenji, M. et al.).

Par un procédé similaire à celui utilisé pour la préparation du co-polyaminoacide BB13 appliqué au sel d'acide para-toluènesulfonique du leucinate de cholestéryle (1,23 g, 1,8 mmol) et à un acide poly-L-glutamique de masse moyenne en nombre Mn = 3600 g / mol (5 g) obtenu par un procédé similaire à celui utilisé pour la préparation du co-polyaminoacide BB5-1, un poly-L-glutamate de sodium cappé à une de ses extrémités par un groupement acétyle et modifié par le leucinate de cholestéryle est obtenu.

Extrait sec : 15,4 mg/g.

DP (estimé par RMN ¹H) = 22.

D'après la RMN ¹H : i = 0,05.

La masse molaire moyenne calculée du co-polyaminoacide CEB2 est de 3973 g/mol.

HPLC-SEC aqueuse (calibrant PEG) : Mn = 2575 g/mol.

### Exemple CEB3 : co-polyaminoacide CEB3 - poly-L-glutamate de sodium cappé à une de ses extrémités par un groupement acétyle et modifié par l'aspartate de dilauryle et ayant une masse molaire moyenne en nombre de 5820 g/mol

Le sel d'acide para-toluènesulfonique du l'aspartate de dilauryle est préparé selon le procédé décrit dans le brevet U.S. Pat. No. 4,826,818 (Kenji, M. et al.).

Par un procédé similaire à celui utilisé pour la préparation du co-polyaminoacide BB13 appliqué au sel d'acide para-toluènesulfonique du l'aspartate de dilauryle (0,89 g, 1,9 mmol) et à un acide poly-L-glutamique de masse moyenne en nombre Mn = 8500 g / mol (5 g) obtenu par un procédé similaire à celui utilisé pour la préparation du co-polyaminoacide BB5-1, un poly-L-glutamate de sodium cappé à une de ses extrémités par un groupement acétyle et modifié par l'aspartate de dilauryle est obtenu.

Extrait sec : 19,3 mg/g.

DP (estimé par RMN ¹H) = 43.

D'après la RMN ¹H : i = 0,05.

La masse molaire moyenne calculée du co-polyaminoacide CEB3 est de 7565 g/mol.

HPLC-SEC aqueuse (calibrant PEG) : Mn = 5820 g/mol.

### Exemple CEB4 : co-polyaminoacide CEB4 - poly-L-glutamate de sodium modifié par l'(±)-alpha-tocophérol et ayant une masse molaire moyenne en nombre de 5085 g/mol

Par un procédé similaire à celui décrit dans le brevet FR 2,840,614 (Ping, C. U. et al.) appliqué à l'(±)-alpha-tocophérol (0,5 g, 1,16 mmol) et à un acide poly-L-glutamique (3 g) obtenu par un procédé similaire à celui décrit dans le brevet FR2985429A1, un poly-L-glutamate de sodium modifié par l'(±)-alpha-tocophérol est obtenu.

Extrait sec : 10,9 mg/g.

DP (estimé par RMN ¹H) = 20.

D'après la RMN ¹H : i = 0,05.

La masse molaire moyenne calculée du co-polyaminoacide CEB4 est de 3473 g/mol.

HPLC-SEC aqueuse (calibrant PEG) : Mn = 5085 g/mol.

### Exemple CEBX4 : co-polyaminoacide CEBX4 - poly-L-glutamate de sodium cappé à une de ses extrémités par un groupement acétyle et modifié par la molécule CEA1 et ayant une masse molaire moyenne en nombre (Mn) de 3300 g/mol

Par un procédé similaire à celui utilisé pour la préparation du co-polyaminoacide BB2 appliqué au sel chlorhydrate de la molécule CEA1 (0,918 g, 1,56 mmol) et à un acide poly-L-glutamique de masse moyenne en nombre Mn = 3700 g / mol (5,0 g) obtenu par un procédé similaire à celui utilisé pour la préparation du co-polyaminoacide BB5-1, un poly-L-glutamate de sodium cappé à une de ses extrémités par un groupement acétyle et modifié par la molécule CEA1 est obtenu.

Extrait sec : 17,4 mg/g

DP (estimé d'après la RMN ¹H) : 22

D'après la RMN ¹H : i = 0,042

La masse molaire moyenne calculée du co-polyaminoacide CEBX4 est de 3941 g/mol.

HPLC-SEC aqueuse (calibrant PEG) : Mn = 3300 g/mol.

### Partie C - Compositions insuline glargine, insuline prandiale ou GLP-1 RA

### Exemple C1 : Solution d'insuline analogue rapide (Humalog®) à 100 U/mL

Cette solution est une solution commerciale d'insuline lispro commercialisée par la société ELI LILLY sous le nom de Humalog®. Ce produit est une insuline analogue rapide. Les excipients dans Humalog® sont le méta-crésol (3,15 mg/mL), le glycérol (16 mg/mL), le phosphate de disodium (1,88 mg/mL), l'oxide de zinc (pour avoir 0,0197 mg d'ion zinc/mL), l'hydroxyde de sodium et l'acide chlorhydrique pour l'ajustement du pH (pH 7-7,8) et de l'eau.

### Exemple C2 : Solution d'insuline analogue rapide (NovoLog®) à 100 U/mL

Cette solution est une solution commerciale d'insuline aspart commercialisée par la société NOVO NORDISK sous le nom de NovoLog® aux Etats-Unis d'Amérique et Novorapid® en Europe. Ce produit est une insuline analogue rapide. Les excipients de Novolog® sont la glycérine (16 mg), le phénol (1,50 mg/mL), le méta-crésol (1,72 mg/mL, du zinc (19,6 µg/mL), le phosphate de disodium dihydrate (1,25 mg/mL), le chlorure de sodium (0,5 mg/mL), l'hydroxyde de sodium et l'acide chlorhydrique pour l'ajustement du pH (pH 7,2-7,6) et de l'eau.

### Exemple C3 : Solution d'insuline analogue rapide (Apidra®) à 100 U/mL

Cette solution est une solution commerciale d'insuline glulisine commercialisée par la société SANOFI sous le nom d'Apidra®. Ce produit est une insuline analogue rapide. Les excipients d'Apidra® sont le méta-crésol (3,15 mg/mL), la trométhamine (6 mg/mL), le chlorure de sodium (5 mg/mL), le polysorbate 20 (0,01 mg/mL), l'hydroxyde de sodium et l'acide chlorhydrique pour l'ajustement du pH (pH 7,3) et de l'eau.

### Exemple C4 : Solution d'insuline analogue lente (Lantus®) à 100 U/mL

Cette solution est une solution commerciale d'insuline glargine commercialisée par la société SANOFI sous le nom de Lantus®. Ce produit est une insuline analogue lente. Les excipients dans Lantus® sont le chlorure de zinc (30 µg/mL), le méta-crésol (2,7 mg/mL), le glycérol (85%) (20 mg/mL), l'hydroxyde de sodium et l'acide chlorhydrique pour l'ajustement du pH (pH 4) et de l'eau.

### Exemple C5 : Solution d'insuline humaine (ActRapid®) à 100 UI/mL

Cette solution est une solution commerciale d'insuline humaine de NOVO NORDISK vendue sous le nom d'ActRapid®. Ce produit est une insuline humaine. Les excipients d'ActRapid® sont le chlorure de zinc, le glycérol le méta-crésol, l'hydroxyde de sodium et l'acide chlorhydrique pour l'ajustement du pH (pH 6,9-7,8) et de l'eau.

### Exemple C6 : Solution d'insuline humaine (Umuline rapide®) à 100 UI/mL

Cette solution est une solution commerciale d'insuline humaine de ELI LILLY vendue sous le nom d'Umuline rapide®. Ce produit est une insuline humaine. Les excipients d'Umuline Rapide® sont le glycérol, le méta-crésol, l'hydroxyde de sodium et l'acide chlorhydrique pour l'ajustement du pH (pH 7,0-7,8) et de l'eau.

### Exemple D1 : Solution de GLP-1 RA dulaglutide (Trulicity®) à 3 mg/mL

Cette solution est une solution de dulaglutide commercialisée par la société ELI LILLY sous le nom de Trulicity®. Les excipients dans Trulicity® sont l'acide citrique anhydre (0,14 mg/mL), le mannitol (46,4 mg/mL), le polysorbate 80 (0,20 mg/mL), le citrate trisodique dihydrate (2,74 mg/mL) et l'eau.

### Exemple D2 : Solution de GLP-1 RA exenatide (Byetta®) à 0,25 mg/mL

Cette solution est une solution d'exenatide commercialisée par la société ELI LILLY sous le nom de Byetta®. Les excipients dans Byetta® sont le méta-crésol (20 mM), le mannitol, l'acide acétique glacial, l'acétate sodique trihydrate et de l'eau.

### Exemple D3 : Solution de GLP-1 RA liraglutide (Victoza®) à 6 mg/mL

Cette solution est une solution de liraglutide commercialisée par la société NOVO NORDISK sous le nom de Victoza®. Les excipients dans Victoza® sont le phosphate disodique dihydrate, le propylène glycol (1,42 mg/mL), le phénol (5,5 mg/mL) et l'eau.

### Exemple D4 : Solution de GLP-1 RA lixisénatide (Lyxumia®) à 0,1 mg/mL

Cette solution est une solution de lixisénatide commercialisée par la société SANOFI sous le nom de Lyxumia®. Les excipients dans Lyxumia® sont le glycérol, l'acétate de sodium, la méthionine, le méta-crésol (25 mM), l'acide chlorhydrique et l'hydroxyde de sodium pour l'ajustement du pH et l'eau.

### Exemple D5 : Solution de GLP-1 RA albiglutide (Tanzeum®) à 60 mg/mL

Albiglutide est présenté sous une forme solide à reconstituer, commercialisée par la société GSK sous le nom de Tanzeum®. Dans un dispositif d'injection, la poudre et le volume d'eau nécessaire à la solubilisation, initialement dans deux compartiments séparés, sont mélangés en actionnant le stylo par rotation de l'extrémité distale du dispositif jusqu'à l'obtention d'un bruit caractéristique « click ». La solution est prête pour l'injection après une procédure de mélanges et de temps de repos décrite dans la notice d'utilisation du produit. Les excipients dans Tanzeum® sont le mannitol (153 mM), le polysorbate 80 (0,01% w/w), le phosphate de sodium (10 mM), le tréhalose dihydrate (117 mM).

### I. Détermination des ratios mininimum pour solubiliser l'insuline glargine

### Exemple G3 : Protocole pour la détermination de la concentration minimale pour solubiliser insuline glargine à 50 U/mL à pH 7.

A une solution mère de co-polyaminoacide à pH 7 sont ajoutées des solutions concentrées de m-crésol et de glycérine de manière à obtenir une solution de co-polyaminoacide de concentration C_{mère co-polyaminoacide /excipients} (mg/mL). La quantité d'excipients ajoutée est ajustée de manière à obtenir une concentration de m-crésol de 35 mM et glycérine de 184 mM dans la composition co-polyaminoacide / insuline glargine 50 U/mL à pH 7,1.

Dans un vial de 3 mL, 0,5 mL d'une solution commerciale d'insuline glargine commercialisée sous le nom de Lantus® à une concentration de 100 U/mL est ajouté à un volume de 0,5 mL d'une solution de co-polyaminoacide à la concentration C_{mère co-polyaminoacide /excipients} (mg/mL) de manière à obtenir une composition C_{co-polyaminoacide} (mg/mL) / insuline glargine 50 U/mL à pH 7,1. Un trouble apparaît. Le pH est ajusté à pH 7,1 par ajout de NaOH concentrée et la solution est placée en statique dans une étuve à 40°C pendant 1 nuit. Cette opération est réalisée pour différentes concentrations de C_{mère co-polyaminoacide /excipients} (mg/mL) de manière à faire varier la concentration de co-polyaminoacide C_{co-polyaminoacide} (mg/mL) par pas de maximum 0,25 mg/mL. Après la nuit à 40°C les échantillons sont inspectés visuellement et soumis à une mesure de diffusion statique de la lumière à un angle de 173° à l'aide d'un zetasizer (Malvern). La concentration minimale de co-polyaminoacide permettant de solubiliser l'insuline glargine est définie comme la concentration la plus basse pour laquelle le mélange co-polyaminoacide / insuline glargine à pH 7 est visuellement limpide et présente une intensité diffusée inférieure à 3000 kcps/s.

**Tableau 2 : ratios minimum pour solubiliser l'insuline glargine.**

| **co-polyaminoacide** | **Concentration de co-polyaminoacide (mg/mL) au seuil de solubilisation de glargine 50 U/mL à pH 7** | **Ratio [Hy]/[insuline glargine] (mol/mol) au seuil de solubilisation** |
|---|---|---|
| BB7 | < 1,5 | < 1,5 |
| BB5 | < 2 | < 1,5 |
| BB3 | < 2 | < 1,5 |
| BB14 | < 2 | < 1,5 |
| BB10 | < 1,5 | < 1 |
| BB15 | < 1,5 | < 1 |
| BB11 | < 1,5 | < 1 |
| BB16 | < 1,5 | < 1 |
| AB6 | < 2 | < 1 |
| AB21 | < 1,5 | < 1,5 |
| AB17 | < 1,5 | < 1 |
| AB18 | < 1,5 | < 1 |
| AB21' | < 1,5 | < 1 |
| BB17 | < 2 | < 1 |
| BB18 | < 1,5 | < 1 |
| BB20 | < 1 | < 1 |
| BB21 | < 1,5 | < 1,5 |
| BB22 | < 1 | < 1 |
| BB23 | < 1 | < 1 |
| BB24 | < 1 | < 1 |
| BB25 | < 1 | < 1 |
| BB26 | < 1 | < 1 |
| BB42 | < 1 | < 1 |

### II. Solubilisation/précipitation

### a) Compositions comprenant de l'insuline glargine

### Procédé de préparation CA1 : Préparation d'une composition diluée co-polyaminoacide / insuline glargine 50 U/mL à pH 7,1, suivant un procédé utilisant l'insuline glargine sous forme liquide (en solution) et un co-polyaminoacide sous forme liquide (en solution).

A une solution mère de co-polyaminoacide à pH 7,1 sont ajoutées des solutions concentrées de m-crésol et de glycérine de manière à obtenir une solution de co-polyaminoacide de concentration C_{mère co-polyaminoacide/excipients} (mg/mL). La quantité d'excipients ajoutée est ajustée de manière à obtenir une concentration de m-crésol de 35 mM et glycérine de 184 mM dans la composition co-polyaminoacide / insuline glargine 50 U/mL à pH 7,1.

Dans un pot stérile, un volume V_{insuline glargine} d'une solution commerciale d'insuline glargine commercialisée sous le nom de Lantus® à une concentration de 100 U/mL est ajouté à un volume V_{mère co-polyaminoacide /excipients} d'une solution de co-polyaminoacide à la concentration C_{mère co-polyaminoacide /excipients} (mg/mL) de manière à obtenir une composition diluée co-polyaminoacide C_{co-polyaminoacide dilué} (mg/mL)/insuline glargine 50 U/mL à pH 7,1. Un trouble apparaît. Le pH est ajusté à pH 7,1 par ajout de NaOH concentrée et la solution est placée en statique dans une étuve à 40°C pendant 2h jusqu'à solubilisation complète. Cette solution visuellement limpide est placée à +4°C.

### Procédé de préparation CA2 : Préparation d'une composition co-polyaminoacide/insuline glargine concentrée à pH 7,1 à l'aide d'un co-polyaminoacide, suivant un procédé de concentration d'une composition diluée.

Une composition co-polyaminoacide/insuline glargine 50 U/mL à pH 7,1 décrite dans l'exemple CA1 est concentrée par ultrafiltration sur une membrane 3 kDa en cellulose régénérée (Amicon® Ultra-15 commercialisée par la société Millipore). A l'issue de cette étape d'ultrafiltration, le rétentat est limpide et la concentration en insuline glargine dans la composition est déterminée par chromatographie en phase inverse (RP-HPLC). La concentration en insuline glargine dans la composition est ensuite ajustée à la valeur souhaitée par dilution dans une solution d'excipients m-crésol/glycérine de manière à obtenir une concentration finale en m-crésol de 35 mM et une osmolarité de 300 mOsm/kg. Le pH est mesuré et ajusté à pH 7,1 par ajout de NaOH et HCl concentré. Cette solution à pH 7,1, visuellement limpide, présente une concentration en insuline glargine C_{insuline glargine} (U/mL) et une concentration en co-polyaminoacide C_{co-polyaminoacide} (mg/mL) = C_{co-polyaminoacide dilué} (mg/mL) x C_{insuline glargine} (U/mL) / 50 (U/mL).

Selon ce procédé de préparation CA2, des compositions co-polyaminoacide/insuline glargine ont été préparées par exemple avec des concentrations en insuline glargine de 200 U/mL et 400 U/mL.

### Exemple CA3 : Préparation de compositions co-polyaminoacide/insuline glargine 200 U/mL à pH 7,1.

Des compositions co-polyaminoacide/insuline glargine 200 U/mL sont préparées selon le procédé décrit dans l'exemple CA2 de manière à obtenir une concentration en insuline glargine C_{insuline glargine} = 200 U/mL et une concentration en co-polyaminoacide C_{co-polyaminoacide} (mg/mL). Ces compositions sont présentées dans le tableau 3 suivant.

### Exemple CA4 : Précipitation de l'insuline glargine dans des compositions co-polyaminoacide / insuline glargine à 200 U/mL

1 mL de solution de co-polyaminoacide/insuline glargine préparée à l'exemple CA3 est ajouté dans 2 mL d'une solution de PBS contenant 20 mg/mL de BSA (bovine serum albumin). Le mélange PBS/BSA simule la composition du milieu sous-cutané. Un précipité apparaît.

Une centrifugation à 4000 trs/min est effectuée pour séparer le précipité du surnageant. Ensuite l'insuline glargine est dosée dans le surnageant par RP-HPLC. Il en résulte que l'insuline glargine se retrouve majoritairement sous une forme précipitée.

Les résultats sont présentés dans le tableau 3 :

**Tableau 3 : Compositions co-polyaminoacide /insuline glargine (200 U/mL) préparées avec les co-polyaminoacides de l'invention ; solubilisation/précipitation de l'insuline glargine.**

| Composition | Insuline glargine (U/mL) | Co-polyaminoacide | Concentratio n en co-polyaminoac ide (mg/mL) | Solubilisati on de l'insuline glargine | Précipitati on de l'insuline glargine |
|---|---|---|---|---|---|
| CA3 | 200 | - | - | NON | na |
| CA3b | 200 | AB18 | 6 | OUI | OUI |
| CA3c | 200 | BB5 | 9 | OUI | OUI |
| CA3d | 200 | BB11 | 6 | OUI | OUI |
| CA3e | 200 | BB14 | 10 | OUI | OUI |
| CA3f | 200 | BB15 | 6 | OUI | OUI |
| CA3g | 200 | BB16 | 6 | OUI | OUI |
| CA3l | 200 | BB15 | 5 | OUI | OUI |
| CA3m | 200 | AB21' | 6 | OUI | OUI |
| CA3n | 200 | BB18 | 6 | OUI | OUI |
| CA3o | 200 | BB17 | 9 | OUI | OUI |
| CA3p | 200 | BB25 | 4,5 | OUI | OUI |
| CA3q | 200 | BB26 | 5 | OUI | OUI |
| CA3t | 200 | BB20 | 5 | OUI | OUI |
| CA3u | 200 | BB21 | 5 | OUI | OUI |

### Exemple DB3 : Préparation de compositions co-polyaminoacide/ insuline glargine/ dulaglutide à pH 7,1

A 3 mL de la solution de co-polyaminoacide / insuline glargine préparée selon le protocole de l'exemple CA2, dont la concentration en insuline glargine est de 400 U/mL, est ajouté 2 mL de la solution de dulaglutide de l'exemple D1 et 1 mL d'eau pour obtenir 6 mL d'une composition à pH 7. Le pH est ajusté à 7,1 avec une solution de soude 0,1 N. La composition contenant 7 mg/mL de co-polyaminoacide AB6, 200 U/mL d'insuline glargine et 1 mg/mL de dulaglutide est limpide attestant de la bonne solubilité de l'insuline glargine et de dulaglutide en présence du co-polyaminoacide à pH 7,1. Cette solution limpide est placée à +4°C.
Selon le protocole de l'exemple DB3, les compositions avec différents co-polyaminoacides sont réalisées et présentées dans le tableau 4 ci-dessous.

### Exemple DB5 : Préparation de compositions co-polyaminoacide/ insuline glargine/ dulaglutide à pH 7,2

Selon le protocole de l'exemple DB3, les compositions avec différents co-polyaminoacides sont réalisées à pH 7,2 et présentées dans le tableau 4 ci-dessous.

**Tableau 4: Compositions co-polyaminoacide /insuline glargine (200 U/mL) / dulaglutide (1 mg/mL) à pH 7,2.**

| Composition | Co-polyaminoacide | Insuline glargine (U/mL) | Dulaglutide (mg/mL) | Co-polyaminoacide (mg/mL) |
|---|---|---|---|---|
| DB3h | **BB10** | 200 | 1 | 6 |
| DB3i | BB15 | 200 | 1 | 6 |

### Exemple DB6 : Préparation de compositions co-polyaminoacide/ insuline glargine à pH 7,2

Selon le procotole de l'exemple CA2, la composition DB3k décrite dans le tableau 5 ci-dessous a été réalisée.

**Tableau 5: Compositions co-polyaminoacide BB15/insuline glargine (200 U/mL) à pH 7,2.**

| | | DB3k |
|---|---|---|
| Insuline glargine | U/mL | 200 |
| BB15 | mg/mL | 5 |
| m-cresol | mM | 25 |
| Zn(II) | mM | 1,12 |
| glycérine | mM | 120 |
| PS20 | mM | 0,032 |
| mannitol | mM | 85 |
| PS80 | mM | 0,051 |
| pH | | 7,2 |

### Partie D' - contre-exemples

### Protocoles de préparation

Des compositions co-polyaminoacide contre-exemple /insuline glargine 200 U/mL sont préparées selon le procédé décrit dans l'exemple CA2 de manière à obtenir une concentration en insuline glargine C_{insuline glargine} = 200 U/mL et une concentration en co-polyaminoacide contre-exemple C_{co-polyaminoacide contre-exemple} (mg/mL). Ces compositions sont présentées dans le tableau 6 suivant.

**Tableau 6: Compositions contre-exemple co-polyaminoacide /insuline glargine (200 U/mL).**

| Composition | Insuline glargine (U/ml) | Co-polyaminoacide | Concentration en co-polyaminoacide (mg/mL) |
|---|---|---|---|
| CA3h | 200 | CEB1 | 13 |
| CA3i | 200 | CEB2 | 6 |
| CA3j | 200 | CEB3 | 7 |
| CA3k | 200 | CEB4 | 10 |
| CA3w | 200 | CEBX4 | 5 |

### III. Détermination de la quantité d'albumine nécessaire pour obtenir la précipitation

### Exemple G1 : Préparation d'une composition diluée co-polyaminoacide / insuline glargine 65 U/mL à pH 7,1.

A une solution mère de co-polyaminoacide à pH 7 sont ajoutées des solutions concentrées de m-crésol et de glycérine de manière à obtenir une solution de co-polyaminoacide de concentration C_{mère co-polyaminoacide /excipients} (mg/mL). La quantité d'excipients ajoutée est ajustée de manière à obtenir une concentration de m-crésol de 35 mM et glycérine de 184 mM dans la composition co-polyaminoacide / insuline glargine 65 U/mL à pH 7,1.

Dans un pot stérile, un volume V_{insuline glargine} d'une solution commerciale d'insuline glargine commercialisée sous le nom de Lantus® à une concentration de 100 U/mL est ajouté à un volume V_{mère co-polyaminoacide /excipients} d'une solution de co-polyaminoacide à la concentration C_{mère co-polyaminoacide /excipients} (mg/mL) de manière à obtenir une composition diluée co-polyaminoacide C_{co-polyaminoacide dilué} (mg/mL) / insuline glargine 65 U/mL à pH 7,1. Un trouble apparaît. Le pH est ajusté à pH 7,1 par ajout de NaOH concentrée et la solution est placée en statique dans une étuve à 40°C pendant 2h jusqu'à solubilisation complète. Cette solution visuellement limpide est placée à +4°C.

### Exemple G2 : Précipitation d'une composition co-polyaminoacide / insuline glargine 65 U/mL à pH 7,1, en faisant varier la concentration d'albumine

Dans une cuve UV jetable sont introduits respectivement 0,3 mL d'une solution de BSA (Bovine serum Albumine, sérum albumine bovine) dans un tampon PBS à pH 7,4 (Phosphate Buffer Saline, tampon phosphate salin) et 1 mL de composition diluée co-polyaminoacide / insuline glargine 65 U/mL pH 7,1 de manière à obtenir un mélange contenant 50 U/mL d'insuline glargine, une concentration d'albumine C_{BSA} (mg/mL) dans un tampon PBS. Plusieurs solutions de BSA dans un tampon PBS de concentrations variables sont préparées de manière à faire varier la concentration d'albumine dans le mélange final entre 1 et 12,7 mg/mL (1 ; 2,9 ; 3,9 ; 6,8 ; 9,7 ; 12,7 mg/mL) et une concentration de sel physiologique par l'intermédiaire du tampon PBS.

Après ajout de la solution de BSA dans le tampon PBS le mélange est rapidement homogénéisé par quelques allez-retour de pipette. Une heure après le mélange, une mesure d'absorbance à 500 nm est réalisée à l'aide d'un spectrophotomètre UV visible JASCO V-530.

La mesure d'absorbance à 500 nm permet d'évaluer la turbité du mélange émanant de la précipitation de l'insuline glargine. La turbidité augmente en fonction de la concentration d'albumine pour atteindre un plateau traduisant la précipitation complète de l'insuline glargine.

La concentration critique d'albumine permettant une précipitation quantitative est définie comme la concentration d'albumine pour laquelle la valeur d'absorbance à 500 nm atteint 80% de l'absorbance mesurée au plateau.

On constate que dans les compositions de l'invention, la quantité de BSA critique est inférieure.

Les résultats sont reportés dans le tableau 7 suivant :

**Tableau 7 : concentration d'albumine critique (mg/ml) pour 80% de précipitation à 1h (insuline glargine à 50 U/ml).**

| **Co-polyaminoacide** | **Concentration d'albumine critique (mg/ml) pour 80% de précipitation à 1h (insuline glargine à 50 U/ml)** | **Concentration de co-polyaminoacide dans solution insuline glargine à 50 U/ml** |
|---|---|---|
| CEB2 | ≥ 3,9 | 1,5 |
| CEB3 | ≥ 3,9 | 1,8 |
| CEB4 | ≥ 3,9 | 1,5 |
| BB7 | < 3,9 | 1,8 |
| BB5 | < 3,9 | 2,2 |
| BB14 | < 3,9 | 2,5 |
| BB10 | < 3,9 | 1,5 |
| BB15 | < 3,9 | 2,0 |
| BB11 | < 3,9 | 1,5 |
| BB16 | < 3,9 | 1,5 |
| AB6 | < 3,9 | 1,8 |
| AB21 | < 3,9 | 1,5 |
| AB17 | < 3,9 | 1,5 |
| AB18 | < 3,9 | 1,5 |
| AB21' | < 3,9 | 1,5 |
| BB42 | < 3,9 | 1,2 |
| BB18 | < 3,9 | 1,5 |
| BB17 | < 3,9 | 1,75 |
| BB25 | < 3,9 | 1,1 |
| BB20 | < 3,9 | 1,25 |
| BB21 | < 3,9 | 1,25 |

### IV. Etude de la stabilité des compositions selon l'invention

### Partie E : Mise en évidence de la stabilité physique des compositions selon l'invention par l'étude de compositions co-polyaminoacide/insuline glargine 200 U/mL

### Exemple E1 : Stabilité accélérée à 25°C en dynamique.

3 vials de 3 mL remplis avec 1 mL de composition co-polyaminoacide/ insuline glargine sont placés verticalement sur un agitateur orbital. L'agitateur est placé dans une étuve à 25°C et les vials sont soumis à une agitation de 250 rpm. Les vials sont inspectés visuellement de manière quotidienne/hebdomadaire afin de détecter l'apparition de particules visibles ou d'une turbidité. Cette inspection est réalisée selon les recommandations de la Pharmacopée Européenne (EP 2.9.20) : les vials sont soumis à un éclairage d'au moins 2000 Lux et sont observés face à un fond blanc et un fond noir. Le nombre de jours de stabilité correspond à la durée à partir de laquelle au moins 2 vials présentent des particules visibles ou sont turbides.

Ces résultats sont en accord avec la pharmacopée US (USP <790>).

Les résultats de stabilité accélérée (obtenus avec différentes compositions sont présentés dans le tableau 8 ci-après.

**Tableau 8: résultats des stabilités des compositions co-polyaminoacide/insuline glargine (200 U/mL) à 25°C en dynamique (sous agitation à 250 rpm). (*Apparition d'un précipité quand le pH de la solution d'insuline glargine est ajusté à pH 7).**

| **Composition** | **Co-polyaminoacide** | **Stabilité à 25°C en dynamique (en jours)** |
|---|---|---|
| CA3 | - | * |
| CA3b | AB18 | 11 |
| CA3c | BB5 | >30 |
| CA3d | BB11 | 19 |
| CA3e | BB14 | 11 |
| CA3f | BB15 | 17 |
| CA3h | CEB1 | 7 |
| CA3i | CEB2 | >15 |
| CA3j | CEB3 | 7 |
| CA3k | CEB4 | 7 |
| CA3m | AB21' | 11 |
| CA3n | **BB18** | >44 |
| CA3o | BB17 | 39 |
| CA3p | BB25 | 18 |
| CA3q | BB26 | 39 |
| CA3t | BB20 | 22 |
| CA3u | BB21 | 28 |
| CA3w | CEBX4 | 2 |

### Exemple E2 : Stabilité accélérée à 30°C en statique

5 vials de 3 mL remplis avec 1 mL de composition sont placés verticalement dans une étuve maintenue à 30°C. Les vials sont inspectés visuellement de manière quotidienne afin de détecter l'apparition de particules visibles ou d'une turbidité. Cette inspection est réalisée selon les recommandations de la Pharmacoppée Européenne (EP 2.9.20) : les vials sont soumis à un éclairage d'au moins 2000 Lux et sont observés face à un fond blanc et un fond noir. Le nombre de semaines de stabilité correspond à la durée à partir de laquelle au moins la moitié des vials présentent des particules visibles ou sont turbides.

Ces résultats sont en accord avec la pharmacopée US (USP <790>).

Les résultats de stabilité accélérée (obtenus avec différentes compositions sont présentés dans les tableaux 9 ci-après.

**Tableau 9 : résultats des stabilités des compositions co-polyaminoacide/insuline glargine (200 U/mL) à 30°C en statique. (*Apparition d'un précipité quand le pH de la solution est ajusté à pH 7).**

| **Composition** | **Co-polyaminoacide** | **Stabilité à 30°C en statique (en semaine)** |
|---|---|---|
| CA3 | - | * |
| CA3f | BB15 | >7 |
| CA3k | CEB4 | Jaune après 2,5 semaines |

### V. Exemples de compositions selon l'invention

### Exemple H1 : Préparation de compositions de co-polyaminoacides et d'insuline glargine à 200 U/mL et à pH 7,1

De manière analogue à l'exemple CA3 des compositions d'insuline glargine à 150 U/mL et de co-polyaminoacides sont préparées. Elles sont présentées dans le tableau 9a suivant :

**Tableau 9a : Compositions de co-polyaminoacides et d'insuline glargine à 200 U/mL et à pH 7,1.**

| Composition | Insuline glargine (U/mL) | Co-polyaminoacide | Concentration en co-polyaminoacide |
|---|---|---|---|
| CA3 | 200 | - | - |
| CA3b | 200 | AB18 | 6 |
| CA3c | 200 | BB5 | 9 |
| CA3d | 200 | BB11 | 6 |
| CA3e | 200 | BB14 | 10 |
| CA3f | 200 | BB15 | 6 |
| CA3g | 200 | BB16 | 6 |

### Exemple H2 : Préparation de compositions de co-polyaminoacides et d'insuline glargine à 150 U/mL et à pH 7,1

De manière analogue à l'exemple H1, des compositions d'insuline glargine à 150 U/mL et de co-polyaminoacides sont préparées. Elles sont présentées dans le tableau 9b suivant :

**Tableau 9b : Compositions co-polyaminoacide /insuline glargine (150 U/mL).**

| Composition | Insuline glargine (U/mL) | Co-polyaminoacide | Concentration en co-polyaminoacide (mg/mL) |
|---|---|---|---|
| H2b | 150 | AB18 | 4,5 |
| H2c | 150 | BB5 | 6,75 |
| H2d | 150 | BB11 | 4,5 |
| H2e | 150 | BB14 | 7,5 |
| H2f | 150 | BB15 | 45 |
| H2q | 150 | BB16 | 4,5 |

### Exemple H3 : Préparation de compositions de co-polyaminoacide et d'insuline glargine à 300 U/mL et à pH 7,1

De manière analogue à l'exemple H1, des compositions d'insuline glargine à 300 U/mL et de co-polyaminoacides sont préparées. Elles sont présentées dans le tableau 10.

**Tableau 10 : Compositions co-polyaminoacide /insuline glargine (300 U/mL)**

| Composition | Insuline glargine | Co-polyaminoacide | Concentration en co-polyaminoacide (mg/mL) |
|---|---|---|---|
| H3b | 300 | AB18 | 9 |
| H3c | 300 | BB5 | 13,5 |
| H3d | 300 | BB11 | 9 |
| H3e | 300 | BB14 | 15 |
| H3f | 300 | BB15 | 9 |
| H3g | 300 | BB16 | 9 |

### Partie F : Etudes de pharmacocinétique et pharmacodynamie chez le chien

Exemple F1 : Etude de pharmacocinétique et pharmacodynamie chez le chien de la composition co-polyaminoacide BB15 (5 mg/mL) / insuline glargine (200 U/mL)

Des études chez le chien ont été conduites dans l'objectif d'évaluer la pharmacocinétique et la pharmacodynamie de l'insuline après administration d'une composition de co-polyaminoacide et d'insuline glargine (composition DB3k).

L'effet hypoglycémiant et les profils pharmacocinétiques de l'insuline de la composition DB3k ont été comparés à ceux de l'injection de la composition C4 (Lantus®) (pH 4) à la même dose.

Dix animaux qui ont été mis à jeun depuis 18 heures environ ont été injectés au niveau du cou, au-dessus de la région interscapulaire, à la dose de 0,5 U/kg d'insuline. Dans l'heure précédant l'injection, un prélèvement sanguin est réalisé afin de déterminer le niveau basal d'insuline et 3 prélèvements sont réalisés afin de déterminer le niveau basal de glucose. Des prélèvements sanguins sont ensuite réalisés pendant les 23h suivant l'administration pour décrire la pharmacocinétique de l'insuline. La glycémie est déterminée pendant 24h au moyen d'un glucomètre. Les niveaux d'insuline sont déterminés par un test ELISA.

Les courbes pharmacocinétiques médianes de l'insuline exprimées en déviation du niveau basal sont présentées dans la Figure 1.

Les courbes de pharmacodynamie moyenne du glucose exprimée en pourcents de déviation du niveau basal sont représentées dans la Figure 2.

Les profils pharmacocinétiques montrent qu'après administration de la composition DB3k un plateau est rapidement atteint et maintenu jusqu'à 14h après l'administration puis que les concentrations d'insuline diminuent lentement. Le profil est similaire à celui obtenu après l'injection de la composition C4 (Lantus®) indiquant que la composition DB3k ne modifie pas la cinétique de l'insuline glargine.

Les résultats de pharmacodynamie obtenus avec l'administration de la composition DB3k et l'injection de la composition C4 (Lantus®) sont présentés sur la figure 2. Après administration de la composition DB3k, une diminution de la glycémie est observée jusqu'à atteindre, environ 4h après l'administration, un plateau qui se maintient jusqu'à environ 13h. La glycémie remonte ensuite progressivement jusqu'à son niveau basal atteint environ 19h après l'administration. Le profil est similaire à celui obtenu après l'injection de la composition C4 (Lantus®) indiquant que l'effet de l'insuline glargine est bien conservé avec la composition DB3k.

## Revendications

1. Composition sous forme d'une solution aqueuse injectable, dont le pH est compris entre 6, 0 et 8,0, comprenant au moins :
a) une insuline basale dont le point isoélectrique pI est compris entre 5,8 et 8,5 ;
b) un co-polyaminoacide porteur de charges carboxylates et de radicaux hydrophobes Hy, ledit co-polyaminoacide étant constitué d'unités glutamiques ou aspartiques et lesdits radicaux hydrophobes Hy étant de formule I suivante : 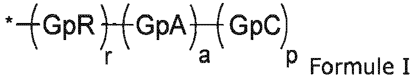 dans laquelle
- GpR est un radical de formules II ou II' : 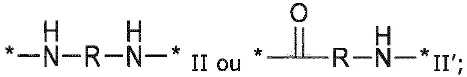
- GpA est un radical de formules III ou III' : 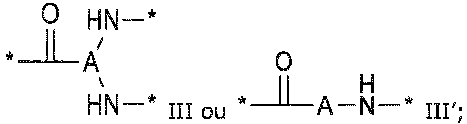
- GpC est un radical de formule IV : 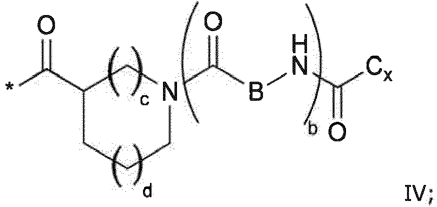
- Hy comprend plus de 30 atomes de carbone,
- les * indiquent les sites de rattachement des différents groupes liés par des fonctions amides ;
- a est un entier égal à 0 ou à 1 ;
- b est un entier égal à 0 ou à 1;
- p est un entier égal à 1 ou à 2 et
∘ si p est égal à 1 alors a est égal à 0 ou à 1 et GpA est un radical de formule III' et,
∘ si p est égal à 2 alors a est égal à 1, et GpA est un radical de formule III;
- c est un entier égal à 0 ou à 1, et si c est égal à 0 alors d est égal à 1 ou à 2;
- d est un entier égal à 0, à 1 ou à 2;
- r est un entier égal à 0 ou à 1, et
∘ si r est égal à 0 alors le radical hydrophobe de formule I est lié au co-polyaminoacide via une liaison covalente entre un carbonyl du radical hydrophobe et un atome d'azote en position N terminale du co-polyaminoacide, formant ainsi une fonction amide, et
∘ si r est égal à 1 alors le radical hydrophobe de formule I est lié au co-polyaminoacide :
▪ via une liaison covalente entre un atome d'azote du radical hydrophobe et un carbonyl du co-polyaminoacide, formant ainsi une fonction amide ou
▪ via une liaison covalente entre un carbonyl du radical hydrophobe et un atome d'azote en position N terminal du co-polyaminoacide, formant ainsi une fonction amide;
- R est un radical choisi dans le groupe constitué par :
∘ un radical alkyle divalent, linéaire ou ramifié, comprenant si GpR est un radical de formule II de 2 à 12 atomes de carbone ou si GpR est un radical de formule II' de 1 à 11 atomes de carbone ;
∘ un radical alkyle divalent, linéaire ou ramifié, comprenant si GpR est un radical de formule II de 2 à 11 atomes de carbone ou si GpR est un radical de formule II' de 1 à 11 atomes de carbone, ledit radical alkyle portant une ou plusieurs fonctions -CONH₂, et
∘ un radical éther ou polyéther non substitué comprenant de 4 à 14 atomes de carbone et de 1 à 5 atomes d'oxygène ;
- A est un radical alkyle linéaire ou ramifié comprenant de 1 à 6 atomes de carbone ;
- B est un radical alkyle linéaire ou ramifié, éventuellement comprenant un noyau aromatique, comprenant de 1 à 9 atomes de carbone ;
- Cₓ est un radical alkyl monovalent linéaire ou ramifié, dans lequel x indique le nombre d'atomes de carbone et :
∘ si p est égal à 1, x est compris entre 11 à 25 (11 ≤ x ≤ 25),
∘ si p est égal à 2, x est compris entre 9 et 15 (9 ≤ x ≤ 15),
- le ratio i entre le nombre de radicaux hydrophobes et le nombre d'unités glutamiques ou aspartiques étant compris entre entre 0 < i ≤ 0,5 ;
- lorsque plusieurs radicaux hydrophobes sont portés par un co-polyaminoacide alors ils sont identiques ou différents,
- le degré de polymérisation DP en unités glutamiques ou aspartiques est compris entre 5 et 250 ;
- les fonctions acides libres étant sous forme de sel de cation alkalin choisi dans le groupe constitué par Na⁺ et K⁺.

2. Composition selon la revendication 1, **caractérisée en ce que** lesdits radicaux hydrophobes sont choisis parmi les radicaux hydrophobes de formule I dans laquelle p = 1, représentée par la formule V suivante : 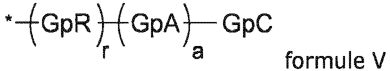 GpR, GpA, GpC, r et a tels que définis dans la revendication 1.

3. Composition selon la revendication 1, **caractérisée en ce que** lesdits radicaux hydrophobes sont choisis parmi les radicaux hydrophobes de formule I dans laquelle a = 1 et p = 2, représentée par la formule VI suivante : 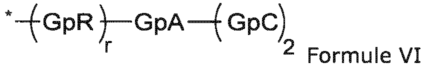 dans laquelle
GpR, GpA, GpC, r et a tels que définis dans la revendication 1.

4. Composition selon l'une quelconque des revendications précédentes, **caractérisée en ce que** le co-polyaminoacide porteur de charges carboxylates et de radicaux hydrophobes est choisi parmi les co-polyaminoacides de formule VII suivante : 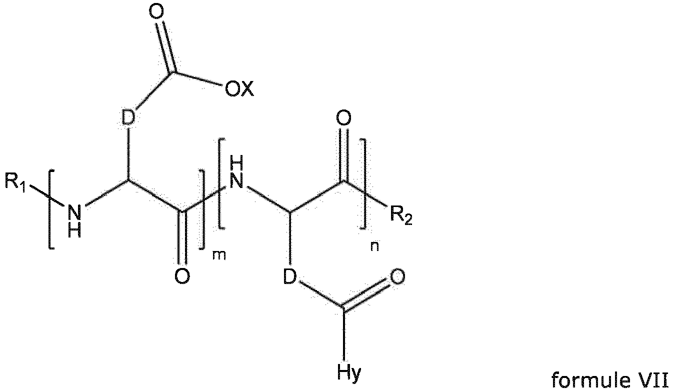 dans laquelle,
• D représente, indépendamment, soit un groupe -CH₂- (unité aspartique) soit un groupe -CH₂-CH₂- (unité glutamique),
• Hy est un radical hydrophobe choisi parmi les radicaux hydrophobes de formules I, V ou VI, dans lesquelles r = 1 et GpR est un radical de Formule II,
• R₁ est un radical hydrophobe choisi parmi les radicaux hydrophobes de formules I, V ou VI dans lesquelles r = 0 ou r = 1 et GpR est un radical de Formule II', ou un radical choisi dans le groupe constitué par un H, un groupe acyle linéaire en C2 à C10, un groupe acyle ramifié en C4 à C10, un benzyle, une unité « acide aminé » terminale et un pyroglutamate,
• R₂ est un radical hydrophobe choisi parmi les radicaux hydrophobes de formules I, V ou VI dans lesquelles r = 1 et GpR est un radical de Formule II, ou un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S,
• X représente une entité cationique choisie dans le groupe comprenant les cations alcalins ;
• n + m représente le degré de polymérisation DP du co-polyaminoacide, c'est-à-dire le nombre moyen d'unités monomériques par chaîne de co-polyaminoacide et 5 ≤ n + m ≤ 250 ;

5. Composition selon la revendication 4, **caractérisée en ce que** le co-polyaminoacide porteur de charges carboxylates et de radicaux hydrophobes est choisi parmi les co-polyaminoacides de formules VII, dans laquelle R₁ = R'₁ et R₂ = R'₂, de formule VIIa suivante : 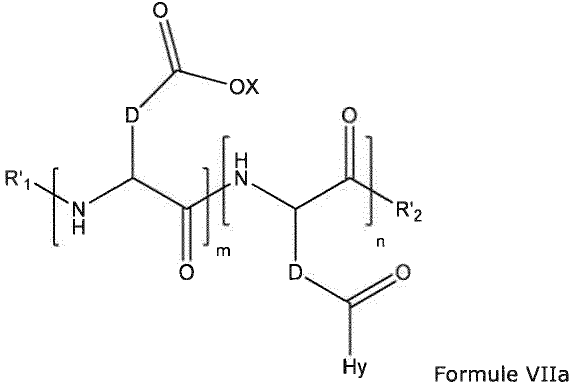 dans laquelle,
- m, n, X, D et Hy tels que définis dans la revendication 4,
- R'₁ est un radical choisi dans le groupe constitué par un H, un groupe acyle linéaire en C2 à C10, un groupe acyle ramifié en C4 à C10, un benzyle, une unité « acide aminé » terminale et un pyroglutamate,
- R'₂ est un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S.

6. Composition selon la revendication 4, **caractérisée en ce que** le co-polyaminoacide porteur de charges carboxylates et de radicaux hydrophobes est choisi parmi les co-polyaminoacides de formule VII dans laquelle n = 0 de formule VIIb suivante : 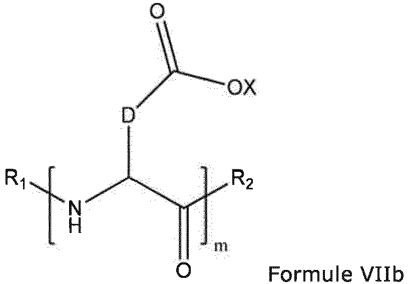 dans laquelle m, X, D, R₁ et R₂ tels que définis dans la revendication 4 et au moins R₁ ou R₂ est un radical hydrophobe de formule I, V ou VI.

7. Composition selon la revendication 6, **caractérisée en ce que** le co-polyaminoacide porteur de charges carboxylates et de radicaux hydrophobes est choisi parmi les co-polyaminoacides de formules VIIb dans laquelle R₂ est un radical hydrophobe de formule I, V ou VI dans lesquelles r = 1 et GpR est de Formule II.

8. Composition selon l'une quelconque des revendications 4 à 7, **caractérisé en ce que** R₁ est un radical choisi dans le groupe constitué par un groupe acyle linéaire en C₂ à C₁₀, un groupe acyle ramifié en C₄ à C₁₀, un benzyle, une unité « acide aminé » terminale et un pyroglutamate.

9. Composition selon la revendication précédente, **caractérisé en ce que** R₁ est un radical choisi dans le groupe constitué par un groupe acyle linéaire en C₂ à C₁₀ ou un groupe acyle ramifié en C₄ à C₁₀.

10. Composition selon l'une quelconque des revendications 4 à 9, **caractérisée en ce que** le co-polyaminoacide porteur de charges carboxylates et de radicaux hydrophobes est choisi parmi les co-polyaminoacides de formules VII, VIIa ou VIIb dans lesquels le co-polyaminoacide est choisi parmi les co-polyaminoacides dans lesquels le groupe D est un groupe -CH₂- (unité aspartique).

11. Composition selon l'une quelconque des revendications 4 à 9, **caractérisée en ce que** le co-polyaminoacide porteur de charges carboxylates et de radicaux hydrophobes est choisi parmi les co-polyaminoacides de formules VII, VIIa ou VIIb dans lesquels le co-polyaminoacide est choisi parmi les co-polyaminoacides dans lesquels le groupe D est un groupe -CH₂-CH₂- (unité glutamique).

12. Composition selon l'une quelconque des revendications précédentes, **caractérisée en ce que** l'insuline basale dont le point isoélectrique est compris entre 5,8 et 8,5 est l'insuline glargine.

13. Composition selon l'une quelconque des revendications précédentes, **caractérisée en ce qu'**elle comprend entre 40 et 500 U/mL d'insuline basale dont le point isoélectrique est compris entre 5,8 et 8,5.

14. Composition selon l'une quelconque des revendications précédentes, **caractérisée en ce que** la concentration en co-polyaminoacide porteur de charges carboxylates et de radicaux hydrophobes est au plus de 60 mg/mL.

15. Copolyaminoacide porteur de charges carboxylates et de radicaux hydrophobes Hy de formule I suivante : 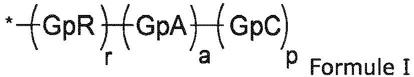 dans laquelle
- GpR est un radical de formules II ou II' : 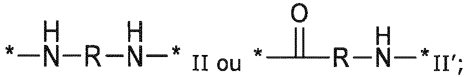
- GpA est un radical de formules III ou III' : 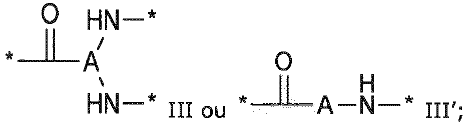
- GpC est un radical de formule IV : 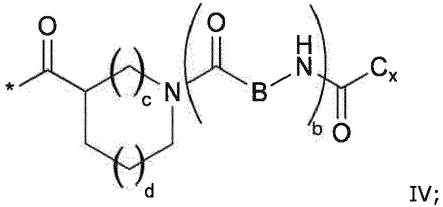
- Hy comprend plus de 30 atomes de carbone,
- les * indiquent les sites de rattachement des différents groupes liés par des fonctions amides ;
- a est un entier égal à 0 ou à 1 ;
- b est un entier égal à 0 ou à 1;
- p est un entier égal à 1 ou à 2 et
∘ si p est égal à 1 alors a est égal à 0 ou à 1 et GpA est un radical de formule III' et,
∘ si p est égal à 2 alors a est égal à 1, et GpA est un radical de formule III;
- c est un entier égal à 0 ou à 1, et si c est égal à 0 alors d est égal à 1 ou à 2;
- d est un entier égal à 0, à 1 ou à 2;
- r est un entier égal à 0 ou à 1, et
∘ si r est égal à 0 alors le radical hydrophobe de formule I est lié au co-polyaminoacide via une liaison covalente entre un carbonyl du radical hydrophobe et un atome d'azote en position N terminale du co-polyaminoacide, formant ainsi une fonction amide, et
∘ si r est égal à 1 alors le radical hydrophobe de formule I est lié au co-polyaminoacide :
▪ via une liaison covalente entre un atome d'azote du radical hydrophobe et un carbonyl du co-polyaminoacide, formant ainsi une fonction amide ou
▪ via une liaison covalente entre un carbonyl du radical hydrophobe et un atome d'azote en position N terminal du co-polyaminoacide, formant ainsi une fonction amide;
- R est un radical choisi dans le groupe constitué par :
∘ un radical alkyle divalent, linéaire ou ramifié, comprenant si GpR est un radical de formule II de 2 à 12 atomes de carbone ou si GpR est un radical de formule II' de 1 à 11 atomes de carbone ;
∘ un radical alkyle divalent, linéaire ou ramifié, comprenant si GpR est un radical de formule II de 2 à 11 atomes de carbone ou si GpR est un radical de formule II' de 1 à 11 atomes de carbone, ledit radical alkyle portant une ou plusieurs fonctions -CONH₂, et
∘ un radical éther ou polyéther non substitué comprenant de 4 à 14 atomes de carbone et de 1 à 5 atomes d'oxygène ;
- A est un radical alkyle linéaire ou ramifié comprenant de 1 à 6 atomes de carbone ;
- B est un radical alkyle linéaire ou ramifié, éventuellement comprenant un noyau aromatique, comprenant de 1 à 9 atomes de carbone ;
- Cₓ est un radical alkyl monovalent linéaire ou ramifié, dans lequel x indique le nombre d'atomes de carbone et :
∘ si p est égal à 1, x est compris entre 11 à 25 (11 ≤ x ≤ 25),
∘ si p est égal à 2, x est compris entre 9 et 15 (9 ≤ x ≤ 15),
- le ratio i entre le nombre de radicaux hydrophobes et le nombre d'unités glutamiques ou aspartiques étant compris entre entre 0 < i ≤ 0,5 ;
- lorsque plusieurs radicaux hydrophobes sont portés par un co-polyaminoacide alors ils sont identiques ou différents,
- le degré de polymérisation DP en unités glutamiques ou aspartiques est compris entre 5 et 250 ;
- les fonctions acides libres étant sous forme de sel de cation alkalin choisi dans le groupe constitué par Na⁺ et K⁺.

## Patentansprüche

1. Zusammensetzung in Form einer injizierbaren wässrigen Lösung, deren pH von 6,0 bis 8,0 beträgt, wenigstens umfassend:
a) ein Basalinsulin, dessen isoelektrischer Punkt pI zwischen 5,8 und 8,5 beträgt;
b) eine Carboxylatladungen und hydrophobe Radikale Hy tragende Co-Polyaminosäure, wobei die Co-Polyaminosäure aus Glutamin- und Asparagineinheiten besteht und die hydrophoben Radikale Hy die folgenden Formel I aufweisen: 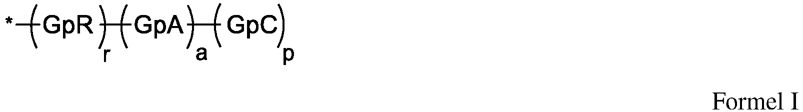 wobei
- GpR ein Radikal gemäß Formel II oder Formel II' ist: 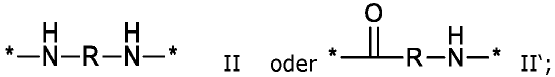
- GpA ein Radikal gemäß Formel III oder III' ist: 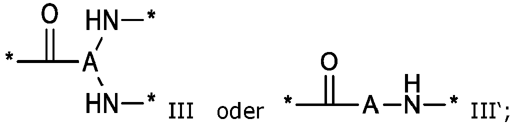
- GpC ein Radikal gemäß Formel IV ist: 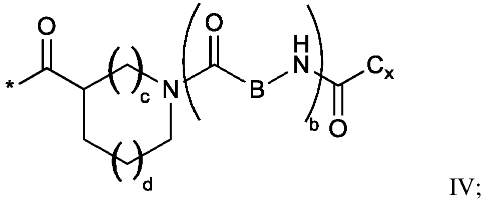
- Hy mehr als 30 Kohlenstoffatome umfasst,
- die * die Bindungsstellen der verschiedenen durch Amidfunktionen verbundenen Gruppen anzeigt;
- a eine ganze Zahl von 0 oder 1 ist;
- b eine ganze Zahl von 0 oder 1 ist;
- p eine ganze Zahl von 1 oder 2 ist und
- wenn p gleich 1 ist, a gleich 0 oder 1 und GpA ein Radikal der Formel III' ist und
- wenn p gleich 2 ist, a gleich 1 und GpA ein Radikal der Formel III ist;
- c eine ganze Zahl von 0 oder 1 ist und wenn c gleich 0 ist, d gleich 1 oder 2 ist;
- d eine ganze Zahl von 0, 1 oder 2 ist;
- r eine ganze Zahl von 0 oder 1 ist, und
- wenn r gleich 0 ist, das hydrophobe Radikal gemäß Formel I mit der Polyaminosäure mittels einer kovalenten Bindung zwischen einem Carbonyl des hydrophoben Radikals und einem Stickstoffatom an N-terminaler Position der Co-Polyaminosäure verbunden ist, wodurch eine Amidfunktion gebildet ist, und
- wenn r gleich 1 ist, das hydrophobe Radikal gemäß Formel I mit der Co-Polyaminosäure verbunden ist:
- mittels einer kovalenten Bindung zwischen einem Stickstoffatom des hydrophoben Radikals und einem Carbonyl der Co-Polyaminosäure, wodurch eine Amidfunktion gebildet ist, oder
- mittels einer kovalenten Bindung zwischen einem Carbonyl des hydrophoben Radikals und einem Stickstoffatom an N-terminaler Position der Co-Polyamionsäure, wodurch eine Amidfunktion gebildet ist;
- R ein Radikal ist, das ausgewählt ist aus der Gruppe bestehend aus:
- einem linearen oder verzweigten divalenten Alkylradikal umfassend, wenn GpR ein Radikal gemäß Formel II ist, 2 bis 12 Kohlenstoffatome und, wenn GpR ein Radikal gemäß Formel II' ist, 1 bis 11 Kohlenstoffatome;
- einem linearen oder verzweigten divalenten Alkylradikal umfassend, wenn GpR ein Radikal gemäß Formel II ist, 2 bis 11 Kohlenstoffatome und, wenn GpR ein Radikal gemäß Formel II' ist, 1 bis 11 Kohlenstoffatome, wobei das Alkylradikal eine oder mehrere -CONH2- Funktionen trägt, und
- einem nicht-substituierten Ether- oder Polyetherradikal umfassend 4 bis 14 Kohlenstoffatome und 1 bis 5 Sauerstoffatome;
- A ein lineares oder verzweigtes Radikal ist umfassend 1 bis 6 Kohlenstoffatome;
- B ein lineares oder verzweigtes Radikal ist, optional umfassend einen aromatischen Ring, umfassend 1 bis 9 Kohlenstoffatome;
- Cx ein lineares oder verzweigtes monovalentes Alkylradikal ist, wobei x die Anzahl von Kohlenstoffatomen angibt und
- wenn p gleich 1 ist, x zwischen 11 und 25 beträgt (11 ≤ x ≤ 25),
- wenn p gleich 2 ist, x zwischen 9 und 15 beträgt (9 ≤ x ≤ 15),
- das Verhältnis i aus der Anzahl hydrophober Radikale und der Anzahl an Glutamin- oder Asparagineinheiten zwischen 0 < i ≤ 0,5 beträgt;
- wenn mehrere hydrophobe Radikale von einer Co-Polyaminosäure getragen sind, sie identisch oder verschieden sind,
- der Polymerisationsgrad DP aus Glutamin- oder Asparagineinheiten zwischen 5 und 250 beträgt;
- die freien Säurefunktionen in Form eines Salzes eines Alkalikations vorliegen, das ausgewählt ist aus der Gruppe bestehend aus Na⁺ und K⁺.

2. Zusammensetzung nach Anspruch 1, **dadurch gekennzeichnet, dass** die hydrophoben Radikale ausgewählt sind aus den hydrophoben Radikalen gemäß Formel I, wobei p = 1 ist, dargestellt durch die folgende Formel V: 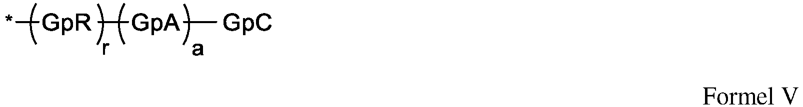 wobei GpR, GpA, GpC, r und a wie in Anspruch 1 definiert sind.

3. Zusammensetzung nach Anspruch 1, **dadurch gekennzeichnet, dass** die hydrophoben Radikale ausgewählt sind aus den hydrophoben Radikalen gemäß Formel I, wobei a = 1 und p = 2 ist, dargestellt durch die folgende Formel VI: 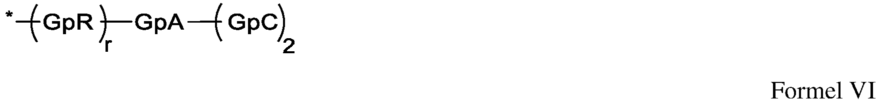 wobei
GpR, GpA, GpC, r und a wie in Anspruch 1 definiert sind.

4. Zusammensetzung nach einem der vorangehenden Ansprüche, **dadurch gekennzeichnet, dass** die Carboxylatladungen und hydrophobe Radikale tragende Co-Polyaminosäure ausgewählt ist aus den Co-Polyaminosäuren gemäß der folgenden Formel VII: 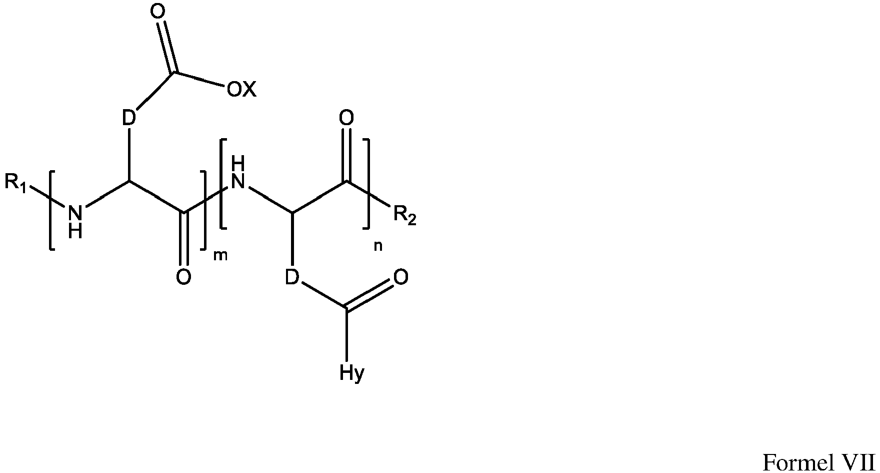 wobei
- D, unabhängig, entweder eine -CH₂- Gruppe (Asparagineinheit) oder eine -CH2-CH2-Gruppe (Glutamineinheit) darstellt,
- Hy ein hydrophobes Radikal ist, das ausgewählt ist aus den hydrophoben Radikalen gemäß Formeln I, V oder VI, wobei r = 1 und GpR ein Radikal gemäß Formel II ist,
- R₁ ein hydrophobes Radikal ist, das ausgewählt ist aus den hydrophoben Radikalen gemäß Formeln I, V oder VI, wobei r = 0 oder r = 1 und GpR ein Radikal gemäß Formel II' ist, oder ein Radikal ist, das ausgewählt ist aus der Gruppe bestehend aus H, einer linearen Acylgruppe aus C2 bis C10, einer verzweigten Acylgruppe aus C4 bis C10, einem Benzyl, einer terminalen "Aminosäure" -Einheit und Pyroglutamat,
- R₂ ein hydrophobes Radikal ist, das ausgewählt ist aus den hydrophoben Radikalen gemäß Formeln I, V oder VI, wobei r = 1 und GpR ein Radikal gemäß Formel II ist, oder ein Radikal -NR'R" ist, wobei R' und R" identisch oder verschieden sind und ausgewählt sind aus der Gruppe bestehend aus H, linearen oder verzweigten oder zyklischen Alkylen aus C2 bis C10, das Benzyl und die R'- und R"- Alkyle zusammen einen oder mehrere gesättigte, ungesättigte und/oder aromatische Kohlenstoffzyklen bilden können und/oder Heteroatome umfassen können, die ausgewählt sind aus der Gruppe bestehend aus O, N und S,
- X eine kationische Einheit darstellt, die ausgewählt ist aus der Gruppe umfassend Alkalikationen;
- n + m den Polymerisationsgrad DP der Co-Polyaminosäure darstellt, d.h. die mittlere Anzahl monomerer Einheiten pro Kette Co-Polyaminosäure und 5 ≤ n + m ≤ 250.

5. Zusammensetzung nach Anspruch 4, **dadurch gekennzeichnet, dass** die Carboxylatladungen und hydrophobe Radikale tragende Co-Polyaminosäure ausgewählt ist aus den Co-Polyaminosäuren gemäß Formeln VII; wobei R₁ = R'₁ und R₂ = R'₂ ist, gemäß der folgenden Formel VIIa: 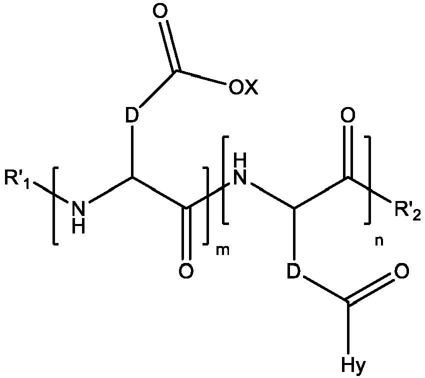 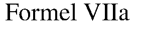 wobei
- m, n, X, D und Hy wie in Anspruch 4 definiert sind,
- R'₁ ein Radikal ist, das ausgewählt ist aus der Gruppe bestehend aus H, einer linearen Acylgruppe aus C2 bis C10, einer verzweigten Acylgruppe aus C4 bis C10, einem Benzyl, einer terminalen "Aminosäure"-Einheit und Pyroglutamat,
- R'₂ ein Radikal -NR'R" ist, wobei R' und R" identisch oder verschieden sind und ausgewählt sind aus der Gruppe bestehend aus H, linearen oder verzweigten oder zyklischen Alkylen aus C2 bis C10, Benzyl und die R'- und R"-Alkyle zusammen einen oder mehrere gesättigte, ungesättigte und/oder aromatische Kohlenstoffzyklen bilden können und/oder Heteroatome tragen können, die ausgewählt sind aus der Gruppe bestehend aus O, N und S.

6. Zusammensetzung nach Anspruch 4, **dadurch gekennzeichnet, dass** die Carboxylatladungen und hydrophobe Radikale tragende Co-Polyaminosäure ausgewählt ist aus den Co-Polyaminosäuren gemäß Formel VII, wobei n = 0 ist, gemäß der folgenden Formel VIIb: 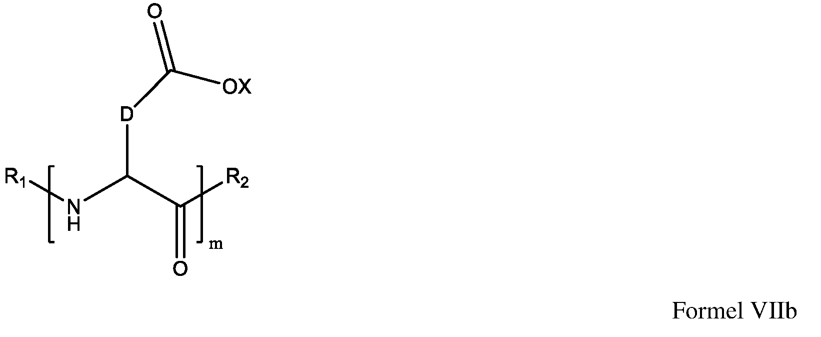 wobei m, X, D, R₁ und R₂ wie in Anspruch 4 definiert sind und wenigstens R₁ oder R₂ ein hydrophobes Radikal gemäß Formel I, V oder VI ist.

7. Zusammensetzung nach Anspruch 6, **dadurch gekennzeichnet, dass** die Carboxylatladungen und hydrophobe Radikale tragende Co-Polyaminosäure ausgewählt ist aus den Co-Polyaminosäuren gemäß Formel VIIb, wobei R₂ ein hydrophobes Radikal der Formel I, V oder VI ist, wobei r = 1 ist und GpR Formel II aufweist.

8. Zusammensetzung nach einem der Ansprüche 4 bis 7, **dadurch gekennzeichnet, dass** R₁ ein Radikal ist, das ausgewählt ist aus der Gruppe bestehend aus einer linearen Acylgruppe aus C₂ bis C₁₀, einer verzweigten Acylgruppe aus C₄ bis C₁₀, einem Benzyl, einer terminalen "Aminosäure"-Einheit und einem Pyroglutamat.

9. Zusammensetzung nach einem der vorangehenden Ansprüche, **dadurch gekennzeichnet, dass** R₁ ein Radikal ist, das ausgewählt ist aus der Gruppe bestehend aus einer linearen Acylgruppe aus C₂ bis C₁₀ oder einer verzweigten Acylgruppe aus C₄ bis C₁₀.

10. Zusammensetzung nach einem der Ansprüche 4 bis 9, **dadurch gekennzeichnet, dass** die Carboxylatladungen und hydrophobe Radikale tragende Co-Polyaminosäure ausgewählt ist aus den Polyaminosäure gemäß Formeln VII, VIIa oder VIIb, wobei die Co-Polyaminosäure ausgewählt ist aus den Co-Polyaminosäuren, wobei die Gruppe D eine -CH₂-Gruppe (Asparagineinheit) ist.

11. Zusammensetzung nach einem der Ansprüche 4 bis 9, **dadurch gekennzeichnet, dass** die Carboxylatladungen und hydrophobe Radikale tragende Co-Polyaminosäure ausgewählt ist aus den Co-Polyaminosäuren gemäß Formeln VII, VIIa oder VIIb, wobei die Co-Polyaminosäure ausgewählt ist aus den Co-Polyaminosäuren, wobei die Gruppe D eine -CH2-CH2- Gruppe (Glutamingruppe) ist.

12. Zusammensetzung nach einem der vorangehenden Ansprüche, **dadurch gekennzeichnet, dass** das Basalinsulin, dessen isoelektrischer Punkt zwischen 5,8 und 8,5 beträgt, Insulin glargin ist.

13. Zusammensetzung nach einem der vorangehenden Ansprüche, **dadurch gekennzeichnet, dass** sie zwischen 40 und 500 u/ml Basalinsulin umfasst, dessen isoelektrischer Punkt zwischen 5,8 und 8,5 beträgt.

14. Zusammensetzung nach einem der vorangehenden Ansprüche, **dadurch gekennzeichnet, dass** die Konzentration an Carboxylatladungen und hydrophobe Radikale tragender Co-Polyaminosäure höchstens 60 mg/ml beträgt.

15. Co-Polyaminosäure tragend Carboxylatladungen und hydrophobe Radikale Hy gemäß der folgenden Formel I: 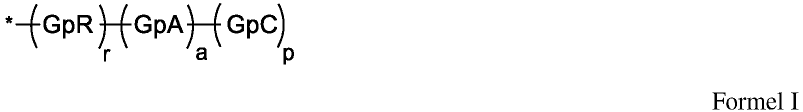 wobei
- GpR ein Radikal gemäß Formel II oder Formel II' ist: 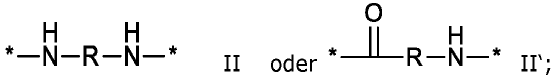
- GpA ein Radikal gemäß Formel III oder III' ist: 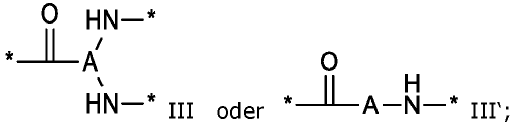
- GpC ein Radikal gemäß Formel IV ist: 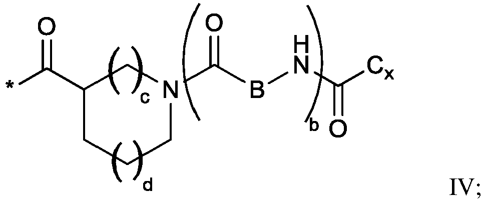
- Hy mehr als 30 Kohlenstoffatome umfasst,
- die * die Bindungsstellen der verschiedenen durch Amidfunktionen verbundenen Gruppen anzeigt;
- a eine ganze Zahl von 0 oder 1 ist;
- b eine ganze Zahl von 0 oder 1 ist;
- p eine ganze Zahl von 1 oder 2 ist und
- wenn p gleich 1 ist, a gleich 0 oder 1 und GpA ein Radikal der Formel III' ist und
- wenn p gleich 2 ist, a gleich 1 und GpA ein Radikal der Formel III ist;
- c eine ganze Zahl von 0 oder 1 ist und wenn c gleich 0 ist, d gleich 1 oder 2 ist;
- d eine ganze Zahl von 0, 1 oder 2 ist;
- r eine ganze Zahl von 0 oder 1 ist, und
- wenn r gleich 0 ist, das hydrophobe Radikal gemäß Formel I mit der Polyaminosäure mittels einer kovalenten Bindung zwischen einem Carbonyl des hydrophoben Radikals und einem Stickstoffatom an N-terminaler Position der Co-Polyaminosäure verbunden ist, wodurch eine Amidfunktion gebildet ist, und
- wenn r gleich 1 ist, das hydrophobe Radikal gemäß Formel I mit der Co-Polyaminosäure verbunden ist:
- mittels einer kovalenten Bindung zwischen einem Stickstoffatom des hydrophoben Radikals und einem Carbonyl der Co-Polyaminosäure, wodurch eine Amidfunktion gebildet ist, oder
- mittels einer kovalenten Bindung zwischen einem Carbonyl des hydrophoben Radikals und einem Stickstoffatom an N-terminaler Position der Co-Polyamionsäure, wodurch eine Amidfunktion gebildet ist;
- R ein Radikal ist, das ausgewählt ist aus der Gruppe bestehend aus:
- einem linearen oder verzweigten divalenten Alkylradikal umfassend, wenn GpR ein Radikal gemäß Formel II ist, 2 bis 12 Kohlenstoffatome und, wenn GpR ein Radikal gemäß Formel II' ist, 1 bis 11 Kohlenstoffatome;
- einem linearen oder verzweigten divalenten Alkylradikal umfassend, wenn GpR ein Radikal gemäß Formel II ist, 2 bis 11 Kohlenstoffatome und, wenn GpR ein Radikal gemäß Formel II' ist, 1 bis 11 Kohlenstoffatome, wobei das Alkylradikal eine oder mehrere -CONH2- Funktionen trägt, und
- einem nicht-substituierten Ether- oder Polyetherradikal umfassend 4 bis 14 Kohlenstoffatome und 1 bis 5 Sauerstoffatome;
- A ein lineares oder verzweigtes Radikal ist umfassend 1 bis 6 Kohlenstoffatome;
- B ein lineares oder verzweigtes Radikal ist, optional umfassend einen aromatischen Ring, umfassend 1 bis 9 Kohlenstoffatome;
- Cx ein lineares oder verzweigtes monovalentes Alkylradikal ist, wobei x die Anzahl von Kohlenstoffatomen angibt und
- wenn p gleich 1 ist, x zwischen 11 und 25 beträgt (11 ≤ x ≤ 25),
- wenn p gleich 2 ist, x zwischen 9 und 15 beträgt (9 ≤ x ≤ 15),
- das Verhältnis i aus der Anzahl hydrophober Radikale und der Anzahl an Glutamin- oder Asparagineinheiten zwischen 0 < i ≤ 0,5 beträgt;
- wenn mehrere hydrophobe Radikale von einer Co-Polyaminosäure getragen sind, sie identisch oder verschieden sind,
- der Polymerisationsgrad DP aus Glutamin- oder Asparagineinheiten zwischen 5 und 250 beträgt;
- die freien Säurefunktionen in Form eines Salzes eines Alkalikations vorliegen, das ausgewählt ist aus der Gruppe bestehend aus Na⁺ und K⁺.

## Claims

1. A composition in the form of an injectable aqueous solution, the pH of which is from 6.0 to 8.0, comprising at least:
a) one basal insulin the isoelectric point pI of which is from 5.8 to 8.5,
b) a co-polyamino acid bearing carboxylate charges and hydrophobic radicals Hy, said co-polyamino acid consisting of glutamic or aspartic units, and said hydrophobic radicals Hy being radicals of the following formula I: 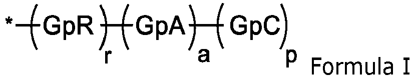 in which
- GpR is a radical of formula II or II' : 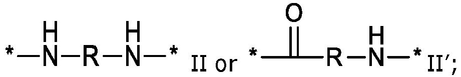
- GpA is a radical of formula III or III' : 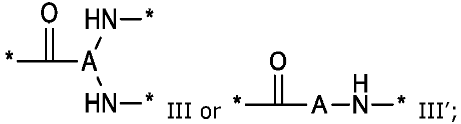
- GpC is a radical of formula IV : 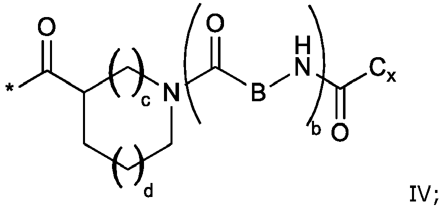
- Hy comprises more than 30 carbon atoms,
- the * indicate the sites of attachment of the different groups bound by amide functions;
- a is a whole number equal to 0 or 1;
- b is a whole number equal to 0 or 1;
- p is a whole number equal to 1 or 2, and
∘ if p is equal to 1, then a is equal to 0 or 1 and GpA is a radical of formula III', and
∘ if p is equal to 2, then a is equal to 1 and GpA is a radical of formula III;
- c is a whole number equal to 0 or 1, and, if c is equal to 0, then d is equal to 1 or 2;
- d is a whole number equal to 0, to 1 or 2;
- r is a whole number equal to 0, to 1, and
∘ if r is equal to 0, then the hydrophobic radical of formula I is bound to the co-polyamino acid via a covalent bond between a carbonyl of the hydrophobic radical and a nitrogen atom in N-terminal position of the co-polyamino acid, thus forming an amide function, and
∘ if r is equal to 1, then the hydrophobic radical of formula I is bound to the co-polyamino acid:
▪ via a covalent bond between a nitrogen atom of the hydrophobic radical and a carbonyl of the co-polyamino acid, thus forming an amide function, or
▪ via a covalent bond between a carbonyl of the hydrophobic radical and a nitrogen atom in N-terminal position of the co-polyamino acid, thus forming an amide function;
- R is a radical selected from the group consisting of:
∘ a linear or branched divalent alkyl radical comprising, if GpR is a radical of formula II, from 2 to 12 carbon atoms, or, if GpR is a radical of formula II', from 1 to 11 carbon atoms;
∘ a linear or branched divalent alkyl radical comprising, if GpR is a radical of formula II, from 2 to 11 carbon atoms, or, if GpR is a radical of formula II', from 1 to 11 carbon atoms, said alkyl radical bearing one or more-CONH₂ functions, and
∘ an un-substituted ether or polyether radical comprising from 4 to 14 carbon atoms and from 1 to 5 oxygen atoms;
- A is a linear or branched alkyl radical comprising from 1 to 6 carbon atoms;
- B is a linear or branched alkyl radical, optionally comprising an aromatic ring, comprising from 1 to 9 carbon atoms;
- Cₓ is a linear or branched monovalent alkyl radical, in which x indicates the number of carbon atoms, and:
∘ if p is equal to 1, x is from 11 to 25 (11 ≤ x ≤ 25),
∘ if p is equal to 2, x is from 9 to 15 (9 ≤ x ≤ 15),
- the ratio i between the number of hydrophobic radicals and the number of glutamic or aspartic units being between 0 < i ≤ 0,5 ;
- when several hydrophobic radicals are borne by a co-polyamino acid, then they are identical or different,
- the degree of polymerization DP in glutamic or aspartic units is from 5 to 250;
- the free acid functions being in the form of a salt of an alkaline cation selected from the group consisting of Na⁺ and K⁺.

2. The composition according to Claim 1, **characterized in that** said hydrophobic radicals are selected from the hydrophobic radicals of formula I in which p = 1, represented by the following formula V: 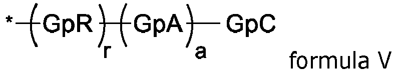 GpR, GpA, GpC, r and a as defined in claim 1.

3. The composition according to Claim 1, **characterized in that** said hydrophobic radicals are selected from the hydrophobic radicals of formula I in which a = 1 and p = 2, represented by the following formula VI: 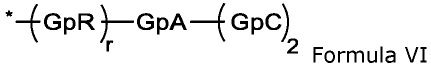 in which
GpR, GpA, GpC, r and a as defined in claim 1.

4. The composition according to any one of the preceding claims, **characterized in that** the co-polyamino acid bearing carboxylate charges and hydrophobic radicals is selected from the co-polyamino acids of the following formula VII: 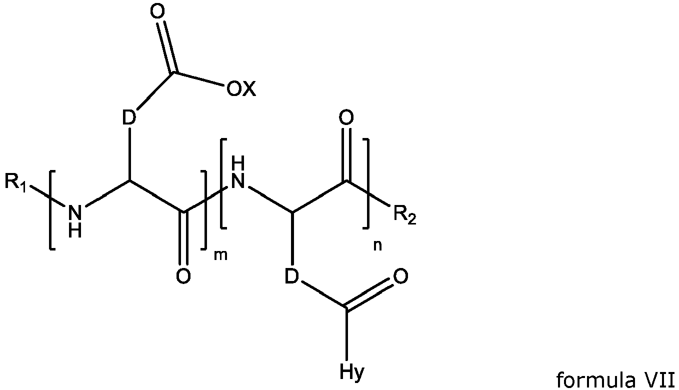 in which,
• D represents, independently, either a -CH₂- group (aspartic unit) or a - CH₂-CH₂- group (glutamic unit),
• Hy is a hydrophobic radical selected from the hydrophobic radicals of formula I, V or VI, in which r = 1 and GpR is a radical of Formula II,
• R₁ is a hydrophobic radical selected from the hydrophobic radicals of formula I, V or VI in which r = 0 or r = 1 and GpR is a radical of Formula II', or a radical selected from the group consisting of H, a C2 to C10 linear acyl group, a C4 to C10 branched acyl group, benzyl, a terminal "amino acid" unit and a pyroglutamate,
• R₂ is a hydrophobic radical selected from the hydrophobic radicals of formula I, V or VI in which r = 1 and GpR is a radical of Formula II, or an -NR'R" radical, R' and R" which are identical or different being selected from the group consisting of H, the C2 to C10 linear or branched or cyclic alkyls, benzyl, and said alkyl R' and R" together optionally forming one or more saturated, unsaturated and/or aromatic carbon rings and/or optionally comprising heteroatoms selected from the group consisting of O, N and S;
• X represents a cationic entity selected from the group comprising the alkaline cations;
• n + m represents the degree of polymerization DP of the co-polyamino acid, that is to say the average number of monomer units per co-polyamino acid chain, and 5 ≤ n + m ≤ 250 .

5. The composition according to Claim 4, **characterized in that** co-polyamino acid bearing carboxylate charges and hydrophobic charges is selected from the co-polyamino acids of formula VII, in which R₁ = R'₁ and R₂ = R'₂, of the following formula VIIa: 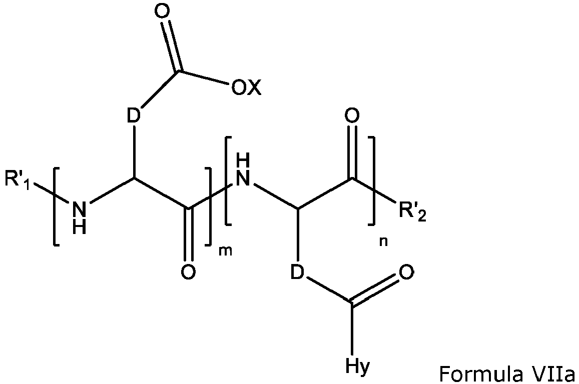 in which,
- m, n, X, D and Hy as defined in claim 4,
- R'₁ is a radical selected from the group consisting of H, a C2 to C10 linear acyl group, a C4 to C10 branched acyl group, benzyl, a terminal "amino acid" unit and a pyroglutamate,
- R'₂ is a -NR'R" radical, R' and R" which are identical or different being selected from the group consisting of H, the C2 to C10 linear or branched or cyclic alkyls, benzyl, and said alkyl R' and R" together optionally forming one or more saturated, unsaturated and/or aromatic carbon rings and/or optionally comprising heteroatoms selected from the group consisting of O, N and S.

6. The composition according to Claim 4, **characterized in that** the co-polyamino acid bearing carboxylate charges and hydrophobic radicals is selected from the co-polyamino acids of formula VII, in which n = 0, of the following formula VIIb: 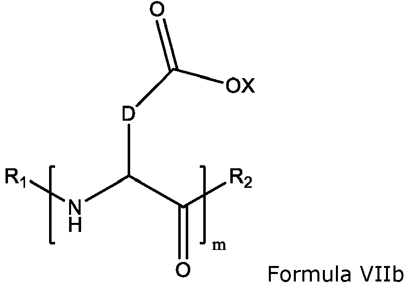 in which m, X, D, R₁ and R₂ as defined in claim 4 and at least R₁ or R₂ is a hydrophobic radical of formula I, V or VI.

7. The composition according to Claim 6, **characterized in that** the co-polyamino acid bearing carboxylate charges and hydrophobic radicals is selected from the co-polyamino acids of formula VIIb, in which R₂ is a hydrophobic radical of formula I, V or VI, in which r = 1 and GpR is of formula II.

8. The composition according to any one of Claims 4 to 7, **characterized in that** R₁ is a radical selected from the group consisting of a C₂ to C₁₀ linear acyl group, a C₄ to C₁₀ branched acyl group, benzyl, a terminal "amino acid" unit and a pyroglutamate.

9. The composition according to the preceding claim, **characterized in that** R₁ is a radical selected from the group consisting of a C₂ to C₁₀ linear acyl group or a C₄ to C₁₀ branched acyl group.

10. The composition according to any one of Claims 4 to 9, **characterized in that** the co-polyamino acid bearing carboxylate charges and hydrophobic radicals is selected from the co-polyamino acids of formula VII, VIIa or VIIb, in which the co-polyamino acid is selected from the co-polyamino acids in which group D is a -CH₂- group (aspartic unit).

11. The composition according to any one of Claims 4 to 9, **characterized in that** the co-polyamino acid bearing carboxylate charges and hydrophobic radicals is selected from the co-polyamino acids of formula VII, VIIA or VIIb, in which the co-polyamino acid is selected from the co-polyamino acids in which group D is a -CH₂-CH₂- group (glutamic unit).

12. The composition according to any one of the preceding claims, **characterized in that** the basal insulin of which the isoelectric point is from 5.8 to 8.5 is insulin glargine.

13. The composition according to any one of the preceding claims, **characterized in that** it comprises from 40 to 500 U/mL of basal insulin of which the isoelectric point is from 5.8 to 8.5.

14. The composition according to any one of the preceding claims, **characterized in that** the concentration of co-polyamino acid bearing carboxylate charges and hydrophobic radicals is at most 60 mg/mL.

15. A co-polyamino acid bearing carboxylate charges and hydrophobic radicals Hy of the following formula I: 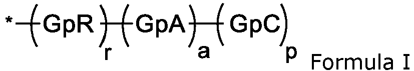 in which
- GpR is a radical of formula II or II' : 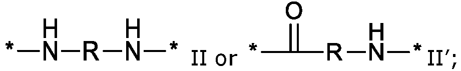
- GpA is a radical of formula III or III' : 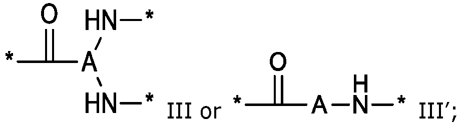
- GpC is a radical of formula IV : 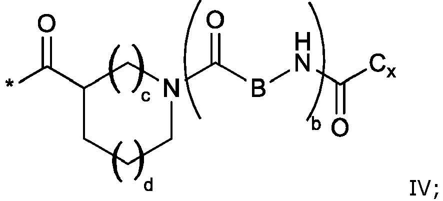
- Hy comprises more than 30 carbon atoms,
- the * indicate the sites of attachment of the different groups bound by amide functions;
- a is a whole number equal to 0 or 1;
- b is a whole number equal to 0 or 1;
- p is a whole number equal to 1 or 2, and
∘ if p is equal to 1, then a is equal to 0 or 1 and GpA is a radical of formula III', and
∘ if p is equal to 2, then a is equal to 1 and GpA is a radical of formula III;
- c is a whole number equal to 0 or 1, and, if c is equal to 0, then d is equal to 1 or 2;
- d is a whole number equal to 0, to 1 or 2;
- r is a whole number equal to 0, to 1, and
∘ if r is equal to 0, then the hydrophobic radical of formula I is bound to the co-polyamino acid via a covalent bond between a carbonyl of the hydrophobic radical and a nitrogen atom in N-terminal position of the co-polyamino acid, thus forming an amide function, and
∘ if r is equal to 1, then the hydrophobic radical of formula I is bound to the co-polyamino acid:
▪ via a covalent bond between a nitrogen atom of the hydrophobic radical and a carbonyl of the co-polyamino acid, thus forming an amide function, or
▪ via a covalent bond between a carbonyl of the hydrophobic radical and a nitrogen atom in N-terminal position of the co-polyamino acid, thus forming an amide function;
- R is a radical selected from the group consisting of:
∘ a linear or branched divalent alkyl radical comprising, if GpR is a radical of formula II, from 2 to 12 carbon atoms, or, if GpR is a radical of formula II', from 1 to 11 carbon atoms;
∘ a linear or branched divalent alkyl radical comprising, if GpR is a radical of formula II, from 2 to 11 carbon atoms, or, if GpR is a radical of formula II', from 1 to 11 carbon atoms, said alkyl radical bearing one or more-CONH₂ functions, and
∘ an un-substituted ether or polyether radical comprising from 4 to 14 carbon atoms and from 1 to 5 oxygen atoms;
- A is a linear or branched alkyl radical comprising from 1 to 6 carbon atoms;
- B is a linear or branched alkyl radical, optionally comprising an aromatic ring, comprising from 1 to 9 carbon atoms;
- Cₓ is a linear or branched monovalent alkyl radical, in which x indicates the number of carbon atoms, and:
∘ if p is equal to 1, x is from 11 to 25 (11 ≤ x ≤ 25),
∘ if p is equal to 2, x is from 9 to 15 (9 ≤ x ≤ 15),
- the ratio i between the number of hydrophobic radicals and the number of glutamic or aspartic units being between 0 < i ≤ 0,5 ;
- when several hydrophobic radicals are borne by a co-polyamino acid, then they are identical or different,
- the degree of polymerization DP in glutamic or aspartic units is from 5 to 250;
the free acid functions being in the form of a salt of an alkaline cation selected from the group consisting of Na⁺ and K⁺.
